# EUROPEAN PATENT SPECIFICATION

(11) **EP 3 988 098 B1**
(45) Date of publication and mention of the grant of the patent: **24.07.2024**
(21) Application number: 20830755.3
(22) Date of filing: 24.06.2020
(51) Int. Cl.: C07D 405/14, C07D 403/14, C07D 401/14, A61K 31/395, A61P 1/16

(54) **HETEROCYCLO ALKYL COMPOUNDS USED AS CCR2/CCR5 ANTAGONISTS**
HETEROCYCLOALKYLVERBINDUNGEN ALS CCR2/CCR5-ANTAGONISTEN
COMPOSÉS HÉTÉROCYCLO-ALKYLES UTILISÉS EN TANT QU'ANTAGONISTES DE CCR2/CCR5

(30) Priority: 24.06.2019 CN 201910554176
(43) Date of publication of application: 27.04.2022
(73) Proprietor: Wuxi Life Fountain Biotech Co., Ltd., Wuxi City (CN)
(72) Inventor: LUO, Yunfu, Shanghai 200131 (CN); BA, Yuyong, Shanghai 200131 (CN); CHEN, Yanhe, Shanghai 200131 (CN); CHEN, Shuhui, Shanghai 200131 (CN)
(74) Representative: Ipey
(86) International application number: PCT/CN2020/098222
(87) International publication number: WO 2020/259620

(56) References cited:
- EP-A1- 1 422 228
- EP-A1- 1 422 228
- EP-A1- 1 593 681
- WO-A1-03/014105
- WO-A1-2018/103757
- WO-A1-2018/103757
- MASAKI SETO ET AL: "Highly Potent and Orally Active CCR5 Antagonists as Anti-HIV-1 Agents: Synthesis and Biological Activities of 1-Benzazocine Derivatives Containing a Sulfoxide Moiety", JOURNAL OF MEDICINAL CHEMISTRY, vol. 49, no. 6, 1 January 2006 (2006-01-01), US, pages 2037 - 2048, XP055460609, ISSN: 0022-2623, DOI: 10.1021/jm0509703
- FRANK TACKE: "Cenicriviroc for the treatment of non-alcoholic steatohepatitis and liver fibrosis", EXPERT OPINION ON INVESTIGATIONAL DRUGS, vol. 27, no. 3, 22 February 2018 (2018-02-22), UK, pages 301 - 311, XP055769263, ISSN: 1354-3784, DOI: 10.1080/13543784.2018.1442436
- MASAKI SETO, AIKAWA KATSUJI, MIYAMOTO NAOKI, ARAMAKI YOSHIO, KANZAKI NAOYUKI, TAKASHIMA KATSUNORI, KUZE YOJI, IIZAWA YUJI, BABA MA: "Highly Potent and Orally Active CCR5 Antagonists as Anti-HIV-1 Agents: Synthesis and Biological Activities of 1-Benzazocine Derivatives Containing a Sulfoxide Moiety", JOURNAL OF MEDICINAL CHEMISTRY, AMERICAN CHEMICAL SOCIETY, US, vol. 49, no. 6, 1 January 2006 (2006-01-01), US, pages 2037 - 2048, XP055460609, ISSN: 0022-2623, DOI: 10.1021/jm0509703
- FRANK TACKE: "Cenicriviroc for the treatment of non-alcoholic steatohepatitis and liver fibrosis", EXPERT OPINION ON INVESTIGATIONAL DRUGS, INFORMA HEALTHCARE, UK, vol. 27, no. 3, 4 March 2018 (2018-03-04), UK, pages 301 - 311, XP055769263, ISSN: 1354-3784, DOI: 10.1080/13543784.2018.1442436

## Description

### CROSS-REFERENCE TO RELATED APPLICATION

This application claims priority of the Chinese invention patent application No. CN201910554176.4, filed to State Intellectual Property Office of P.R.China on June 24, 2019.

### TECHNICAL FIELD

The invention relates to a series of compounds with heterocycloalkyl , or isomers or pharmaceutically acceptable salts thereof, and to a use thereof in manufacturing a medicine of treatment CCR2/ CCR5 antagonist-related diseases, specifically to a compound of Formula (I), or an isomer or pharmaceutically acceptable salt thereof.

### BACKGROUND

The chemokine is a small, secreted pro-inflammatory cytokine family, which functions as a chemical inducer for white blood cells. They promote transportation of leukocytes from the vessel bed to the surrounding tissue responding to inflammatory signals. Chemotaxy starts at binding of chemokine and receptor thereof (GPCR), initiating increased calcium flow, inhibition of production of cyclic adenosine monophosphate, rearrangement of cytoskeleton, activation of integrin and signalling pathway of cell movement process, and increasing expression of adhesion protein.

Chemical inducers (i.e., chemokines) are relatively small proteins (8-10 kD), which stimulates migration of cells. Based on the number of amino acid residues between the first and second highly conserved cysteines, the family of chemokines is classified into four subfamilies. Monocyte chemoattractant protein-1 (MCP-1) is a member of CC chemokine subfamily (where CC represents a subfamily having the adjacent first and second cysteines) and binding cell surface chemokine receptor 2 (CCR2). MCP-1 is an effective chemokine, which mediates the migration of monocytes and lymphocytes to sites of inflammation after binding to CCR2 (i.e. chemotaxis). MCP-1 is also expressed by cardiomyocytes, vascular endothelial cells, fibroblasts, cartilage cells, smooth muscle cells, glomerular cell, alveolar cells, T lymphocytes, esophageal cancers. After monocytes enter into the inflammatory tissue, differentiate into macrophages expressing CCRS, providing several secondary sources of pro-inflammatory regulators, including tumor necrosis factor-α (TNF-α), interleukin-1 (IL-1) ), IL-8 CXC chemokine subfamily where CXC represents a amino acid residue between the first and second cysteines), IL-12, peanut tetraneic acid metabolites (eg, PGE 2 and LTB 4 ), oxygen-derived free radicals, matrix metalloprotease and complement components.

CCR2 (also known as CKR-2, MCP-1RA or MCIRB) is mainly expressed in monocytes and macrophages, and is required for macrophage-dependent inflammation. CCR2 is a G protein-coupled receptor (GPCR) to several members of the MCP family (CCL2, CCI7, CCL8, etc.) in high affinity, which triggers a chemotaxis signal to result in migration of orientation receptor. The animal model of chronic inflammatory disease has proven that the antagonist inhibits the binding of MCP-1 and CCR2 to inhibit the inflammatory response.

CCRS is a G protein coupled receptor binding to a plurality of CC chemokine ligands, including CCL3, CCL3L1, CCL4, CCL5, CCL7, CCL11 and CCL13. Compared with CCR2, the function of CCRS in vivo is less clear. Compared with CCR2, CCRS is mainly expressed in activated Th1 cells and tissue macrophages differentiated from blood mononuclear cells which down-regulates CCR2 expression concomitantly. CCRS has been shown that CCRS contributes to survival of macrophages during inflammation and infection, and can also play a role in retaining macrophages in inflammatory tissue. In addition, CCRS mediates recruitment and activation of Th1 cells in inflammation. CCRS is also expressed in osteoclasts and is important for osteoclast formation, which indicates the contribution of CCRS in the pathological pathology of rheumatoid arthritis. The activation of vascular smooth cells in which CCL4/CCRS involved can also facilitate atherosclerosis and AIH pathology (accelerated intimal hyperplasia).

Complementary cell distribution and differential cell functions of CCR2 and CCRS provide that dual-targeting to two receptors may have greater efficacy than targeting to a single receptor. In monocyte / macrophage biology, CCR2 plays an important role in mediating migration of monocytes from bone marrow to blood and from blood to tissue, where CCRS mainly regulates activation, survey and possible reservation of macrophages in inflammatory tissue. In addition, CCRS block can improve treatment potential of dual antagonists by inhibiting T cell responses in addition to the effects of monocyte / macrophages. Based on the advantages of dual targeting of CCR2 and CCRS, CCR2 / CCRS antagonists have also been studied in depth, and there are four drugs that have entered into the clinic stage, for example, Cenicriviroc from Tobira, BMS-813160 from Bristol-Myers Squibb and PF-04634817 from Pfizer. Therefore, CCR2/CCRS antagonists have good potential for drug-making . Herein, we claim right for patent protection for heterocycloalkyl compounds as CCR2/CCRS antagonists.

WO2003014105A1 reports a compound, Cenicriviroc (CVC), and Reference A, and the structures are shown below.

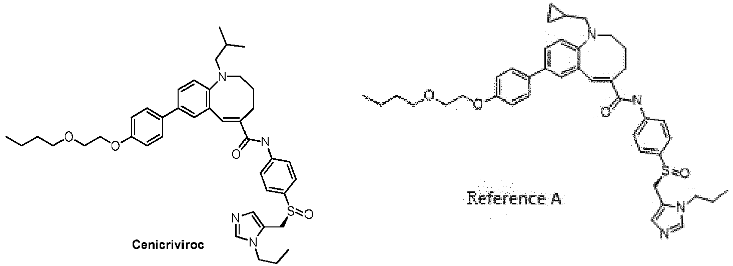

### SUMMARY

The invention provides a compound of formula (I), or an isomer or pharmaceutically acceptable salt thereof, 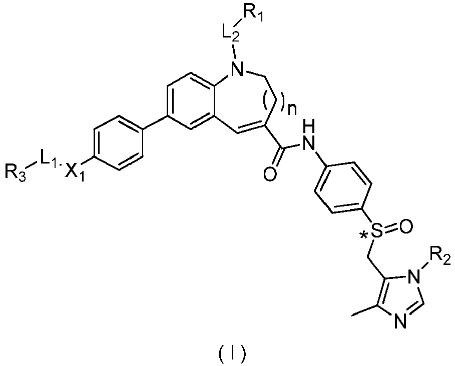 wherein,
n is selected from the group consisting of 1, 2, and 3;
R₁ is 4 to 6 membered heterocycloalkyl, which is optionally substituted by 1, 2 or 3 Rₐ;
R₂ is a C₁₋₆ alkyl, which is optionally substituted by 1, 2 or 3 R_{b};
R₃ is a C₁₋₆ alkoxy,which is optionally substituted by 1, 2 or 3 R_{c};
X₁ is selected from the group consisting of O, S and -NH-;
L₁ is - (CRR)ₘ-;
L₂ is -CRR-;
m is selected from the group consisting of 1, 2, 3, and 4;
each Rₐ, R_{b} and R_{c} are independently selected from the group consisting of H, F, Cl, Br, I, OH, NH₂, CN, and CH₃;
each R is independently selected from the group consisting of H, F, Cl, Br, I, OH, NH₂, CN, and CH;
the 4 to 6-membered heterocycloalkyl comprises 1, 2, 3 or 4 heteroatoms or heteroatom groups independently selected from the group consisting of -NH -, - O - S- and N; wherein the isomer thereof is a geometric or stereoisomeric isomer; and
the sulfur atom with"*" is a chiral sulfur atom, which exists as a (R) or (S) single enantiomer or is riched in one of the enantiomers.

In some embodiments of the invention, R₂ is a C₁₋₃ alkyl, which is optionally substituted by 1, 2 or 3 R_{b}, and other variables are as defined in the invention.

In some embodiments of the invention, R₂ is CH₂CH₃, and the other variables are as defined in the invention.

In some embodiments of the invention, R₃ is a C₄₋₆ alkoxy, which is optionally substituted by 1, 2 or 3 R_{c}, and other variables are as defined in the invention.

In some embodiments of the invention, R₃ is defined by the 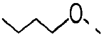 other variables as defined in the invention.

In some embodiments of the invention, L₁ is -CH₂CH₂-, and other variables are as defined in the invention.

In some embodiments of the invention, the structural unit 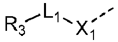 is 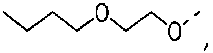 and other variables are as defined in the invention.

In some embodiments of the invention, L₂ is -CH₂-, and other variables are as defined in the invention.

In some embodiments of the invention, R₁ is selected from the group consisting of oxetanyl, tetrahydrofuranyl, tetrahydropyranyl, Hexapyridyl, morpholinyl and dioxanyl, which is optionally substituted by 1, 2 or 3 Rₐ, and other variables are as defined in the invention.

In some embodiments of the invention, R₁ is selected from the group consisting of 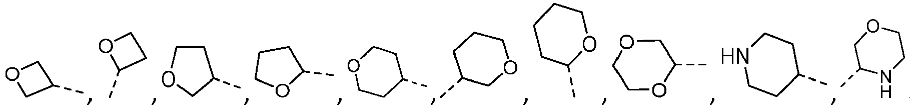 and 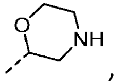 which is optionally substituted by 1, 2 or 3 Rₐ, and other variables are as defined in the invention.

In some embodiments of the invention, the structural unit 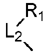 is selected from the group consisting of 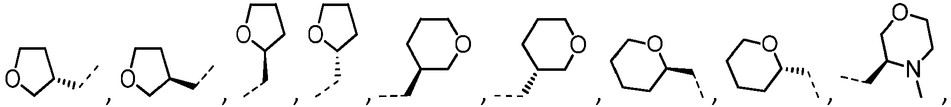 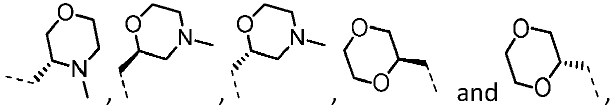 and other variables are as defined in the invention.

In some embodiments of the invention, the structural unit 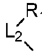 1 is selected from the group consisting of 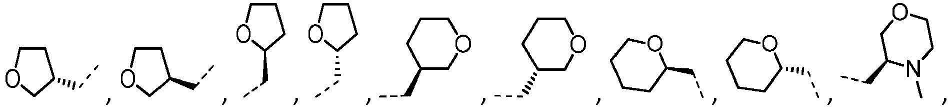 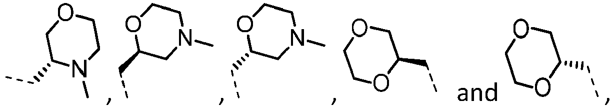 and other variables are as defined in the invention.

In some embodiments of the invention, the compound or pharmaceutically acceptable salt thereof is selected from the group consisting of 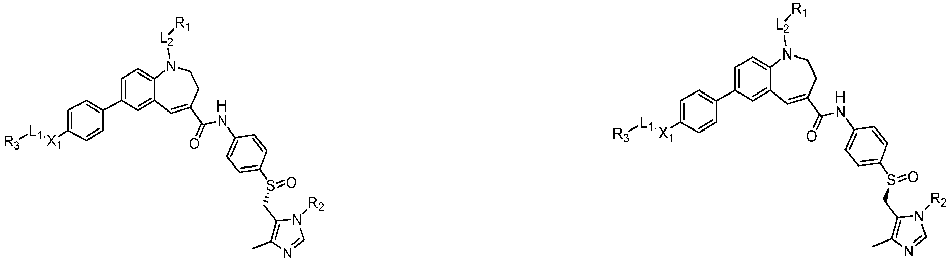 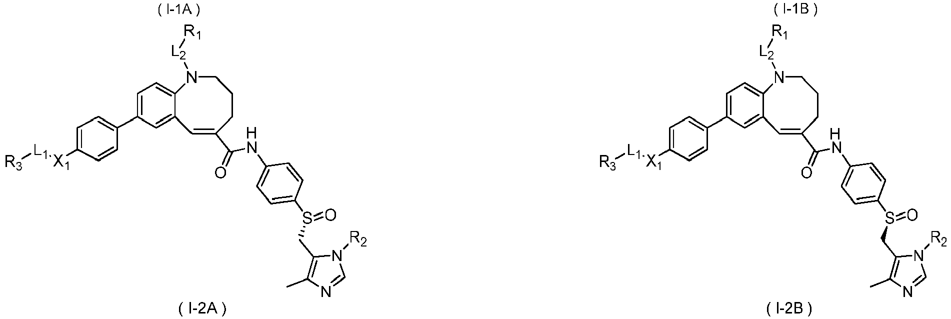 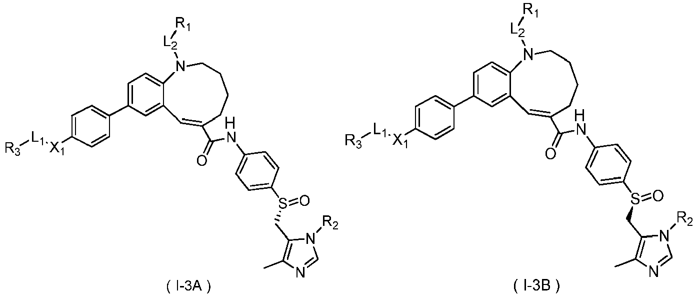 wherein, R₁, R₂, R₃, X₁, L₁, L₂ are as defined in the invention.

Some of embodiments of the invention are any combination of the above variables.

The invention also provides a compound of the following formula, or an isomer or pharmaceutically acceptable salt thereof 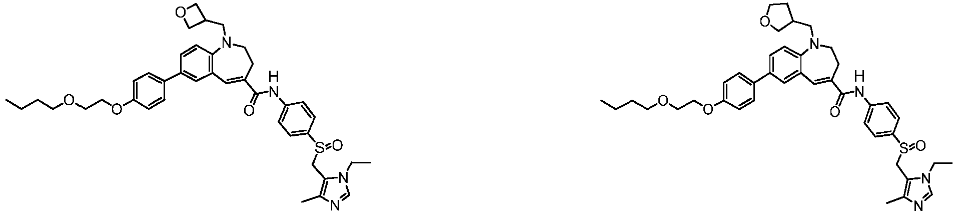 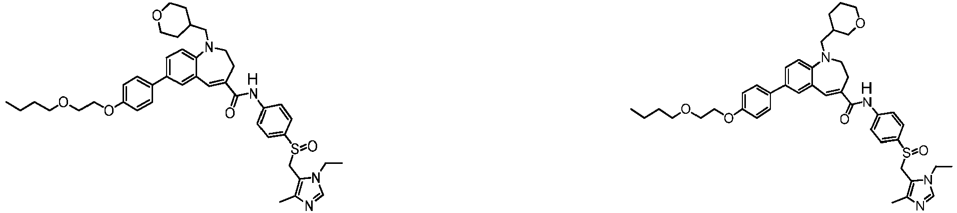 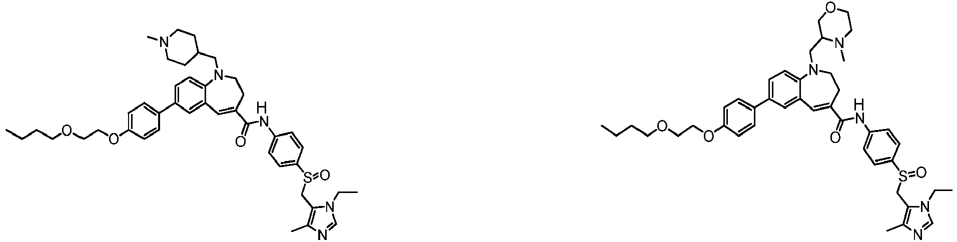 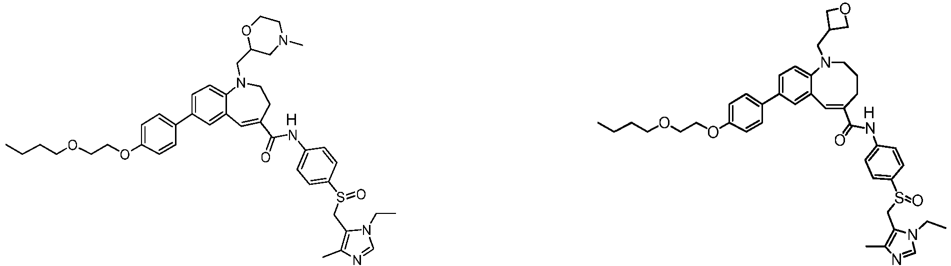 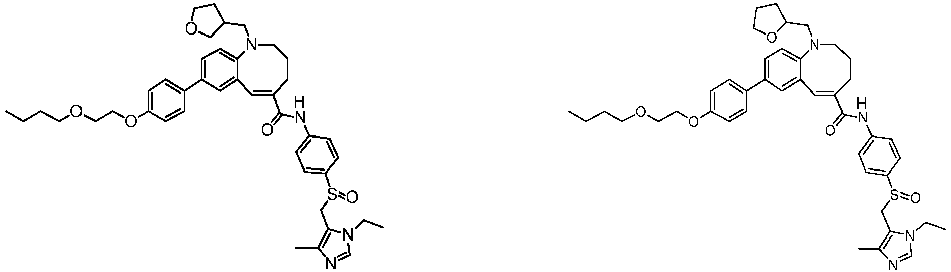 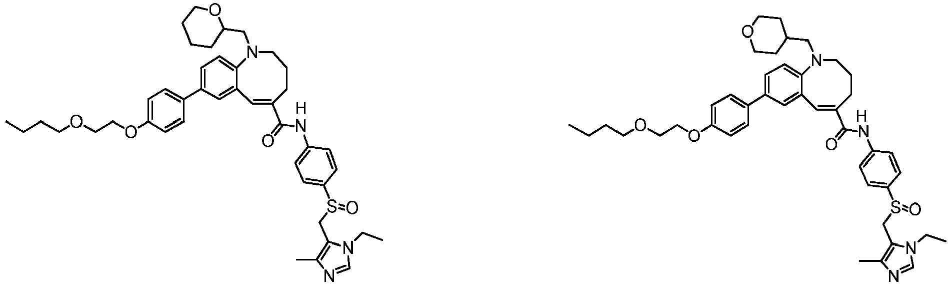 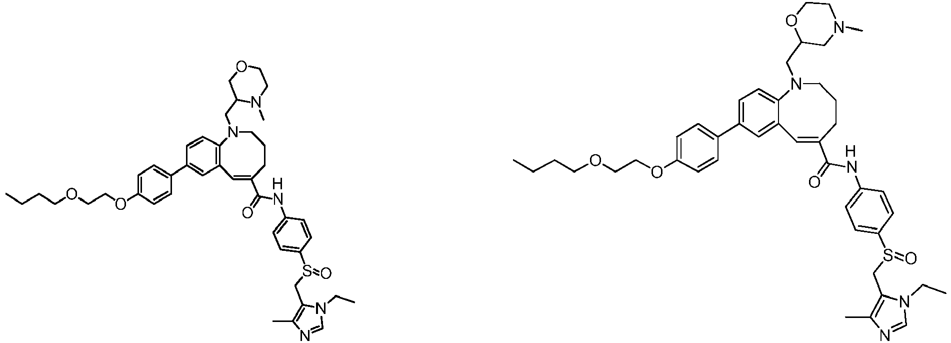 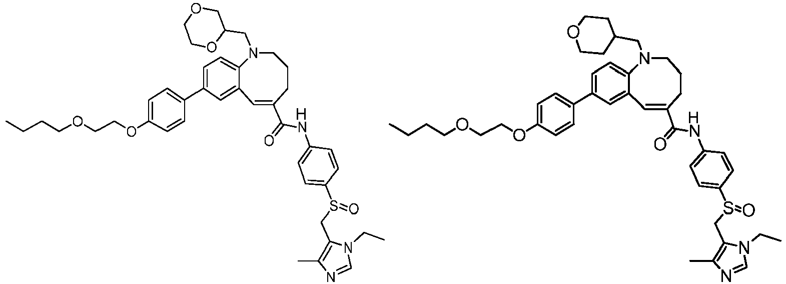 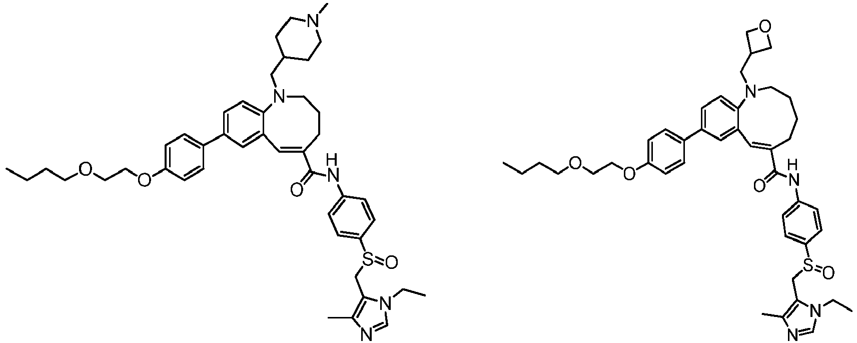 or

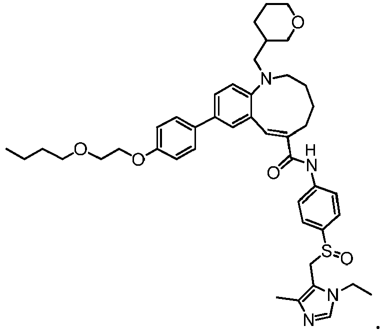

In some embodiments of the invention, the compounds, or the isomers or pharmaceutically acceptable salts thereof are selected from the group consisting of 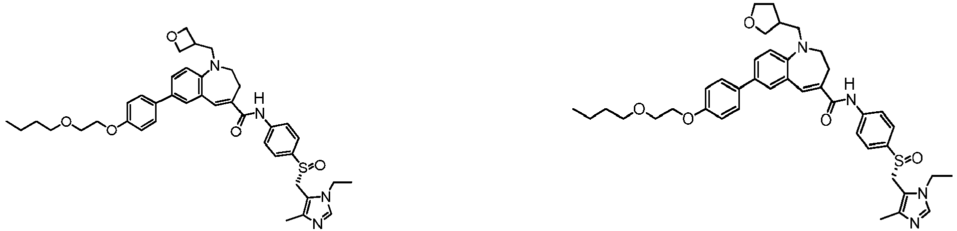 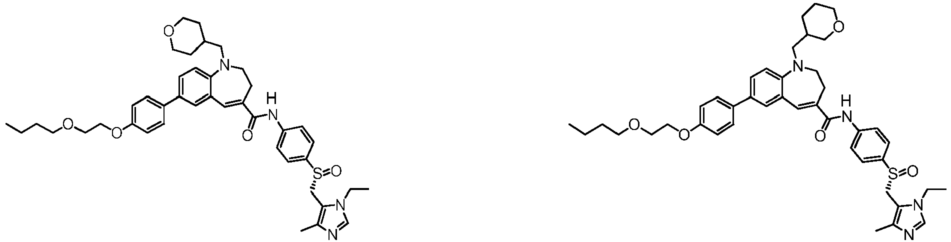 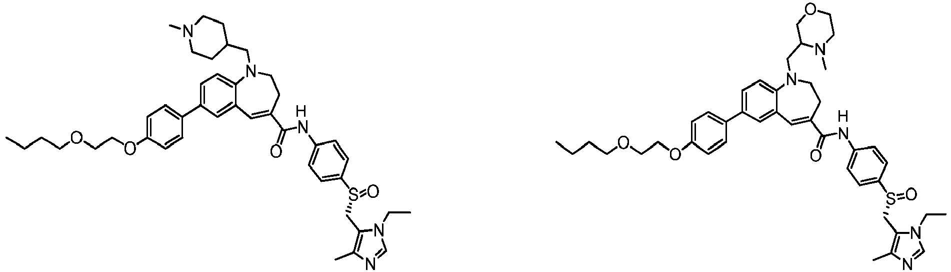 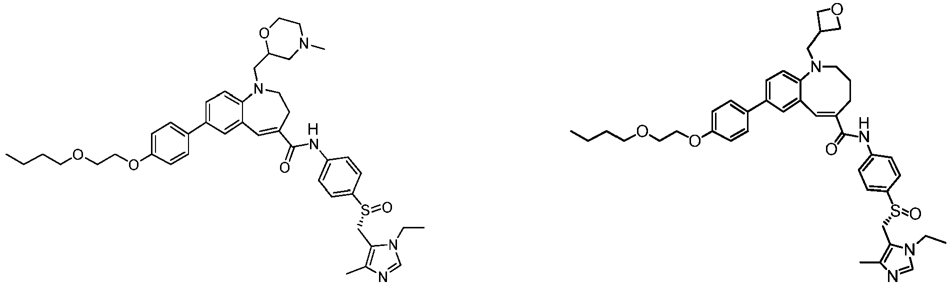 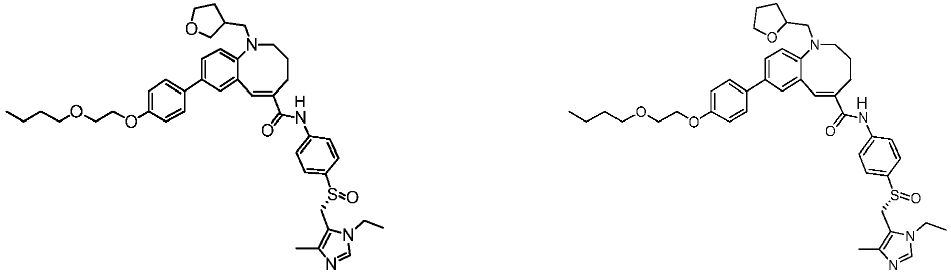 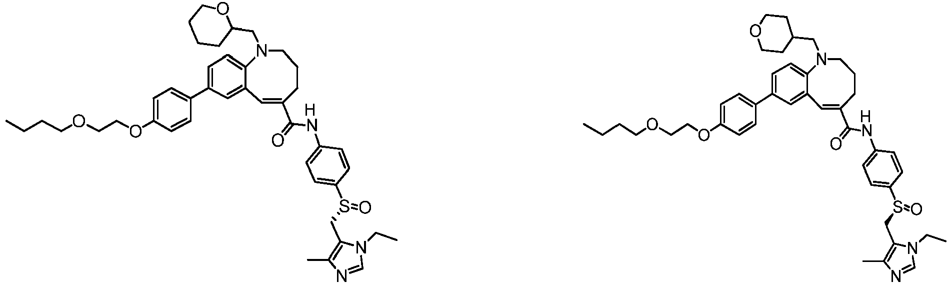 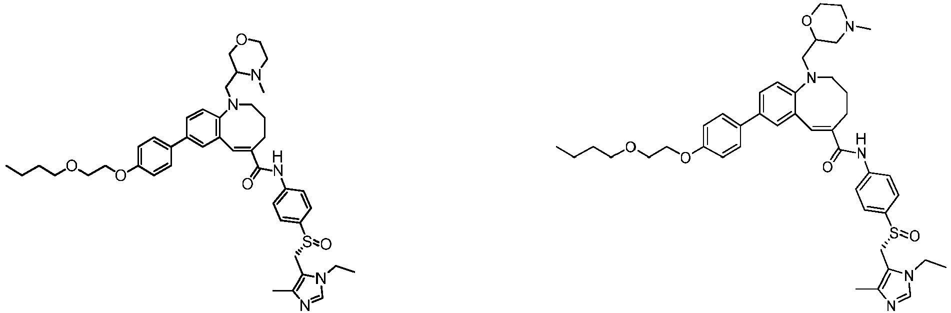 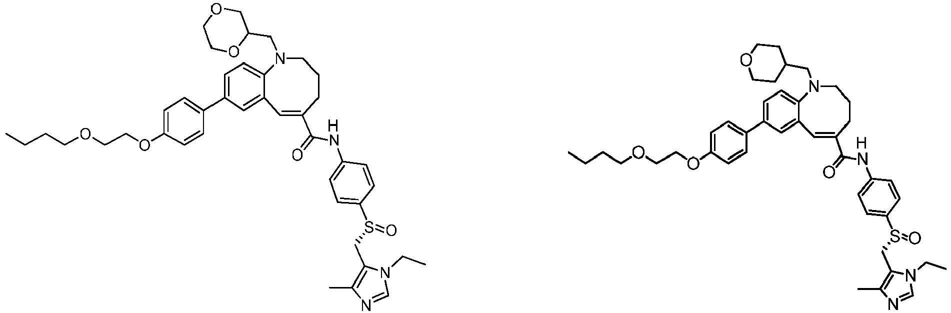 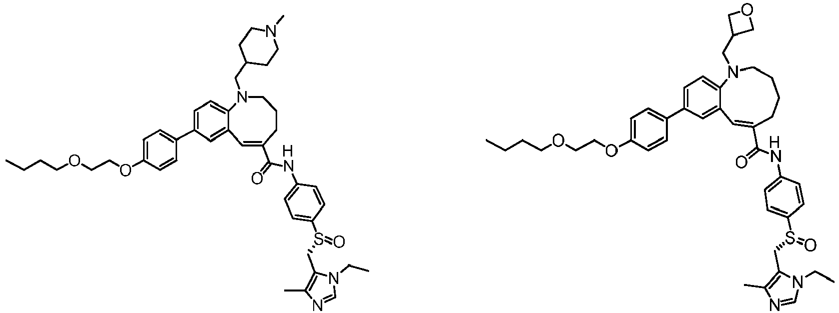 or

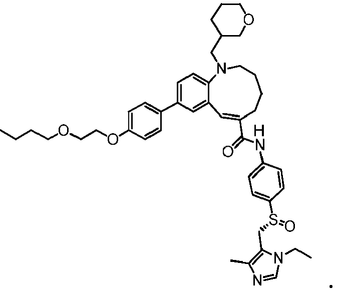

In some embodiments of the invention, the compound, or the isomer, or pharmaceutically acceptable salt is selected from the group consisting of

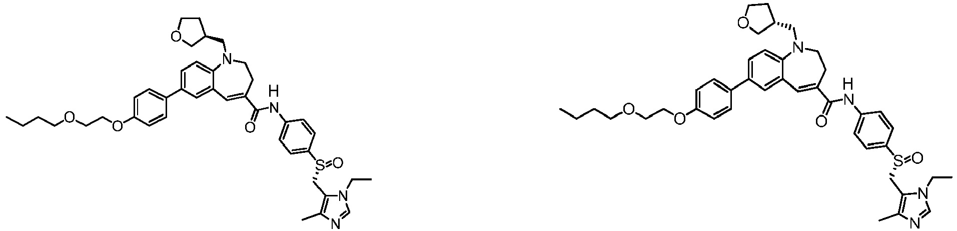

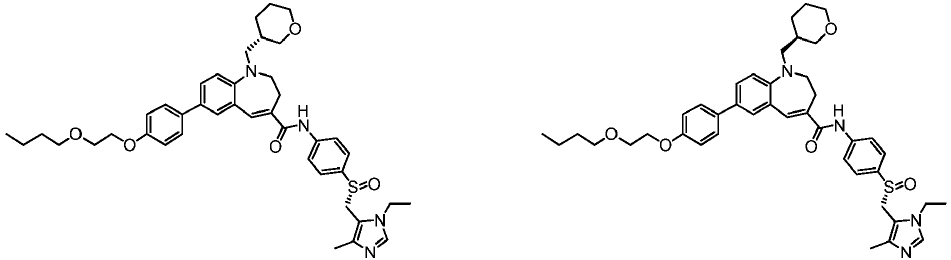

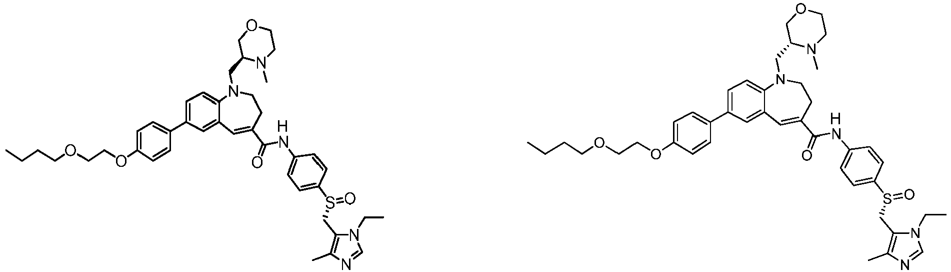

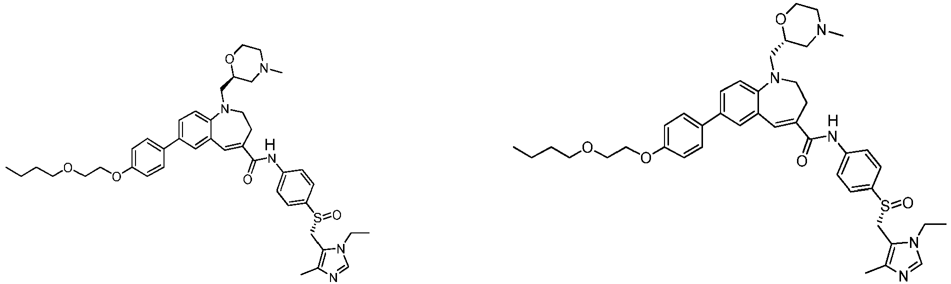

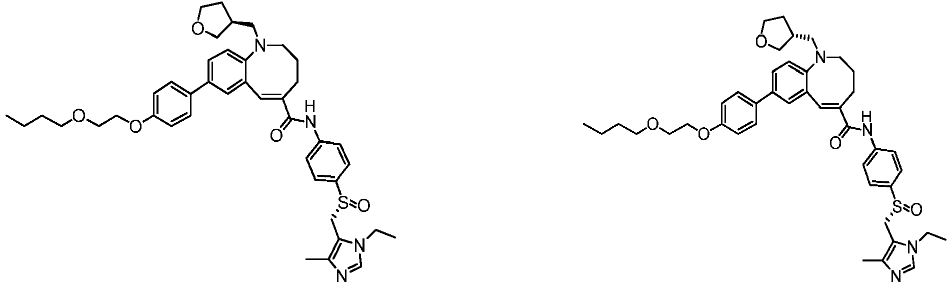

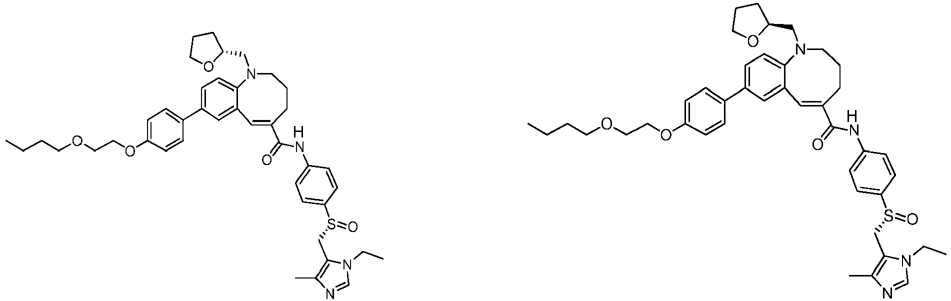

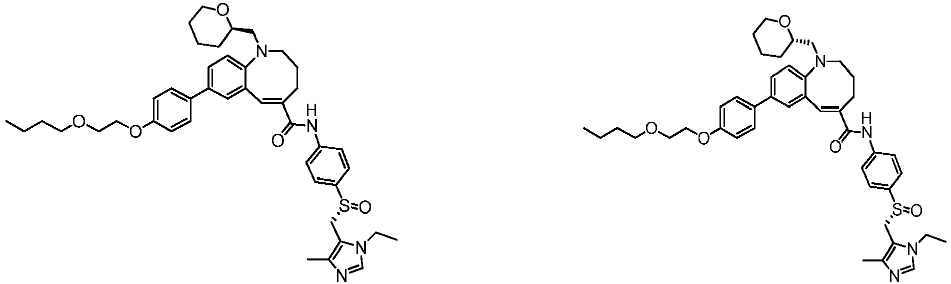

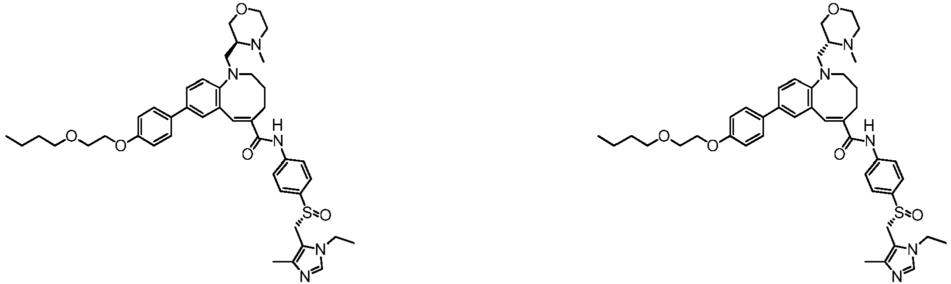

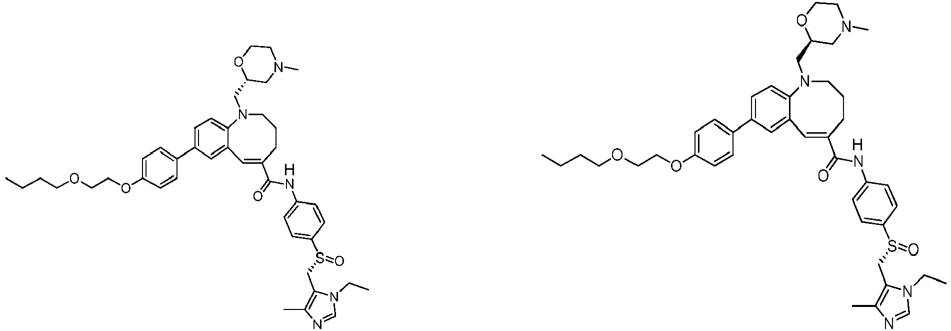

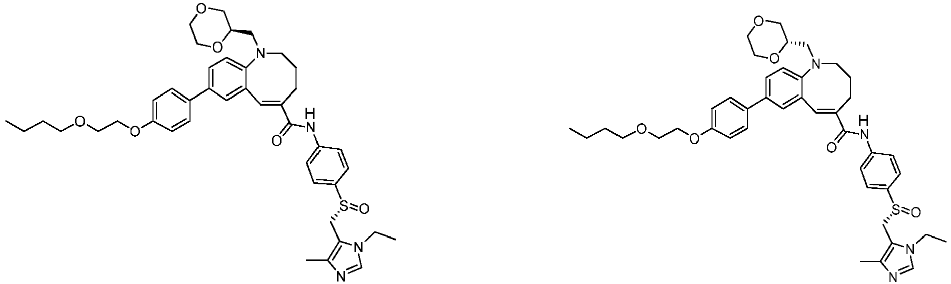

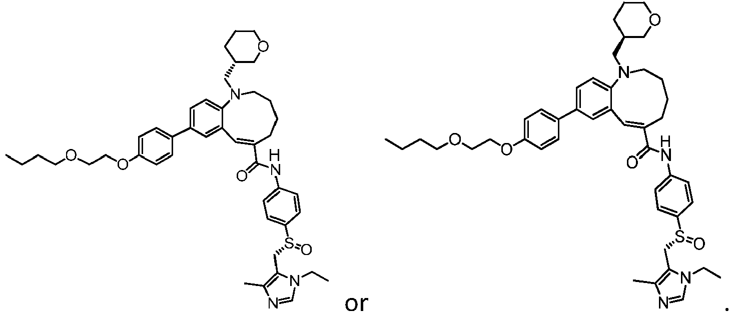

The invention also provides a pharmaceutical composition comprising a therapeutically effective amount of the compound or the pharmaceutically acceptable salt thereof as an active ingredient, and a pharmaceutically acceptable carrier.

The invention also provides a compound or isomer or a pharmaceutically acceptable salt thereof or pharmaceutical composition according to the invention for use in the treatment of CCR2/CCRS antagonist-related diseases.

The invention also provides a compound or isomer or a pharmaceutically acceptable salt thereof or pharmaceutical compositions according to the invention for use in the treatment of non-alcoholic fatty hepatitis.

### Technical effect

The compounds of the invention have significant effect on the antagonism of CCR2 and CCRS receptors. The compounds of the invention significantly reduce safety risks caused by drug-drug interaction, and significantly improve anti-tumor effect when combined with an antibody compared to the reference compound when combined with an antibody.

### Definition and description

Unless otherwise described, the following terms and phrases used herein are intended to have the following meanings. A specific term or phrase without being specifically defined should be understood by the plain meaning thereof rather than being regarded as uncertain or unclear. A brand name presented herein is intended to refer to a corresponding commercial product or the active component thereof.

The term "pharmaceutically acceptable" as used herein is directed to those compounds, materials, compositions and/or formulations which are within the scope of reliable medical judgment, suitable for use in contact with human and animal tissues but without too much toxicity, irritation, allergic reactions or other problems or complications, and also commensurate with a reasonable benefit/risk ratio.

The term "pharmaceutically acceptable salt" refers to a salt of the compound of the invention which is prepared from the compound with specific substituents discovered by the invention and a relatively non-toxic acid or alkali. When the compound of the invention contains a relatively acidic functional group, an alkali-addition salt can be obtained by contacting the compound in a neutral form with a sufficient amount of alkali in a pure solution or suitable inert solvent. The pharmaceutically acceptable alkali-addition salt includes the salt of sodium, potassium, calcium, ammonium, organic ammine or magnesium or the like. When the compound of the invention contains a relatively alkaline functional group, an acid-addition salt can be obtained by contacting the compound in a neutral form with a sufficient amount of acid in a pure solution or suitable inert solvent. Examples of the pharmaceutically acceptable acid-addition salt include a salt of an inorganic acid, where the inorganic acid includes such as hydrochloric acid, hydrobromic acid, nitric acid, carbonic acid, bicarbonate, phosphoric acid, hydrogen phosphate, dihydrogen phosphate, sulfuric acid, bisulfate, hydriodic acid, phosphorous acid etc; and a salt of an organic acid, where the organic acid includes such as acetic acid, propionic acid, isobutyric acid, maleic acid, malonic acid, benzoic acid, succinic acid, suberic acid, fumaric acid, lactic acid, mandelic acid, phthalic acid, phenylsulfonic acid, p-toluene sulfonic acid, citric acid, tartaric acid, methylsulfonic acid and the like; and also includes a salt of an amino acid (e.g. arginine etc.), and salts of organic acids such as glucuronic acid and the like. Some specific compounds of the invention contain both alkaline and acidic functional groups and thereby may be transformed to any of the alkali-addition or acid-addition salt.

The pharmaceutically acceptable salt of the invention can be prepared by a conventional chemical method with a parent compound containing an acidic or alkaline group. Generally, the preparation method of such salts comprises in water or an organic solvent or a mixture of both, reacting these compounds which are in the form of free acids or alkalis with a stoichiometric amount of proper alkalis or acids.

The compound of the invention may exist as a specific geometric or stereoisomeric isomer. The invention is envisaged that all of this class of compounds, including cis- and trans-isomers, (-)- and (+)-enantiomers, *(R)*- and *(S)*-enantiomers, diastereomers, *(D)-*isomer, *(L)*-isomer, as well as racemic mixtures and other mixtures, such as enantiomers- or diastereoisomers-enriched mixtures, and all of these mixtures are within the scope of the invention. Other asymmetric carbon atoms may exist in substituents such as an alkyl. All of these isomers and their mixtures are included within the scope of the invention.

Unless otherwise specified, the term "enantiomer" or "optical isomer" refers to stereoisomers that are mirror images of each other.

Unless otherwise specified, the term "cis-trans isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 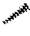 ) and a wedged dashed bond ( 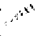 ), and the relative configuration of a stereogenic center is represented by a straight solid bond ( 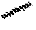 ) and a straight dashed bond ( 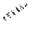 ), a wave line ( 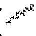 ) is used to represent a wedged dashed bond ( 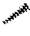 ) or a wedged dashed bond ( 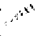 ), or the wave line ( 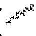 ) is used to represent a straight solid bond ( 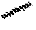 ) and a straight dashed bond ( 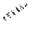 ).

Unless otherwise specified, when double bond structure, such as carbon-carbon double bond, carbon-nitrogen double bond, and nitrogen-nitrogen double bond, exists in the compound, and each of the atom on the double bond is connected to two different substituents (including the condition where a double bond contains a nitrogen atom, the lone pair of electrons attached on the nitrogen atom is regarded as a sub substituent connected), if the atom on the double bond in the compound is connected to its sub substituent by a wave line ( 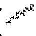 ) this refers to the (Z) isomer, (F) isomer or a mixture of two isomers of the compound. For example, the following formula (A) means that the compound exists as a single isomer of formula (A-1) or formula (A-2) or as a mixture of two isomers of formula (A-1) and formula (A-2); the following formula (B) means that the compound exists in the form of a single isomer of formula (B-1) or formula (B-2) or in the form of a mixture of two isomers of formula (B-1) and formula (B-2). The following formula (C) means that the compound exists as a single isomer of formula (C-1) or formula (C-2) or as two a mixture of two isomers of formula (C-1) and formula (C-2).

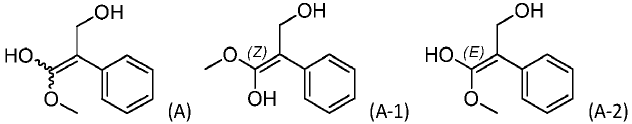

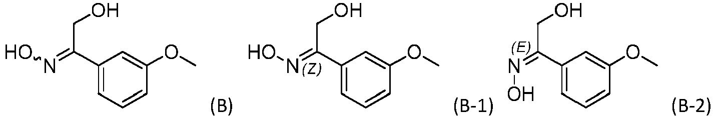

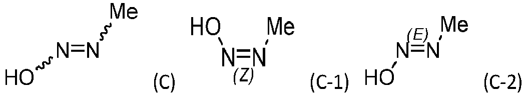

Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Active *(R)*- and *(S)*-isomers, and also *(D)-* and *(L)*-isomers can be prepared by asymmetric synthesis or chiral reagents or other conventional techniques. If desired, an enantiomer of a compound of the invention may be prepared by asymmetric synthesis or derivatization action of chiral auxiliaries, in which the resultant diastereomer mixtures are isolated, and the auxiliary groups are cleaved to provide the pure desired enantiomer. Or, when a molecule contains an alkaline functional group (such as an amino) or an acidic functional group (such as a carboxyl), a diastereomeric salt is formed with an appropriate optical active acid or alkali, followed by diastereoisomeric resolution by fractional crystallization or chromatography method known in the art and subsequent recovery to obtain the pure enantiomer. In addition, the separation of an enantiomer and a diastereomer is usually accomplished by chromatography, where the chromatography employs a chiral stationary phase, optionally in combination with chemical derivatization method (e.g. generating a carbamate from an amine).

The compound of the present disclosure may contain an unnatural proportion of atomic isotope at one or more than one atom(s) that constitute the compound. For example, the compound can be radiolabeled with a radioactive isotope, such as tritium (³H), iodine-125 (¹²⁵I) or C-14 (¹⁴C). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more hydrogen atom(s) on a specific atom are substituted with the substituent, including deuterium and hydrogen variants, as long as the valence of the specific atom is normal and the substituted compound is stable. When the substituent is oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0-2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent does not exist. For example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When an enumerative linking group does not indicate its linking direction, its linking direction is arbitrary. For example, when the linking group L in 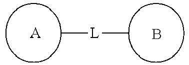 is -M-W-, the -M-W- can be linked to the ring A and the ring B in the same direction as the reading order from left to right to constitute 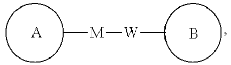 or can be linked to the ring A and the ring B in the reverse direction as the reading order from left to right to constitute 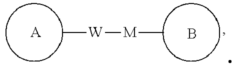 A combination of the linking groups, substituents and/or variants thereof is allowed only when such combination can result in a stable compound.

Unless otherwise specified, when a group has one or more connectable sites, any one or more of the sites of the group may be connected to other groups by chemical bonds. When there is no designated connecting mode for a chemical bond and H atoms are present at a connectable site, the number of the H atoms at the connectable site is correspondingly reduced based on the number of the connected chemical bonds, and a group with a corresponding valence number is thus formed. The chemical bond that connects the site to another group may be represented by a straight solid bond ( 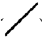 ), a straight dashed line bond ( 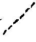 ), or a wavy line 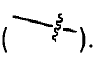 For example, the straight solid bond in -OCH₃ refers to being connected to another group via the oxygen atom in the group; the straight dashed bond in 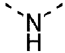 refers to being connected to another group via two ends of the nitrogen atom in the group; the wavy line in 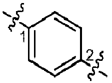 refers to being connected to another group via the carbon atoms at positions 1 and 2 in the phenyl group; 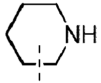 means that any connectable site on the piperidinyl can be connected to another group via 1 bond, and at least 4 connecting modes 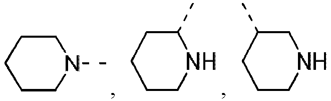 and 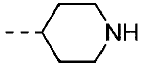 are possible; even if -N- is connected to a H atom, 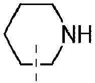 includes the connecting mode of 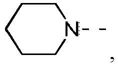 except that when 1 bond is connected to a site, the number of H at that site is correspondingly reduced by 1 and a monovalent piperidinyl is thus formed.

Unless otherwise specified, the term "4- to 6-membered heterocycloalkyl" itself or in combination with other terms means a saturated cyclic group consisting of 4 to 6 ring atoms, which 1, 2, 3 or 4 ring atoms are heteroatoms independently selected from O, S, and N, and the rest are carbon atoms, wherein the nitrogen atom is optionally quaternized. The term "4- to 6-membered heterocycloalkyl" is single ring systems. In addition, as far as the "4- to 6-membered heterocycloalkyl group" is concerned, a heteroatom may occupy a connection position between the heterocycloalkyl group and the rest of the molecule. The 4- to 6-membered heterocycloalkyl includes 4- to 6, 4- to 5, 5- to 6, 4-, 5-, and 6-membered heterocycloalkyl. Examples of 4- to 6-membered heterocycloalkyl include, but are not limited to, azetidinyl, oxetanyl, pyrrolidinyl, pyrazolidinyl, imidazolidinyl, tetrahydrofuryl (including tetrahydrofuran-2-yl, etc.), tetrahydropyranyl, piperidinyl (including 1-piperidinyl, 2- Piperidinyl and 3-piperidinyl, etc.), piperazinyl (including 1-piperazinyl and 2-piperazinyl, etc.), morpholinyl (including 3-morpholinyl and 4-morpholinyl, etc.) and so on.

Unless otherwise specified, the term "C ₁₋₆ alkyl" is used to indicate a straight or branched chain saturated hydrocarbon group consisting of 1 to 6 carbon atoms. The C ₁₋₆ alkyl includes C₁₋₅, C₁₋₄, C₁₋₃, C₁₋₂, C₂₋₆, C₂₋₄, C₆ and C₅ alkyl, etc .; it may Is monovalent (such as methyl), divalent (such as methylene) or polyvalent (such as methine). Examples of C₁₋₆ alkyl include, but are not limited to, methyl (Me), ethyl (Et), propyl (including n-propyl and isopropyl), butyl (including n-butyl, isobutyl , S-butyl and t-butyl), pentyl (including n-pentyl, isopentyl and neopentyl), hexyl and the like.

Unless otherwise specified, the term "C₁₋₃ alkyl" is used to indicate a straight or branched chain saturated hydrocarbon group consisting of 1 to 3 carbon atoms. The C₁₋₃ alkyl includes C₁₋₂ and C₂₋₃ alkyl groups, and the like; it may be monovalent (such as methyl), divalent (such as methylene), or polyvalent (such as methine). Examples of C_{1- 3} alkyl include, but are not limited to, methyl (Me), ethyl (Et), propyl (including n- propyl and isopropyl) and the like.

Unless otherwise specified, the term "C₁₋₃ alkoxy" refers to those alkyl groups containing 1 to 3 carbon atoms that are attached to the rest of the molecule through one oxygen atom. The C₁₋₃ alkoxy group includes C₁₋₂ and C₂₋₃ alkoxy, and the like. it may be monovalent (such as methyl), divalent (such as methylene), or polyvalent (such as methine). Examples of C₁₋₃ alkoxy include, but are not limited to, methoxy (Me), ethoxy (Et), propoxy (including n-propoxy and isopropoxy), and the like.

Unless otherwise specified, the term "C₁₋₆ alkoxy" refers to those alkyl groups containing 1 to 6 carbon atoms that are attached to the rest of the molecule through an oxygen atom. The C₁₋₆ alkoxy group includes C₁₋₄, C₁₋₃, C₁₋₂, C₂₋₆, C₂₋₄, C₆, C₅, C₄, and C₃ alkoxy groups and the like. Examples of C₁₋₆ alkoxy include but are not limited to methoxy, ethoxy, propoxy (including n-propoxy and isopropoxy), butoxy (including n-butoxy, isobutoxy, s-butoxy and t-butoxy), pentoxy (including n-pentoxy, isopentoxy and neopentoxy), hexyloxy and the like.

Unless otherwise specified, the term "C₄₋₆ alkoxy" refers to those alkyl groups containing 4 to 6 carbon atoms that are attached to the rest of the molecule through an oxygen atom. The C₄₋₆ alkoxy group includes C₄₋₆, C₄₋₅, C₆, C₅, C₄ alkoxy and the like. Examples of C5-6 alkoxy include but are not limited to butoxy (including n-butoxy, isobutoxy, s-butoxy and t-butoxy), pentoxy (including n-pentoxy, isopentyloxy and neopentyloxy), hexyloxy and the like.

The term "leaving group" refers to a functional group or atom that can be replaced by another functional group or atom through a substitution reaction (eg, an affinity substitution reaction). For example, representative leaving groups include triflate; chlorine; bromine; and iodine; sulfonate groups such as mesylate, tosylate, p-bromobenzenesulfonate, and p-toluenesulfonic acid esters, etc.; acyloxy, such as acetoxy; trifluoroacetoxy and the like.

The term "protecting group" includes but is not limited to "amino protecting group", "hydroxy protecting group" or "mercapto protecting group". The term "amino protecting group" refers to a protecting group suitable for preventing side reactions at the amino nitrogen position. Representative amino protecting groups include, but are not limited to: formyl; acyl, such as alkanoyl (such as acetyl, trichloroacetyl, or trifluoroacetyl); alkoxycarbonyl, such as tert-butoxycarbonyl (Boc); arylmethoxycarbonyl, such as benzyloxycarbonyl (Cbz) and 9-fluorenylmethoxycarbonyl (Fmoc); arylmethyl, such as benzyl (Bn), trityl (Tr), 1,1-di -(4'-methoxyphenyl) methyl; silyl, such as trimethylsilyl (TMS) and tert-butyldimethylsilyl (TBS) and the like. The term "hydroxy protecting group" refers to a protecting group suitable for preventing side reactions of a hydroxyl group. Representative hydroxy protecting groups include, but are not limited to: alkyl groups such as methyl, ethyl, and tert-butyl; acyl groups such as alkanoyl (such as acetyl); aryl methyl groups such as benzyl (Bn), oxybenzyl (PMB), 9-fluorenylmethyl (Fm) and diphenylmethyl (diphenylmethyl, DPM); silyl groups such as trimethylsilyl (TMS) and tert-butyl Dimethylsilyl (TBS) and the like.

The compounds of the invention can be prepared by a variety of synthetic methods well known to those skilled in the art, including the specific embodiments listed below, the embodiments formed by combining them with other chemical synthesis methods, and equivalent alternatives familiar to those skilled in the art. Preferred embodiments include, but are not limited to, the embodiments of the invention.

The solvent used in the invention is commercially available. The following abbreviations are used in the invention: aq stands for water; HATU stands for O-(7-azabenzotriazol-1-yl) -N, N, N ', N'-tetramethylurea hexafluorophosphate ; EDC stands for N- (3-dimethylaminopropyl) -N'-ethylcarbodiimide hydrochloride; m-CPBA stands for 3-chloroperoxybenzoic acid; CDI stands for Carbonyl diimidazole; DCM stands for dichloromethane; PE stands for petroleum ether; DIAD stands for diisopropyl azodicarboxylate; DMF stands for N, N-dimethylformamide; DMSO stands for dimethyl sulfoxide; EtOAc stands for ethyl acetate EtOH for ethanol; MeOH for methanol; CBz for benzyloxycarbonyl, an amine protecting group; BOC for t-butoxycarbonyl, an amine protecting group; HOAc for acetic acid; NaCNBH₃ for sodium cyanoborohydride Rt stands for room temperature; THF stands for tetrahydrofuran; Boc₂O stands for di-tert-butyl dicarbonate; TFA stands for trifluoroacetic acid; DIPEA stands for diisopropylethylamine; SOCl₂ stands for subchloride Sulfone; CS₂ for carbon disulfide; TsOH for p-toluenesulfonic acid; NFSI stands for N-fluoro-N-(benzenesulfonyl)benzenesulfonamide; *n*-Bu₄NF stands for tetrabutylammonium fluoride; iPrOH stands for 2-propanol; Pd(dppf)Cl₂ stands for 1, 1'-Bis(diphenylphosphino)ferrocene palladium(II) dichloride; DEA stands for diethylamine; mp stands for melting point; LDA stands for lithium diisopropylamine; r.t. stands for room temperature; O/N stands for overnight; Eq stands for equivalent, or equal quantity; r/min is a unit of rotation speed and revolutions per minute.

Compounds are named according to the conventional naming principles in the art or using ChemDraw^{®} software. Commercially available compounds use supplier catalog names.

### BRIEF DESCRIPTION OF THE DRAWINGS

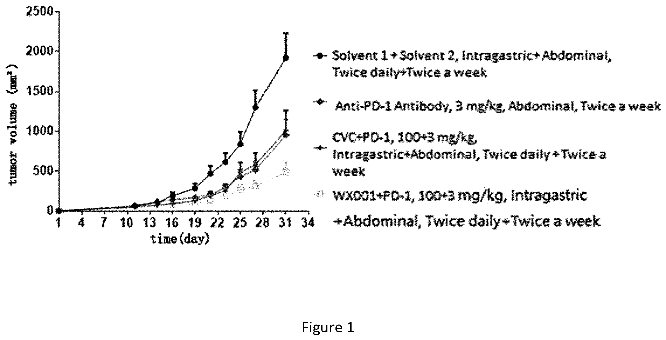

Figure 1 shows effect of WX001 on tumor volume of MC38 colon cancer tumor model of mice.

### DETAILED DESCRIPTION

The invention is as set out in the independent claims, further aspects of the invention are outlined in the dependent claims. Embodiments that do not fall within the scope of the claims do not describe part of the invention.

The invention will be described in detail below by examples, which is not intended to be inappropriate restrictions to the invention. The invention has been described in detail herein, in which embodiments of the invention are disclosed. It will be obvious to those skilled in the art that various changes and improvements can be made to the specific embodiments disclosed herein. It is therefore contemplated that the appended claims will cover any such modifications or embodiments that fall within the scope of the invention.

### Reference Example 1: Fragment BB-1A-7

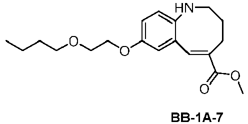

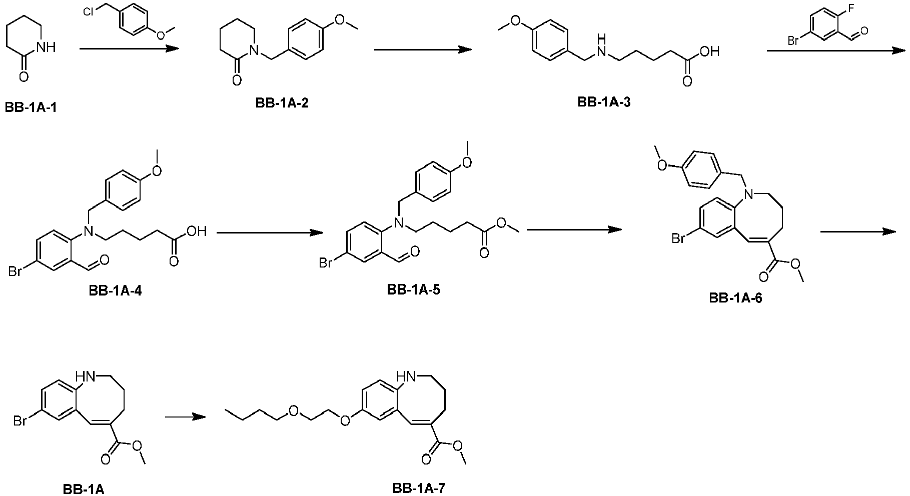

### Step 1: Synthesis of compound BB-1A-2

Tetrabutylammonium bromide (86.58 g, 302.63 mmol) and potassium hydroxide (339.6 g, 6.05 mol) were suspended in toluene (5000 mL) to form a mixture, after the mixture was refluxed for 16 hours, piperidine-2-ketone (500.00 g, 5.04 mol) and methoxy chloride (1.03 kg, 6.56 mol) was added thereto. The mixture was held at 100 °Cfor 24 hours, was cooled to room temperature, and washed three times with water (2000 mL × 3). The organic phase was dried with anhydrous sodium sulfate, filtered and concentrated. The concentrated solution is separated by a chromatographic column to obtain the compound **BB-1A-2.**

### Step 2: Synthesis of compound BB-1A-4

A solution of compound **BB-1A-2** (40 g) and sodium hydroxide (7.29 g) in water (200 mL) was stirred at 80 °C for 24 hours. The mixture was neutralized with concentrated hydrochloric acid (7.38 mL) at 0°C to obtain a mixed solution of **BB-1A-3.** To this mixed solution were added sodium carbonate (65.66 g) and dimethyl sulfoxide (600 mL) at room temperature. Then 5-bromo-2-fluoro-benzaldehyde (46.11 g) was added dropwise to the mixture at 100°C. The reaction mixture was then refluxed at 100°C for 48 hours and was adjusted to pH=7 with ammonium chloride (1 mol/L) aqueous solution under ice-cooling , and extracted with ethyl acetate (1 L×3). The organic layer was washed with water and brine, dried with anhydrous sodium sulfate and concentrated. The residue was purified by column chromatography (petroleum ether/ethyl acetate=200:1 to 1:1) to obtain the compound **BB-1A-4.**

### Step 3: Synthesis of compound BB-1A-5

To a mixture of compound BB-1A-4 (10 g) and sodium carbonate (12.61 g) in acetonitrile (100 mL) was added a solution of methyl iodide (22 g) in acetonitrile (50 mL). Under the atmosphere of nitrogen, the reaction mixture was stirred at 15-30°C for 24 hours, was quenched with water (300 mL), and extracted with ethyl acetate (400 mL×2). The organic layer was washed with brine, dried with anhydrous sodium sulfate, and concentrated. The crude product was purified by silica gel column chromatography (petroleum ether/ethyl acetate = 50:1 to 20:1 to 10:1) to obtain the compound **BB-1A-5.**

**¹H NMR** (400 MHz, CDCl₃) δ ppm 10.31 (s, 1H) 7.89 (d, *J*=2.5 Hz, 1H) 7.54 (dd, *J*=9.0, 2.51 Hz, 1H) 7.04 (d, *J*=8.5 Hz, 2H) 6.97 (d, *J*=9.0 Hz, 1H) 6.80 (d, *J*=8.5 Hz, 2H) 4.21 (s, 2H) 3.77 (s, 3H) 3.63 (s, 3H) 3.07 (t, *J*=6.8 Hz, 2H) 2.25 (t, *J*=6.8 Hz, 2H) 1.46 - 1.58 (m, 4H).

### Step 4: Synthesis of compound BB-1A-6

To a solution of compound **BB-1A-5** (11 g) in dimethyl carbonate (42.8 g) was added sodium methoxide (6.84 g) to form a mixture. The mixture was heated to 60°C and stirred at 60°C for 35 hours, adjusted to pH (7-8) with ammonium chloride in aqueous solution (1 mol/L), and filtered. The filter cake was evaporated under reduced pressure to obtain the compound **BB-1A-6.**

**¹H NMR** (400 MHz, CDCl₃) δ ppm 7.74 (s, 1H) 7.26 (s, 1H) 7.05 - 7.11 (m, 3H) 6.88 (d, *J*=8.53 Hz, 2H) 6.53 (d, *J*=9.03 Hz, 1H) 4.39 (s, 2H) 3.80 (d, *J*=2.01 Hz, 6H) 3.48 (t, *J*=5.02 Hz, 2H) 2.58 (t, *J*=6.02 Hz, 2H) 1.47 (d, *J*=5.52 Hz, 2H).

### Step 5: Synthesis of compound BB-1A

To a solution of compound **BB-1A-6** (10 g) in toluene (50 mL) was added trifluoroacetic acid (100 mL). The reaction mixture was heated to 60°C and stirred at 60°C for 8 hours, was neutralized to pH=7 with saturated sodium bicarbonate under ice cooling, and extracted with ethyl acetate (250 mL×2). The organic layer was washed with brine, dried with anhydrous sodium sulfate, and concentrated. The residue was purified by a silica gel column (petroleum ether/ethyl acetate=50:1 to 5:1) to obtain the compound **BB-1A.**

**¹H NMR** (400 MHz, CDCl₃) δ ppm 7.66 (s, 1H) 7.39-7.05 (m, 1H) 6.85 (d, *J*=8.8 Hz, 1H) 6.36 (d, *J*=8.8 Hz, 1H) 3.81 (s, 3H) 3.51 (t, *J*=5.2 Hz 2H) 2.75 (d, *J*=6.8 Hz, 2H) 1.49-1.40 (m, 2H).

### Step 6: Synthesis of compound BB-1A-7

At 25°C, **BB-1A** (7.5 g, 25.32 mmol, 1 *eq*) and **BB-2** (8.92 g, 27.86 mmol, 1.1 *eq*) were dissolved in dimethyl sulfoxide (62.5 mL) and water (12.5 mL), and then Pd(dppf)Cl₂ (926.50 mg, 1.27 mmol, 0.05 *eq*) and potassium carbonate (10.50 g, 75.97 mmol, 3 *eq*) were added thereto to form a reaction mixture. The reaction mixture was reacted at 80°C under the atmosphere of nitrogen for 18 hours, and filtered. The filtrate was added with 100 mL of water, and then extracted three times with ethyl acetate (100 mL×3). The combined organic phases are washed once with saturated brine, dried with anhydrous sodium sulfate, and filtered and evaporated under reduced pressure to obtain a crude product. The crude product was separated by column chromatography (petroleum ether: ethyl acetate EA=20:1 to 8:1) to obtain the compound **BB-1A-7** (8 g).
**MS-ESI** (m/z):410.0(M+1)⁺

### Reference Example 2: Fragment BB-1B

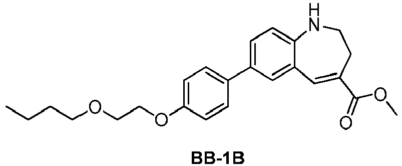

### synthetic route:

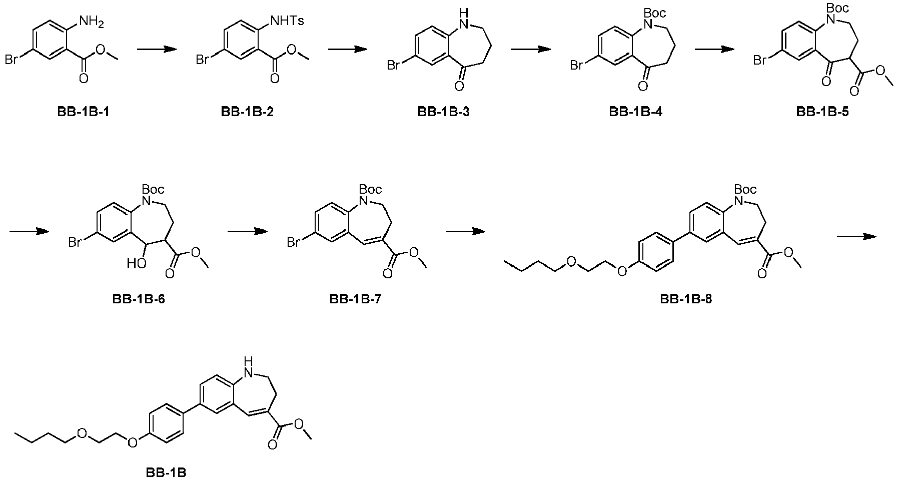

### Step 1: Synthesis of compound BB-1B-2

To a solution of compound **BB-1B-1** (45 g) in pyridine (450 mL) was added p-toluenesulfonyl chloride (55.94 g) at 25°C and stirred at 55°C for 5 hours to form a mixture. The mixture was added with water (200 mL) and extracted with ethyl acetate (100 mL×3). The combined organic layer was washed with saturated brine (200 mL), dried with anhydrous sodium sulfate, filtered and evaporated under reduced pressure to obtain a residue. The residue was purified by a silica gel column (petroleum ether/ethyl acetate = 100:1 to 10:1) to obtain **BB-1B-2.**
**¹H NMR** (400MHz, CDCl₃) δ ppm 10.51 (s, 1H), 8.04 (d, *J*=2.3 Hz, 1H), 7.73 (d, *J*=8.5 Hz, 2H), 7.63 - 7.59 (m, 1H), 7.57 - 7.52 (m, 1H), 7.24 (d, *J*=8.0 Hz, 2H), 3.89 (s, 3H), 2.38 (s, 3H)

### Step 2: Synthesis of compound BB-1B-3

Sodium hydrogen (6.14g, 60% purity) and DMF (50 mL) were added to a solution of BB-1B-2 (59 g) in DMF (200 mL) at 0 °C , and then stirred at 25 °C under the atmosphere of nitrogen for 2 hours, sodium iodide (23.02 g) and ethyl 4-bromobutyrate (32.95 g) were added thereto at 0 °C and stirred at 80 °C under the atmosphere of nitrogen for 16 hours, and then sodium hydrogen (6.14 g, 60% purity) and DMF (50 mL) were added thereto at 0°C and stirred at 80°C under the atmosphere of nitrogen for 15 hours. The mixture was added with water (200 mL) and extracted with ethyl acetate (50 mL×3). The combined organic layer was washed with saturated brine (200 mL), dried with anhydrous sodium sulfate, and filtered and evaporated under reduced pressure to obtain a yellow oil. Concentrated sulfuric acid (100 mL, 98% purity) and acetic acid (157.5 g) were add to the yellow oil at 0° and stirred at 90°C for 2.5 hours. The mixture was adjusted to pH = 8 with 12 mol/L sodium hydroxide in aqueous solution and then extracted with ethyl acetate (300 mL×3), and washed with saturated brine (300 mL) to combine organic layers. The combined organic layer was dried with anhydrous sodium sulfate, and filtered and evaporated under reduced pressure to obtain a residue. The residue was purified with a silica gel column (petroleum ether/ethyl acetate = 50:1 to 5:1) to obtain **BB-1B-3.**

### Step 3: Synthesis of compound BB-1B-4

The reactants **BB-1B-3** (6 g) and Boc anhydride (15.16 g) were dissolved in tetrahydrofuran (100 mL), and N,N-lutidine (4.58 g) was added to form a mixture. The mixture was stirred at 70°C for 4 hours, quenched with water (100 mL) and was extracted with ethyl acetate (100 mL×3). The combined organic layer was washed with saturated brine (100 mL), dried with anhydrous sodium sulfate, filtered and evaporated under reduced pressure to obtain a residue. The residue was purified by silica gel column chromatography (petroleum ether/ethyl acetate = 50:1 to 20:1) to obtain **BB-1B-4.**
**MS-ESI** (m/z): 283.9[M-55]⁺

### Step 4: Synthesis of compound BB-1B-5

Sodium methoxide (2.13 g) was added to a solution of reactant BB-1B-4 (2.68 g) in dimethyl carbonate (50 mL) at 25°C, and stirred at 100°C under the atmosphere of nitrogen for 2 hours. The mixture was quenched with water (50 mL) and extracted with ethyl acetate (100 mL×3). The combined organic layer was washed with saturated brine (100 mL), dried with anhydrous sodium sulfate, filtered and evaporated under reduced pressure to obtain a residue. The residue was purified by silica gel column (petroleum ether/ethyl acetate=50:1 to 5:1) to obtain compound **BB-1B-5.**

### MS-ESI (m/z): 342.1[M-55]⁺

### Step 5:Synthesis of compound BB-1B-6

Sodium borohydride (484.49 mg) was added to a solution of reactant BB-1B-5 (1.7 g) in tetrahydrofuran (20 mL) at -40°C and methanol (10 mL) was added thereto. The mixture was stirred at -15°C for 0.5 hour, quenched with water (50 mL), and extracted with ethyl acetate (100 mL×3). The combined organic layer was washed with saturated brine (100 mL), dried with anhydrous sodium sulfate, filtered and evaporated under reduced pressure to obtain the compound **BB-1B-6.**

### Step 6 :Synthesis of compound BB-1B-7

The reactants BB-1B-6 (1.93 g) and triethylamine (1.56 mL) were dissolved in a tetrahydrofuran (20 mL) solution, and methanesulfonyl chloride (1.29 g) was added thereto at 0°C. The mixture was stirred at room temperature for 15 hours under the atmosphere of nitrogen, and then 1,8-diazacyclo[5,4,0]-undecene (1.14 g) was added thereto, and stirred at room temperature for 1 hour and quenched with water (50 mL) and extracted with ethyl acetate (100 mL×3). The combined organic layer was washed with saturated brine (100 mL), dried with anhydrous sodium sulfate, filtered and evaporated under reduced pressure. The residue was purified by silica gel column chromatography (petroleum ether/ethyl acetate = 50:1 to 5: 1) to obtain the compound **BB-1B-7.**
**MS-ESI** (m/z): 326.0[M-55]⁺

### Step 7: Synthesis of compound BB-1B-8

Under the atmosphere of nitrogen, the reactants **BB-1B-7** (0.35 g), **BB-2** (351.86 mg) and potassium carbonate (379.64 mg) were added to a solution of dimethyl sulfoxide (10 mL) and water [1,1 '-Bis(diphenylphosphine)ferrocene]dichloride palladium dichloromethane complex (74.77 mg), and then the mixture was stirred at 80°C under nitrogen protection for 16 hours. Water (50 mL) was added to the mixture and extracted with ethyl acetate (50 mL×3). The combined organic layer was washed with brine (100 mL), dried over anhydrous sodium sulfate, filtered and evaporated under reduced pressure. The residue was passed through Purification by preparative thin layer chromatography (PE:EA = 5:1) gave compound BB-1B-8.

**¹H NMR** (400MHz, CDCl₃) δ ppm 7.74 (s, 1H), 7.58 (s, 1H), 7.51 (br d, *J*=8.8 Hz, 3H), 7.48 (br s, 1H), 7.01 (d, *J*=8.8 Hz, 2H), 4.20-4.16 (m, 2H), 3.85-3.80 (m, 5H), 3.56 (t, *J*=6.7 Hz, 2H), 2.92 (s, 2H), 2.05 (s, 1H), 1.66-1.58 (m, 2H), 1.56 (s, 9H), 1.45-1.35 (m, 4H), 0.94 (t, *J*=7.4 Hz, 3H).

### Step 8: Synthesis of compound BB-1B

To a solution of compound BB-1B-8 (0.23 g) in ethyl acetate (10 mL) was added a solution of 4M hydrochloric acid ethyl acetate(10 mL), and stirred at room temperature for 1 hour. The reaction solution was adjusted to pH 8 with a saturated aqueous solution of sodium bicarbonate and extracted with ethyl acetate (100 mLX 3). The combined organic layer was washed with saturated brine (100 mL), dried with anhydrous sodium sulfate, filtered and evaporated under reduced pressure, to obtain the compound **BB-1B.**
**MS-ESI** (m/z): 396.3[M+1] ⁺

### Reference Example 3: Fragment BB-1C

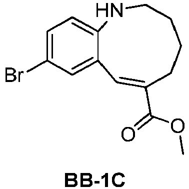

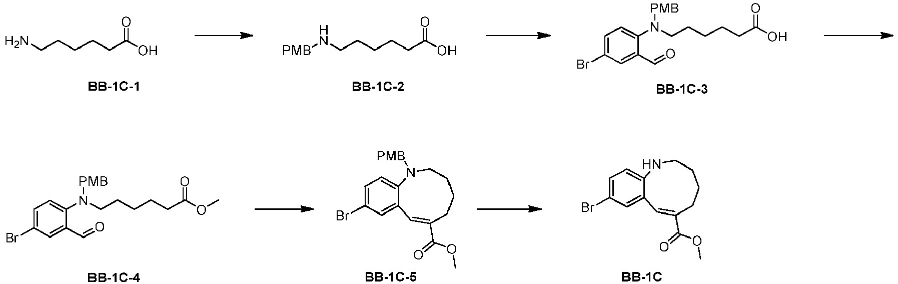

### Step 1: Synthesis of compound BB-1C-2

To 1M NaOH aqueous solution (381.18 mL) were added methanol (600 mL), 6-aminocaproic acid (50 g) and 4-methoxybenzaldehyde (52 g), and palladium on carbon (4.5 g, 10% purity) was added thereto. The reaction mixture was stirred at room temperature under the atmosphere of hydrogen (137895 -206843 Pa, i.e., "20-30 psi") for 24 hours, and filtered and evaporated under reduced pressure, acetone (300 mL). The residue was added with acetone (50 mL) and stirred at room temperature for 30 minutes, filtered and washed with acetone. The filter cake was vacuum dried to obtain the compound **BB-1C-2.**
**MS-ESI** *m*/*z*: 252.3 [M+H]⁺

### Step 2: Synthesis of compound BB-1C-3

**BB-1C-2** (86 g, 342.19 mmol, 1 equivalent) and 5-bromo-2-fluoro-benzaldehyde (55.57 g) were dissolved in dimethyl sulfoxide (350 mL), and sodium carbonate (90.67 g) ) and water (175 mL) were added thereto. The reaction solution was stirred at 100°C for 12 hours, and was poured into water (1200 mL) and adjusted to pH = 3-4 with concentrated hydrochloric acid, and extracted with ethyl acetate (500 mL×2), The combined organic layer was washed with saturated brine (500 mL) and dried with anhydrous sulfuric acid sodium, and filtered and concentrated. The residue was subjected to silica gel column chromatography (petroleum ether/ethyl acetate = 10/1 to 1/1) to obtain the compound **BB-1C-3.**
**MS-ESI** *m*/*z:* 434.2 [M+H]⁺

### Step 3: Synthesis of compound BB-1C-4

To the N,N-dimethylformamide (300 mL) solution of **BB-1C-3** (58 g) was added methyl iodide (15.49 mL) and potassium carbonate (36.91 g). The reaction solution was stirred at 25°C for 4 hours and poured into water (1000 mL), and extracted with ethyl acetate (500 mL×2). the combined organic layer was washed with saturated brine (500 mL), dried with anhydrous sodium sulfate, and filtered and concentrated. The residue was subjected to silica gel column chromatography (petroleum ether/ethyl acetate = 10/1 to 7/3) to obtain the compound **BB-1C-4.**

**¹H NMR** (400MHz, CDCl₃) δ ppm 10.31 (s, 1H), 7.90 (d, *J* = 2.4 Hz, 1H), 7.54 (dd, *J* = 8.8, 2.4 Hz, 1H), 7.06 (d, *J*=8.8 Hz, 2H), 6.98 (d, *J*=8.8 Hz, 1H), 6.81 (d, *J*=8.8 Hz, 2H), 4.22 (s, 2H), 3.79 (s, 3H), 3.65 (s, 3H), 3.06 (t, *J*=7.6 Hz, 2H), 2.25 (t, *J*=7.6 Hz, 2H), 1.58-1.51 (m, 4H), 1.27-1.25 (m, 2H).
**MS-ESI** *m*/*z:* 448.2 [M+H]⁺

### Step 4: Synthesis of compound BB-1C-5

Sodium methoxide (6.51 g) was added to a solution of **BB-1C-4** (10.8 g) in diethyl carbonate (500 mL), and was stirred at 25°C for 12 hours. The reaction solution was added with water (500 mL) and extracted with ethyl acetate (300 mL×2). The combined organic layer was washed with brine (500 mL), dried with anhydrous sodium sulfate, filtered and concentrated. The residue was subjected to silica gel Column chromatography (PE / EtOAc = 10/1) to obtain the compound **BB-1C-5.**
**MS-ESI** *m*/*z*: 430.2, 432.2 [M+1, M+3]⁺

### Reference Example 4: Fragment BB-2

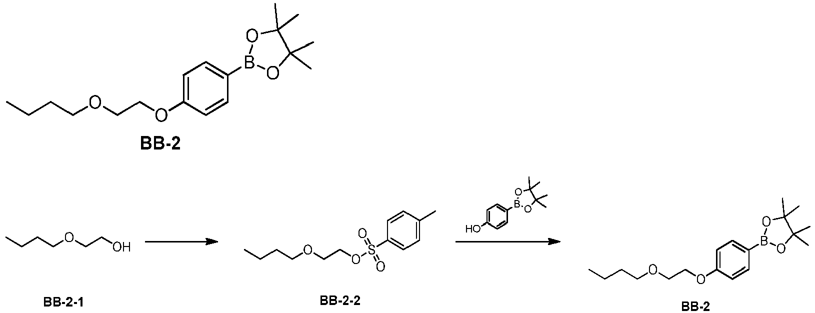

### Step 1: Synthesis of compound BB-2-2

The compound **BB-2-1** (100 g, 0.846 mol), p-toluenesulfonyl chloride (146.67 g, 769.31 mmol), and triethylamine (233.54 g, 2.31 mol) were dissolved in dichloromethane (0.5 L), and stirred at room temperature for 12 hours, and cooled to room temperature and filtered under reduced pressure to remove the solvent. the residue was dissolved in water (400 mL) and was extracted three times with ethyl acetate (1.5 L). The combined organic phase was washed twice with saturated brine (400 mL), dried with anhydrous sodium sulfate, and evaporated under reduced pressure after filtering off the desiccant to remove the solvent, in order to obtain the compound BB-2-2.

**¹H NMR** (400 MHz, CDCl₃) δ ppm 7.73 (d, *J*=8.28 Hz, 2H) 7.27 (d, *J*=8.03 Hz, 2H) 4.05-4.10 (m, 2H) 3.50-3.55 (m, 2H) 3.30 (t, *J*=6.53 Hz, 2H) 2.37 (s, 3H) 1.35-1.44 (m, 2H) 1.16-1.27 (m, 2H) 0.81 (t, *J*=7.40 Hz, 3H).

### Step 2: Synthesis of the compound BB-2.

Compound **BB-2-2** (170.10 g, 624.54 mmol), p-hydroxyphenylboronic acid pinacol ester (137.44 g, 624.54 mmol) was dissolved in acetonitrile (1.6 L), and potassium carbonate (86.32 g, 624.54mmol) and potassium iodide (10.37 g, 62.45 mmol) were added thereto at room temperature. The resulting solution was heated to reflux and stirred for 12 hours under nitrogen atmosphere at 60°C, and filtered under reduced pressure after cooling to room temperature to remove the solvent. The residue was dissolved in water (500 mL) and the aqueous phase was extracted three times with ethyl acetate (2 L×3), combined and then filtered under reduced pressure remove the solvent, and subjected to by column chromatography to obtain the compound **BB-2.**

**¹H NMR** (400MHz, CDCl₃) δ ppm 7.78-7.71 (m, 2H), 6.95-6.88 (m, 2H), 4.19-4.12 (m, 2H), 3.83-3.75 (m, 2H), 3.54 (t, *J*=6.8 Hz, 2H), 1.65-1.57 (m, 2H), 1.44-1.36 (m, 2H), 1.34 (s, 12H), 0.93 (t, *J*=7.3 Hz, 3H) ₒ

### Reference Example 5: Fragment BB-4A, and fragment BB-4B

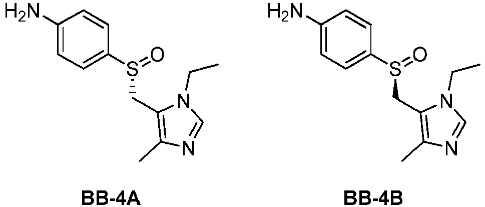

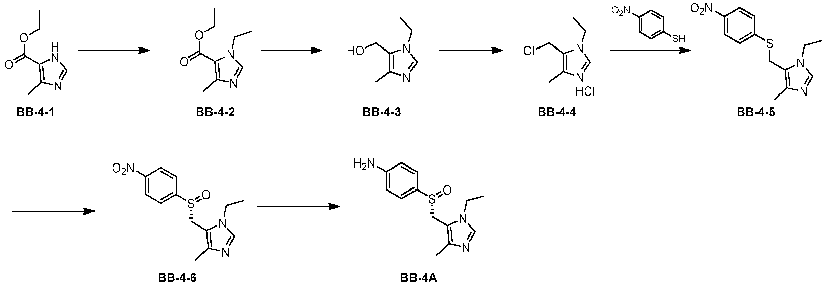

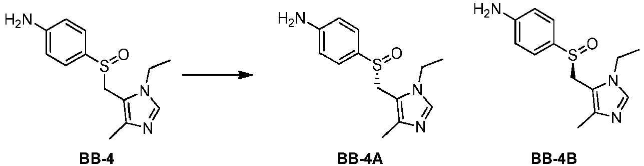

### Step 1: Synthesis of compound BB-4-2

Triphenylphosphine (765.61g, 1.5eq) was dissolved in 3.5 L of anhydrous tetrahydrofuran under mechanical stirring (219 r/min) in an ice bath, and DIAD (567.54 mL) was slowly added dropwise within 100 minutes thereto with controlling the internal temperature below 10 °C, and then continually stirred for 30 minutes at an increased speed of 400 r/min such that the reaction was allowed to proceed sufficiently to form a white viscous solid. 453.91 mL of absolute ethanol was continually and slowly added in an ice bath within 90 minutes e (the reaction exotherm is violent at the beginning of dripping, it is important to notice the dripping speed). BB- 4-1 (300 g) was added when the solid was slowly dissolved to form a clear solution. The solution was mechanically stirred at 200 r/min for 5 hours under the condition that the ice bath was replaced with an oil bath to control the temperature of 30-35°C (when the temperature exceeds 40°C, the selectivity is poor and the by-products increase).The reaction solution was placed under reduced pressure at 40°C to give tetrahydrofuran, adjusted to pH 2 with 4mol/L aqueous hydrochloric acid solution (700 mL) , and added with 2 L of ethyl acetate solution after being fully stirred for 30 min for extraction. After carrying out extraction for 5 times and observing a clean aqueous phase by TLC plate and LCMS, the organic phase was discarded. the aqueous phase was added with 65 g of NaOH and stirred for 40 min, and adjusted to pH above 10 (the reaction exotherm is violent, it is important to notice the temperature) , and extracted with 3 L of ethyl acetate as a solvent. After repeating extraction for 3 times and observing a clean organic phase by TLC plate and LCMS, the organic phases were combined and dried with 40 g of anhydrous sodium sulfate, and filter and evaporated under reduced pressure to remove ethyl acetate, in order to obtain BB-4-2.
**¹H NMR** (400MHz, CDCl₃) δ ppm 7.46 (s, 1H), 4.41 - 4.24 (m, 4H), 2.48 (s, 3H), 1.39 (q, *J*=7.4 Hz, 6H)
**MS-ESI** (m/z): 182.9 [M+H]⁺

### Step 2: Synthesis of compound BB-4-3

Tetrahydroaluminum lithium (59.36 g) was weighed, added to 1 L of anhydrous tetrahydrofuran solution in an ice bath, and mechanically stirred at a rotational speed (289 r/min) to form a solution. The compound **BB-4-2** (285 g) was dissolved in 1L of anhydrous tetrahydrofuran, and slowly dripped into the above solution in an ice bath at the controlled temperature below 10 °C within 1.5 hours. The solution was continually and mechanically stirred at the unchanged speed at room temperature for 12 hours under the condition that the ice bath was removed. 60 mL of deionized water was slowly added into the solution with mechanical stirring in an ice bath until the reaction was complete. The reaction product was slowly added dropwise with sodium hydroxide aqueous solution (4mol/L, 60 mL) and stirred for 15 minutes, added with 180 mL of deionized water and continually stirred for 20 minutes, and then added with 80 g of anhydrous magnesium sulfate and stirred well, and finally filtered under reduced pressure. The filter cake was repeatedly washed 5-6 times with 5 L of dichloromethane, and the filtrate was combined and evaporated under reduced pressure at 40°C to obtain **BB-4-3.**

**¹H NMR** (400MHz, CDCl₃) δ ppm 7.51 (s, 1H), 4.82 (s, 3H), 4.27 (q, *J*=7.3 Hz, 2H), 2.33 (s, 3H), 1.69 (t, *J*=7.3 Hz, 3H).

### Step 3: Synthesis of compound BB-4-4

Compound BB-4-3 (180g) was dissolved in 1.5 L of anhydrous dichloromethane and stirred magnetically, and thionyl chloride (186.3 mL) was slowly added dropwise thereto in an ice bath to form a mixture. After the addition, the mixture was heated to 40 °C under the condition that the ice bath was replaced with an oil bath and stirred for 6 hours under nitrogen atmosphere until the reaction was complete by detection of TLC plate. The reaction solution was cooled to room temperature, and evaporated under reduced pressure at 40 °C to remove the solvent. Dichloromethane (1L) was added to the evaporated residue and distilled under reduced pressure which is repeated three times, and rotarily evaporated under reduced pressure for 45 min under the condition that the water pump was changed to an oil pump to obtain 190 g of a crude product of red viscous solid. The crude product was dissolved by adding 380 mL of acetonitrile solution, heating to 70 °C and refluxing for 2 hours, and cooled to room temperature by turning off the heating device and continually stirred for 12 hours. The mixed solution was slowly added dropwise to 3 L of ethyl acetate under stirring and the stirring was kept for 30 minutes after the addition was completed. The compound **BB-4-4** was obtained by suction filtration under reduced pressure.

**1H NMR** (400MHz, DMSO-d6) δ ppm 9.19 (s, 1H), 5.06 (s, 2H), 4.24 - 4.20 (m, 2H), 2.34 (s, 3H), 1.45 (t, *J*=7.3 Hz, 3H).

### Step 4: Synthesis of compound BB-4-5

p-fluoronitrobenzene (97.64 g) was weighed and dissolved in dimethyl sulfoxide (1.1 L), and sodium sulfide (59.38 g) was added thereto under stirring in an ice bath, and stirred for 44 hours to formed a reaction solution. The compound BB-4-4 (90 g) was dissolved in DMSO (450 mL) and slowly dropped into the reaction solution within 7 hours. Then, the reaction solution was adjusted to pH 3 by addition of aqueous hydrochloric acid solution (4M, 0.5 L), extracted with 4 L of water and 4 L of ethyl acetate, and stirred for half an hour. After the phage separation, the aqueous phase is adjusted to pH 10 by addition of solid sodium hydroxide and extracted with 2 L of ethyl acetate twice. The twice extracted organic phases were combined and washed with 1 L of saturated brine, and evaporated. The organic phase was dried by adding anhydrous sodium sulfate, filtered, and evaporated. The spin-dried solid was crushed, and added with 2 mL of ethyl acetate and 100 mL of petroleum ether, stirred at room temperature for 1 hour, and filtered. The filter cake was dried under reduced pressure to obtain **BB-4-5.**

**¹H NMR** (400 MHz, CDCl₃) δ ppm 8.07 (d, *J*=8.78 Hz, 2H) 7.37 (s, 1H) 7.30 (d, *J*=8.78 Hz, 2H) 4.13 (s, 2 H) 3.92 (q, *J*=7.28 Hz, 2H) 2.06 (s, 3H) 1.40 (t, *J*=7.28 Hz, 3H).

### Step 5: Synthesis of compound BB-4-6

**BB-4-5** (64.72 g) was added to a solution of (1R, 2R)-1,2-diphenylethane-1,2-diol (100 g) in isopropanol (5000 mL), and stirred at 25°C for 1 hour to form a mxiture. Then, the mixture was added dropwise with titanium tetraisopropoxide (66.32 g) and stirred at 25°C for 3 hours, and filtered to give a yellow solid (95 g). The obtained solid (72 g) was dissolved in dichloromethane (360 mL), and tert-butanol peroxy (14.17 mL, 65% purity) was added to the solution and was stirred at room temperature for 4 hours under nitrogen atmosphere. Then, the reaction solution was quenched with saturated aqueous sodium sulfite (200 mL), and adjusted to pH 3 with 2M aqueous hydrochloric acid. After the phage separation, the organic phase was discarded and the aqueous phase was adjusted to pH 8 with 8M of sodium hydroxide aqueous solution. The aqueous phase was extracted with ethyl acetate (300 mL×3). The combined organic phase was washed with saturated brine (300 mL), dried, filtered and evaporated under vacuum to obtain **BB-4-6** (crude).

### Step 6: Synthesis of compound BB-4A

To a solution of compound **BB-4-6** (19 g) and ammonium chloride (34.65 g) in methanol (250 mL) was added zinc powder (42.35 g) and stirred at 20°C for 24 hours. After filtration, the filtrate was evaporated under reduced pressure to obtain a residue. The residue was purified by preparative high performance liquid chromatography to obtain the compound **BB-4A.**
**¹H NMR** (400MHz, CDCl₃) δ ppm 7.41 (s, 1H), 7.14-7.09 (m, *J*=8.5 Hz, 2H), 6.71-6.66 (m, *J*=8.5 Hz, 2H), 4.07-3.94 (m, 4H), 3.84 (dd, *J*=1.9, 7.4 Hz, 2H), 1.69 (s, 3H), 1.38 (t, *J*=7.3 Hz, 3H)
**MS-ESI** (m/z):263.7(M+H)⁺

### Phase separation: Synthesis of compound BB-4A and BB-4B

The compound BB-4 (prepared by referring to the synthesis of compound BB-3I in WO2018103757A1) was subjected to supercritical fluid chromatography (separation conditions: Column: ChiralPaK AD-3 150*4.6mm ID, 3µm; mobile phase: A: CO₂ B: ethanol (0.05%DEA); gradient: 5% -40% B, 5.5min, keeping 40% for 3min and then keeping 5% B for 1.5 min; flow rate: 2.5mL/min; column temperature: 40°C; wavelength: 220nm) to obtain the isomers BB-4A (retention time:5.828min) and BB-4B (retention time:6.163min).

**¹H NMR** (400MHz, CDCl₃) δ ppm 7.43 (s, 1H), 7.15-7.10 (m, 2H), 6.71-6.66 (m, 2H), 4.07-3.95 (m, 4H), 3.85 (dd, *J*=1.9, 7.3 Hz, 2H), 1.70 (s, 3H), 1.39 (t, *J*=7.3 Hz, 3H). **MS-ESI** *m*/*z*: 264.0 [M+H] ⁺.

### Example 1

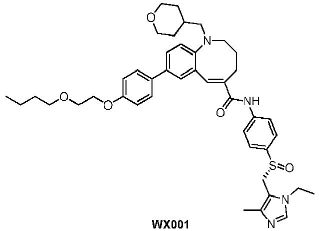

### Synthetic route:

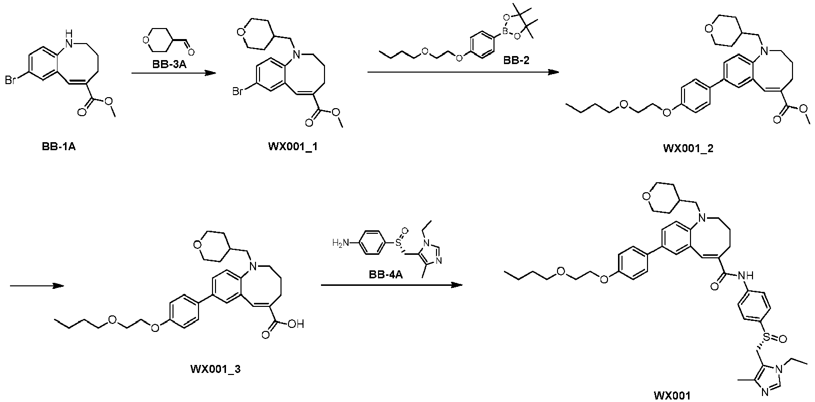

### Step 1: Synthesis of compound WX001_1

The compound BB-1A (3.89 g, 13.14 mmol) and BB-3A (3 g, 26.28 mmol) were dissolved in 1,2-dichloroethane (50 mL) at 25°C, and sodium triacetoxyborohydride (11.14 g, 52.57 mmol) was added thereto and was stirred at 25°C for 5 hours to form a reaction solution. The reaction solution was adjusted to pH 8 with 8M sodium hydroxide aqueous solution, and extracted with ethyl acetate (100 mL×3). The organic phases were combined, washed with 100 mL saturated brine once, dried with anhydrous sodium sulfate, and filtered to remove the desiccant. The filtrate was evaporated under reduced pressure to obtain the compound **WX001_1.**
**MS-ESI** (m/z):394.20[M+1]⁺
**¹H NMR** (400MHz, CDCl₃) δ = 7.69 (s, 1H), 7.24 (s, 1H), 7.23 - 7.20 (m, 1H), 6.61 (d, J=9.5 Hz, 1H), 4.00 (dd, J=4.4, 10.9 Hz, 2H), 3.80 (s, 3H), 3.44 - 3.32 (m, 5H), 3.07 (d, J=7.0 Hz, 2H), 2.54 - 2.48 (m, 2H), 2.05 - 1.98 (m, 2H), 1.65 (br d, J=12.3 Hz, 2H), 1.46 - 1.41 (m, 2H).

### Step 2: Synthesis of compound WX001_2

At 25°C, the compound **WX001_1** (0.6 g, 1.52 mmol), the compound **BB-2** (487.29 mg, 1.52 mmol) and anhydrous potassium carbonate (630.91 mg, 4.57 mmol) were dissolved in a mixed solvent of dimethyl sulfoxide (10 mL) and water (2 mL), and Pd(dppf)Cl2 (222.68 mg, 304.34 µmol) was added thereto to form a reaction solution. The reaction solution was stirred for 20 hours under nitrogen atmosphere at 80°C and poured into water (50 mL), and extracted with ethyl acetate (30 mL×3). The organic phases were combined and washed with saturated brine (100 mL) once, dried with anhydrous sodium sulfate, and filtered to remove the desiccant. The filtrate was evaporated under reduced pressure to obtain a crude product. The crude product was separated by column chromatography to obtain **WX001_2** (0.603 g).
**MS-ESI** (m/z):508.20[M+1]⁺
**¹H NMR** (400MHz, CDCl₃) δ = 7.88 (s, 1H), 7.45 (d, J=8.8 Hz, 2H), 7.40 (dd, J=2.4, 8.9 Hz, 1H), 7.34 (d, J=2.3 Hz, 1H), 6.96 (d, J=8.8 Hz, 2H), 6.82 - 6.77 (m, 1H), 4.18 - 4.14 (m, 2H), 4.01 (dd, J=3.8, 11.0 Hz, 2H), 3.83 - 3.78 (m, 7H), 3.56 (t, J=6.8 Hz, 2H), 3.48 (br t, J=5.3 Hz, 2H), 3.38 (t, J=11.0 Hz, 2H), 3.14 (d, J=7.0 Hz, 2H), 2.58 - 2.53 (m, 2H), 1.70 (br d, J=13.1 Hz, 2H), 1.65 - 1.60 (m, 2H), 1.51 - 1.44 (m, 3H), 1.29 - 1.26 (m, 2H), 0.94 (t, J=7.3 Hz, 3H).

### Step 3: Synthesis of compound WX001_3

The compound **WX001_2** (0.603 g, 872 µM) was dissolved in methanol (10 mL) and water (2 mL) at 25°C and sodium hydroxide (520.50 mg, 13.01 mmol) was added thereto to form a reaction solution. The reaction solution was heated to 50°C and stirred for 43 hours, and adjusted to pH 3 with 2M dilute hydrochloric acid solution, and extracted with ethyl acetate (30 mL×3). The organic phases were combined , washed with saturated brine (100 mL) once, dried with anhydrous sodium sulfate, and filtered to remove the dryness The filtrate was concentrated under reduced pressure and evaporated to remove the desiccant to obtain **WX001_3** (0.537 g).
**MS-ESI** (m/z):494.20[M+1]⁺

### Step 4: Synthesis of compound WX001

At 25°C, the compound WX001_3 (0.1 g, 202.58 µmοl) and the compound BB-4A (53.35 mg, 202.58 µmοl) were dissolved in pyridine (5 mL) , and EDCI (77.67 mg, 405.16 µmοl) was added thereto to form a reaction solution. The reaction solution was heated to 60°C and stirred for 6 hours under nitrogen atmosphere, and poured into water (50 mL), and extracted with ethyl acetate (30 mL×3). The organic phases were combined, washed with saturated brine (100 mL) once, dried with anhydrous sodium sulfate, and filter to remove the desiccant. The filtrate was evaporated under reduced pressure to remove the solvent, in order to obtain the crude product. The crude product was separated by preparative HPLC (alkaline) to obtain **WX001** (0.032 g).
**MS-ESI** (m/z):739.90[M+1]⁺.
**¹H NMR** (400MHz, CDCl₃) δ = 7.92 (br s, 1H), 7.75 (d, J=8.8 Hz, 2H), 7.54 (s, 1H), 7.48 - 7.41 (m, 4H), 7.34 (s, 2H), 7.32 (s, 1H), 6.98 (d, J=8.8 Hz, 2H), 6.84 (d, J=8.8 Hz, 1H), 4.18 - 4.14 (m, 2H), 4.06 (s, 2H), 4.05 - 3.99 (m, 2H), 3.92 - 3.84 (m, 2H), 3.83 - 3.79 (m, 2H), 3.59 - 3.50 (m, 4H), 3.39 (br t, J=11.0 Hz, 2H), 3.17 (br d, J=7.0 Hz, 2H), 2.61 (br s, 2H), 2.10 (br s, 1H), 1.72 (br s, 1H), 1.69 (s, 4H), 1.66 - 1.63 (m, 2H), 1.46 - 1.42 (m, 2H), 1.42 - 1.36 (m, 7H), 0.94 (t, J=7.3 Hz, 3H).

### Example 18

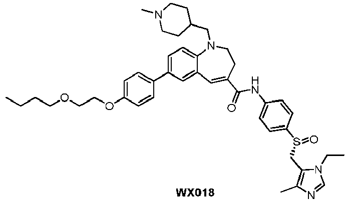

### Synthetic route:

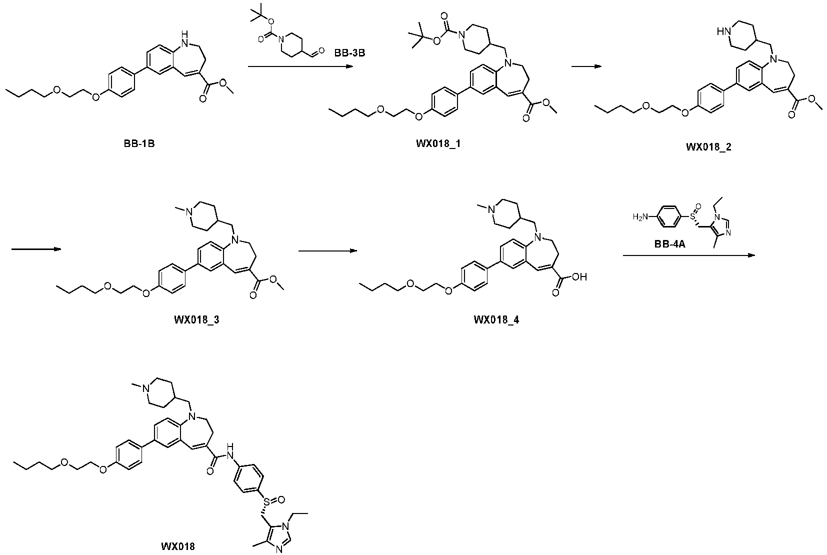

### Step 1: Synthesis of compound WX018_1

At 25°C, the compound **BB-1B** (200 mg, 937.77 µmοl) was dissolved in dichloromethane (5 mL) to form a solution. The compound **BB-3B** (370.88 mg, 937.77 µmοl), zinc chloride (63.91 mg, 468.88 µmοl, 21.96 µL) and magnesium sulfate (225.75 mg, 1.88 mmol) were added to the solution and stirred for 2 hours, and sodium acetate borohydride (596.25 mg, 2.81 mmol) was added to form a reaction solution. The reaction solution was stirred for 12 hours under nitrogen atmosphere and added with water (50 mL), and extracted with dichloromethane (30 mL×2). The organic phases were combined and dried with anhydrous sodium sulfate. The organic phase was spin-dried, and separated and purified by a silica gel column (silica gel, petroleum ether/ethyl acetate=10/1 to 3/1) to obtain **WX018_1** (360 mg). **MS-ESI** (m/z):615.4[M+23] ⁺

**¹H NMR** (400 MHz, CDCl₃) δ ppm 7.77 (s, 1H), 7.53 (d, J=2.26 Hz, 1H), 7.47 (d, J=8.53 Hz, 2H), 7.41 (dd, J=8.78, 2.26 Hz, 1H), 6.99 (d, J=8.53 Hz, 2H), 6.88 (d, J=8.53 Hz, 1H), 4.23 - 4.06 (m, 4H), 3.84 - 3.80 (m, 5H), 3.56 (t, J=6.65 Hz, 2H), 3.35 - 3.22 (m, 4H), 2.82 (br s, 2H), 2.73 - 2.60 (m, 2H), 1.89 (br s, 1H), 1.74 (br d, J=12.05 Hz, 2H), 1.66 - 1.59 (m, 2H), 1.46 (s, 9H), 1.44 - 1.36 (m, 2H), 1.14 (br d, J=10.54 Hz, 2H), 0.94 (t, J=7.40 Hz, 3H).

### Step 2: Synthesis of compound WX018_2

The compound **WX018_1** (360 mg, 607.32 µmοl) was dissolved in hydrochloric acid/ethyl acetate (4 M, 5 mL), and stirred at 25°C for 0.5 hours to form a reaction. The reaction solution was adjusted to pH 8 with saturated sodium carbonate solution, and added with water (20 mL), and extract with ethyl acetate (20 mL×2). the organic phases were combined,washed with saturated brine (20 mL), dried with anhydrous sodium sulfate and filtered. The filtrate was evaporated to obtain compound **WX018_2** (300 mg).

### Step 3: Synthesis of compound WX018_3

Aqueous formaldehyde solution (45.34 µL, concentration: 37%) was added to a solution of compound WX018_2 (300 mg, 608.95 µmοl) in methanol (3 mL) and dichloromethane (3 mL) and stirred for 30 minutes, and sodium acetate borohydride ( 193.59 mg, 913.43 µmοl) was added and stirred at 25°C for 12 hours to from a reaction solution. The reaction solution was evaporated to dryness, and added with water (20 mL), and extracted with ethyl acetate (20 mL×2). The organic phases were combined, washed with saturated brine (20 mL), and dried with anhydrous sodium sulfate. After filtration, the filtrate was evaporated to obtain the **compound** WX018_3 (254 mg).
**MS-ESI** (m/z):507.1[M+1]⁺

### Step 4: Synthesis of compound WX018_4

At 25°C, sodium hydroxide (80.20 mg, 2.01 mmol) was added to a solution of compound **WX018_3** (254 mg, 501.31 µmοl) in ethanol (5 mL) and water (5 mL), and stirred at 50°C for 4 hours to form a reaction solution. The reaction solution was evaporated to remove ethanol, adjusted to pH 3 with 3N HCl, added with water (30 mL), and extracted with ethyl acetate (30 mL×2). The organic phases were combined, washed with saturated brine (50 mL), dried with anhydrous sulfuric acid, and filtered. The filtrate was evaporated to obtain the compound **WX018_4** (212 mg).
**MS-ESI** (m/z):493.0[M+1]⁺

### Step 5: Synthesis of compound WX018

At 25°C, the compound **WX018_4** (113.33 mg, 430.33 µmοl) was added to a solution of BB-4A (212 mg, 430.33 µmοl) in pyridine (5 mL), and 1-(3-dimethylaminopropyl)-3 -ethylcarbodiimide hydrochloride (82.49 mg, 430.33 µmοl) was add and stirring at 60°C for 12 hours form a reaction solution. The reaction solution was added with water (20 mL) , and extracted with ethyl acetate (20 mL×2). The organic phases were combined, and washed with saturated brine (50 mL), dried with anhydrous sodium sulfate, and filtered and evaporated to give a crude product. The crude product was separated and purified by preparative HPLC (alkaline) to obtain **WX018 (20 mg).**
**MS-ESI** (m/z):738.3[M+1]⁺

**¹H NMR** (400 MHz, CDCl₃) δ ppm 8.44 (br s, 1H), 7.63 (d, J=8.78 Hz, 2H), 7.34 - 7.26 (m, 6H), 7.17 - 7.13 (d, J=8.78 Hz, 2H), 6.83 (m, 2H), 6.75 (d, J=8.53 Hz, 1H), 4.05 - 3.96 (m, 2H), 3.91 - 4.81 (m, 2H), 3.72- 3.64 (m, 4H), 3.42 (t, J=6.65 Hz, 2H), 3.21 (br t, J=4.39 Hz, 2H), 2.13 (s, 2H) 2.80 - 2.70 (m, 4H), 2.13 (s, 3H), 2.03 (br s, 2H), 1.77 (br t, J=11.17 Hz, 2H), 1.67 - 1.57 (m, 3H), 1.52 - 1.45 (m, 5H), 1.19 (t, J=7.40 Hz, 5H), 0.80 (t, J=7.40 Hz, 3H).

With reference to the synthesis method of steps **BB-1A** to **WX001** in Example 1, the compounds of Examples in the following table were synthesized.

| Exampl es | Fragment 1 | Fragment 2 | Fragment 3 | Fragment 4 | Structures in Examples and compound numbers |
|---|---|---|---|---|---|
| 2 | 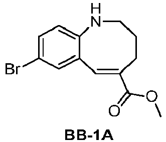 | 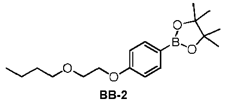 | 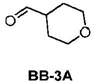 | 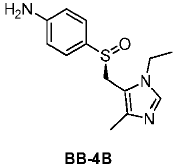 | 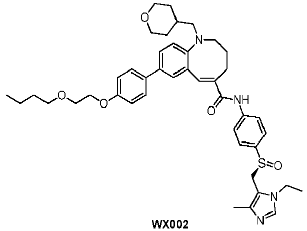 |
| 4 | 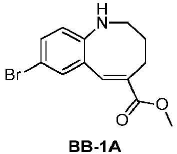 | 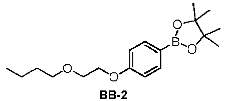 | 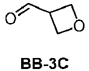 | 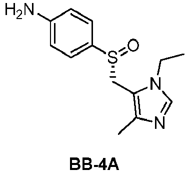 | 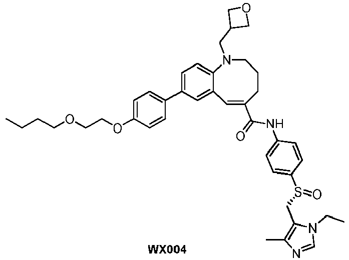 |
| 5 | 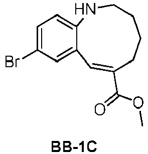 | 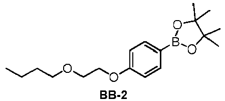 | 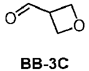 | 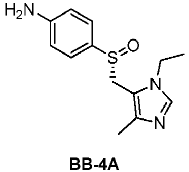 | 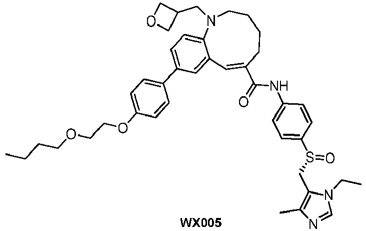 |
| 6 | 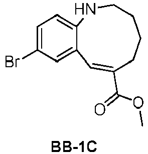 | 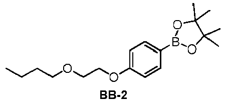 | 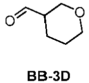 | 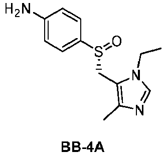 | 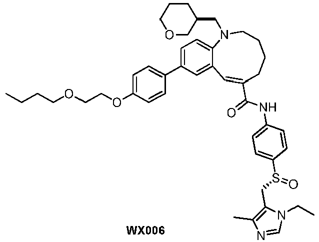 |
| 7 | 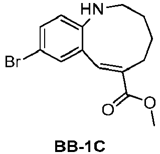 | 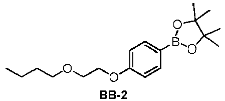 | 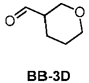 | 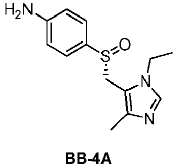 | 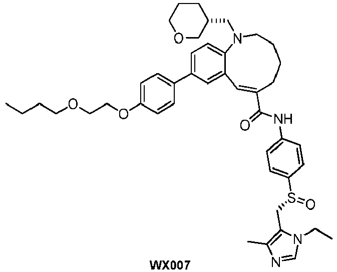 |
| 8 | 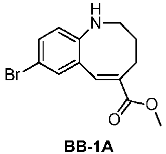 | 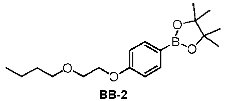 | 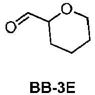 | 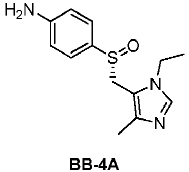 | 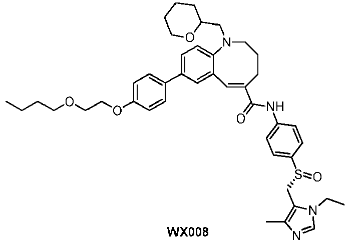 |
| 13 | 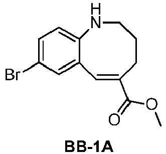 | 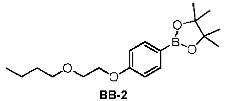 | 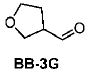 | 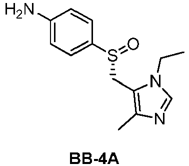 | 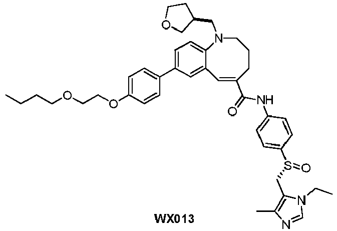 |
| 14 | 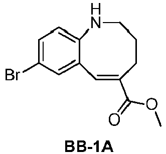 | 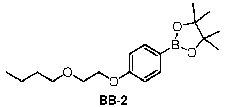 | 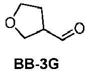 | 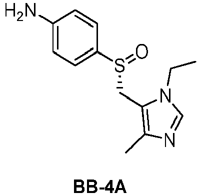 | 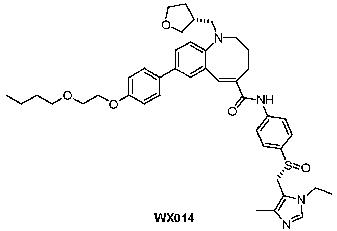 |
| 17 | 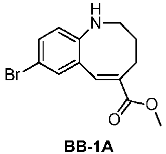 | 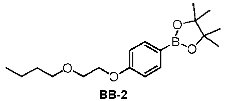 | 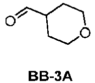 | 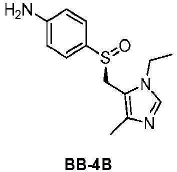 | 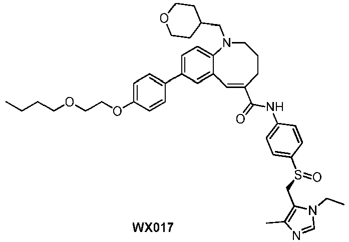 |
| 25 | 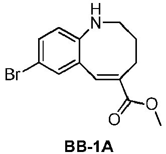 | 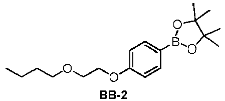 | 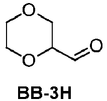 | 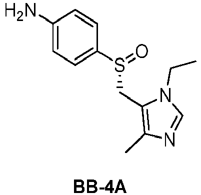 | 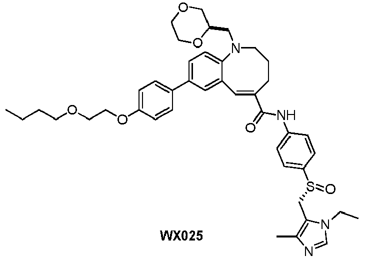 |
| 26 | 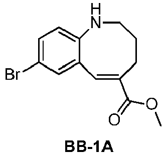 | 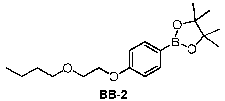 | 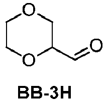 | 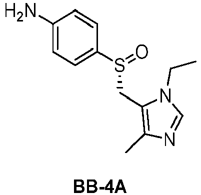 | 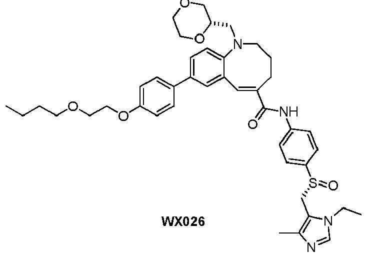 |
| 27 | 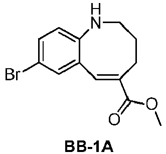 | 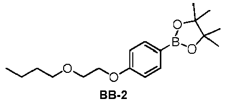 | 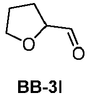 | 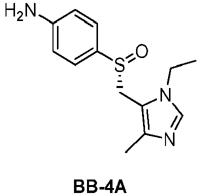 | 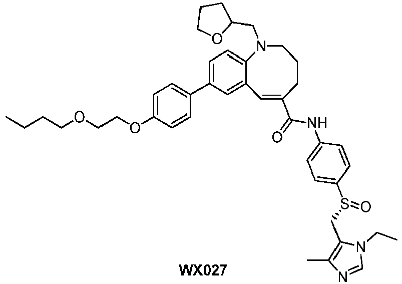 |
| 28 | 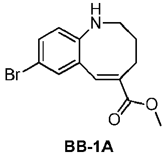 | 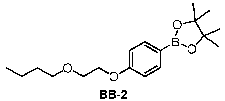 | 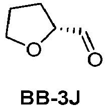 | 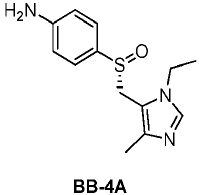 | 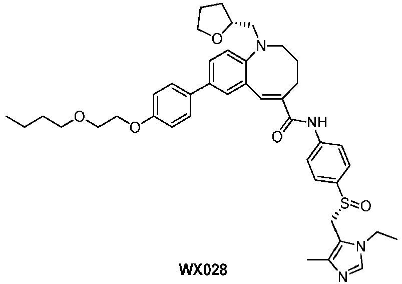 |

With reference to the synthesis method of steps **BB-1B** to **WX018** in Example 18, the compounds of Examples in the following table were synthesized.

| Examp les | Fragment 1 | Fragment 2 | Fragment 3 | Structures in Examples and compound numbers |
|---|---|---|---|---|
| 3 | 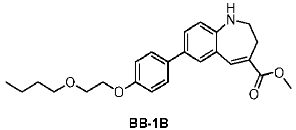 | 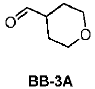 | 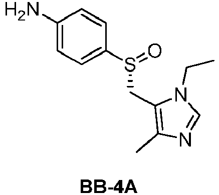 | 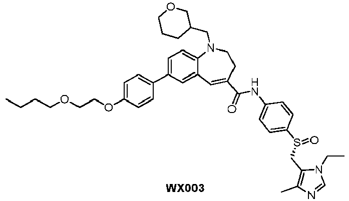 |
| 10 | 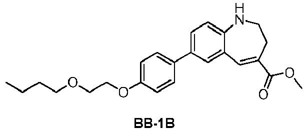 | 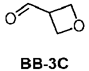 | 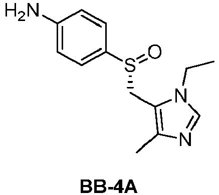 | 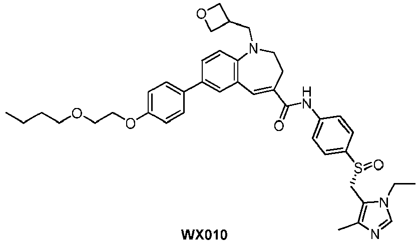 |
| 11 | 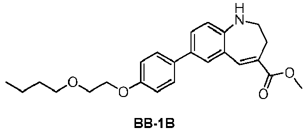 | 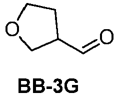 | 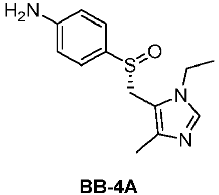 | 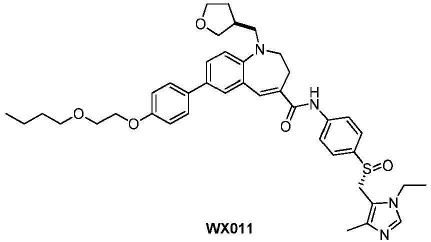 |
| 12 | 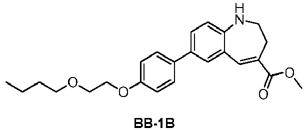 | 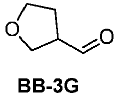 | 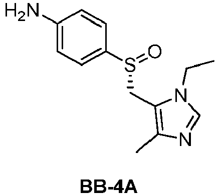 | 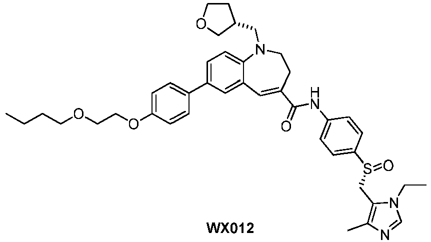 |
| 15 | 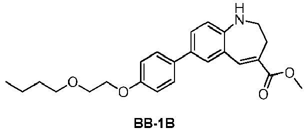 | 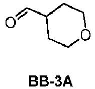 | 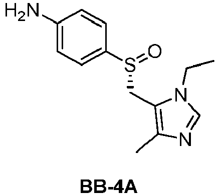 | 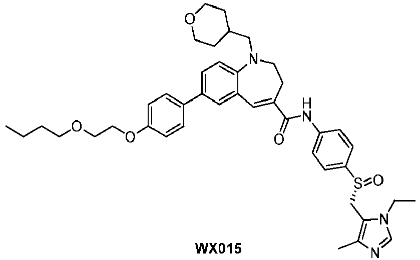 |

With reference to the synthesis method of steps **BB-1B** to **WX018** in Example 18, the compounds of Examples in the following table were synthesized.

| Exam ples | Fragment 1 | Fragment 2 | Fragment 3 | Structures in Examples and compound numbers |
|---|---|---|---|---|
| 9 | 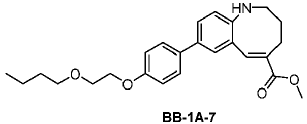 | 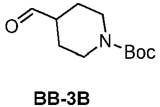 | 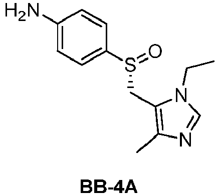 | 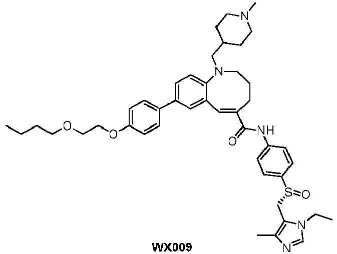 |
| 16 | 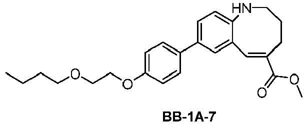 | 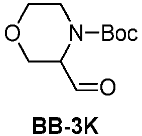 | 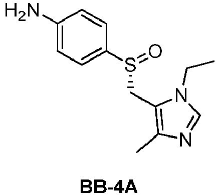 | 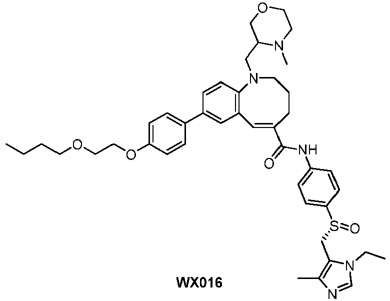 |
| 19 | 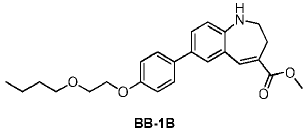 | 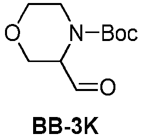 | 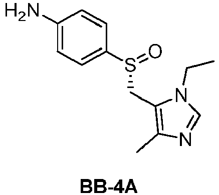 | 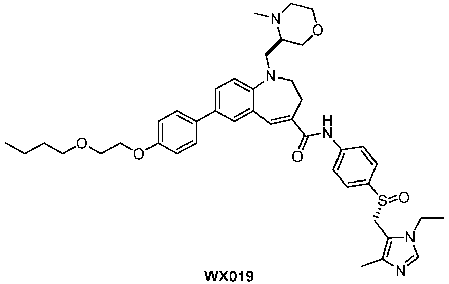 |
| 20 | 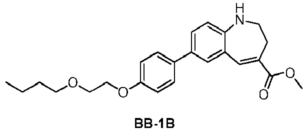 | 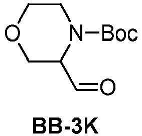 | 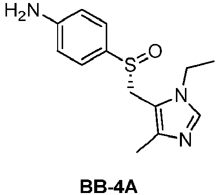 | 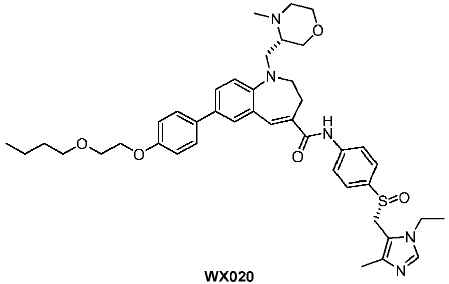 |
| 21 | 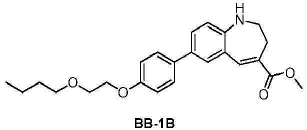 | 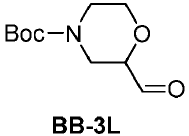 | 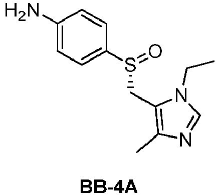 | 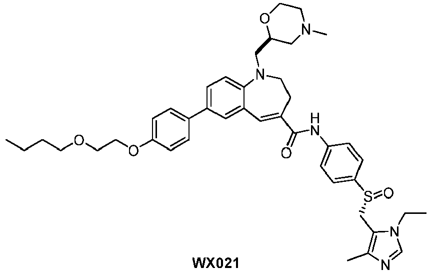 |
| 22 | 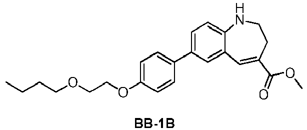 | 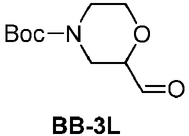 | 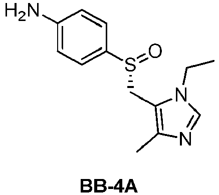 | 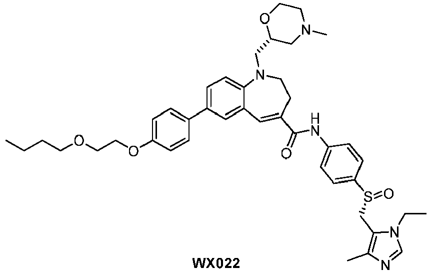 |
| 23 | 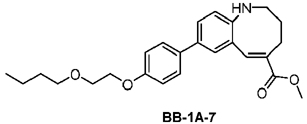 | 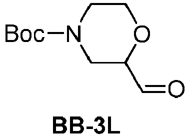 | 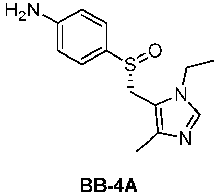 | 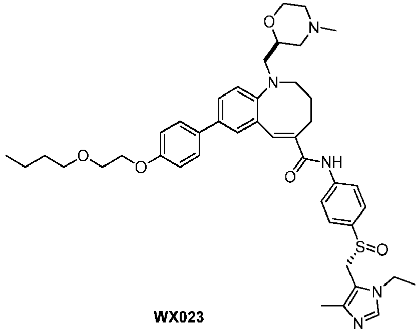 |
| 24 | 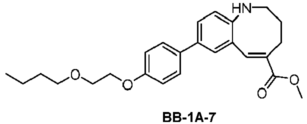 | 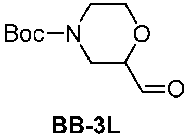 | 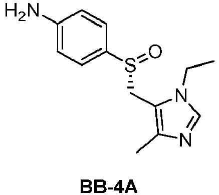 | 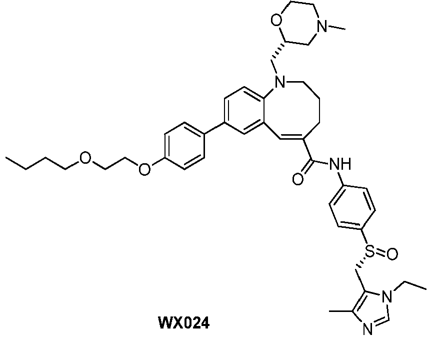 |

### ¹H NMR and MS data in each of Examples

| Exam ples | Compou nd Nos | NMR | MS *m*/*z*: |
|---|---|---|---|
| 2 | **WX002** | ¹H NMR (400MHz, CDCl₃) δ ppm 7.90 (s, 1H), 7.78 - 7.72 (m, 1H), 7.75 (d, J=8.8 Hz, 1H), 7.54 (s, 1H), 7.48 - 7.40 (m, 4H), 7.35 - 7.33 (m, 2H), 7.32 (s, 1H), 6.98 (d, J=8.8 Hz, 2H), 6.84 (d, J=8.8 Hz, 1H), 4.19 - 4.14 (m, 2H), 4.06 (s, 2H), 4.02 (br d, J=11.3 Hz, 2H), 3.92 - 3.84 (m, 2H), 3.84 - 3.79 (m, 2H), 3.58 - 3.50 (m, 4H), 3.39 (br t, J=11.3 Hz, 2H), 3.17 (d, J=6.5 Hz, 2H), 2.64 - 2.56 (m, 2H), 2.10 (br s, 1H), 1.69 (s, 5H), 1.63 (m, 4H), 1.47 - 1.37 (m, 7H), 0.94 (t, J=7.3 Hz, 3H). | 739. 9 |
| 3 | **WX003** | ¹H NMR (400MHz, CDCl₃) δ ppm 7.81 (s, 1H), 7.74 (d, J=8.3 Hz, 2H), 7.53 (s, 1H), 7.49 (s, 1H), 7.48 - 7.42 (m, 4H), 7.33 (d, J=9.0 Hz, 2H), 6.99 (d, J=8.5 Hz, 2H), 4.17 (t, J=5.1 Hz, 2H), 4.06 (s, 2H), 3.97 (q, J=8.3 Hz, 4H), 3.87 (br d, J=10.3 Hz, 2H), 3.83 - 3.80 (m, 2H), 3.56 (t, J=6.7 Hz, 2H), 3.47 (d, J=6.0 Hz, 2H), 3.43 (br d, J=4.3 Hz, 2H), 2.97 (br s, 1H), 1.82 (br d, J=8.3 Hz, 2H), 1.71 - 1.69 (m, 1H), 1.71 - 1.68 (m, 1H), 1.44 - 1.37 (m, 5H), 1.30 (br s, 2H), 1.26 - 1.26 (m, 3H), 0.96 - 0.92 (m, 3H), 0.89 (br s, 2H). | 725. 5 |
| 4 | **WX004** | ¹H NMR (400MHz, CDCl₃) δ ppm 8.91 (s, 1H), 7.75 (d, J = 8.4 Hz, 2H), 7.42-7.40 (m, 4H), 7.36 (s, 1H), 7.30-7.29 (m, 3H), 6.97-6.95 (m, 2H), 6.89-6.87 (m, 1H), 4.82 (t, J = 6.8 Hz, 2H), 4.55 (t, J = 6.0 Hz, 2H), 4.15 (t, J = 4.8 Hz, 2H), 4.07-3.98 (m, 2H), 3.86-3.78 (m, 4H), 3.57-3.54 (m, 4H), 3.43-3.39 (m, 3H), 2.61 (brs, 2H), 1.65-1.58 (m, 7H), 1.44-1.32 (m, 5H), 0.93 (t, J = 7.2 Hz, 3H). | 711. 2 |
| 5 | **WX005** | ¹H NMR (400MHz, CDCl₃) δ ppm 8.72 (s, 1H), 7.78 (d, J = 8.8 Hz, 2H), 7.54-7.59 (m, 3H), 7.44 (s, 1H), 7.35-7.32 (m, 5H), 7.00 (d, J = 8.8 Hz, 2H), 4.74 (t, J = 7.2 Hz, 2H), 4.45(t, J = 6.0 Hz, 2H), 4.17 (t, J = 4.8 Hz, 2H), 4.06 (s, 2H), 3.90-3.80 (m, 4H), 3.56 (t, J = 6.8 Hz, 2H), 3.21 (d, J = 6.0 Hz, 2H), 3.10-3.05 (m, 3H), 2.32-2.31 (m, 2H), 1.72-1.65 (m, 5H), 1.64-1.58 (m, 4H), 1.45-1.35 (m, 5H), 0.94 (t, J = 7.2 Hz, 3H). | 725. 3 |
| 6 | **WX006** | ¹H NMR (400MHz, CDCl₃) δ ppm 8.91 (d, J = 7.2 Hz, 1H), 7.78 (dd, J = 8.4, 2.0 Hz, 2H), 7.54-7.27 (m, 9H), 7.00 (d, J = 8.4 Hz, 2H), 4.17 (t, J = 4.8 Hz, 2H), 4.11-4.02 (m, 2H), 3.88-3.68 (m, 8H), 3.56 (t, J = 6.8 Hz, 2H), 3.00-2.61 (m, 5H), 2.01-1.58 (m, 10H), 1.48-1.26 (m, 10H), 0.94 (t, J = 7.2 Hz, 3H). | 753. 4 |
| 7 | **WX007** | ¹H NMR : (400MHz, CDCl₃) δ ppm 8.99 (s, 1H), 7.78 (dd, J = 8.8, 2.8 Hz, 2H), 7.52-7.27 (m, 9H), 7.00 (d, J = 8.8 Hz, 2H), 4.16 (t, J = 4.8 Hz, 2H), 4.04 (brs, 2H), 3.86-3.72 (m, 8H), 3.55 (t, J = 6.8 Hz, 2H), 2.96-2.58 (m, 5H), 2.04 (brs, 3H), 1.84-1.35 (m, 17H), 0.93 (t, J = 7.2 Hz, 3H). | 753. 4 |
| 8 | **WX008** | ¹H NMR (400 MHz, CDCl₃) δ ppm 7.83 ppm (s, 1H), 7.75 (d, J=8.78 Hz, 2H), 7.57 (s, 1H), 7.48 - 7.43 (m, 3H), 7.43 - 7.38 (m, 1H), 7.36 - 7.31 (m, 3H), 6.97 (d, J=8.78 Hz, 2H), 6.86 (d, J=8.78 Hz, 1H), 4.18 - 4.13 (m, 2H), 4.07 (s, 2H), 4.01 (br d, J=14.31 Hz, 1H), 3.91 - 3.83 (m, 2H), 3.83 - 3.79 (m, 2H), 3.73 (br s, 2H), 3.56 (t, J=6.65 Hz, 3H), 3.46 - 3.35 (m, 1H), 3.30 (br d, J=5.27 Hz, 2H), 2.63 - 2.52 (m, 2H), 1.71 (m, 5H), 1.63 (br s, 2H), 1.62 - 1.60 (m, 2H), 1.54 - 1.49 (m, 2H), 1.42 - 1.35 (m, 5H), 1.26 (s, 2H), 0.94 (t, J=7.40 Hz, 3H). | 370. 3 (M/ 2+1)⁺ |
| 9 | **WX009** | ¹H NMR (400 MHz, CDCl₃) δ ppm 8.83 ppm (br s, 1H), 7.86 (br d, J=8.28 Hz, 2H), 7.53 (br s, 1H), 7.45 (br s, 4H), 7.39 (br s, 2H), 7.31 (br d, J=8.03 Hz, 2H), 6.97 (br d, J=8.03 Hz, 2H), 4.15 (br s, 2H), 4.05 (br s, 2H), 3.92 (m, 2H), 3.91 - 3.84 (m, 2H), 3.84 - 3.79 (m, 4H), 3.45 - 3.27 (m, 4H), 2.62 (br s, 5H), 2.48 (br s, 2H), 2.02 (br s, 2H), 1.69 (br s, 3H), 1.61 (br s, 5H), 1.48 - 1.31 (m, 7H), 0.93 (br t, J=7.15 Hz, 3H). | 752. 3 |
| 10 | **WX010** | ¹H NMR (400MHz, CDCl₃) δ ppm 8.66 (s, 1H), 7.75 (d, J = 8.8 Hz, 2H), 7.45-7.38 (m, 6H), 7.27 (d, J = 8.4 Hz, 2H), 6.97 (d, J = 8.8 Hz, 2H), 6.87 (d, J = 8.4 Hz, 1H), 4.83-4.79 (m, 2H), 4.41 (t, J = 6.0 Hz, 2H), 4.16 (t, J = 4.8 Hz, 2H), 4.05 - 3.95 (m, 2H), 3.85 - 3.79 (m, 4H), 3.71 (d, J = 7.6 Hz, 2H), 3.55 (t, J = 6.4 Hz, 2H), 3.29 (t, J = 4.8 Hz, 2H), 2.85 (t, J = 4.0 Hz, 2H), 2.00-1.85 (brs, 1H), 1.65 - 1.58 (m, 5H), 1.44 - 1.31 (m, 5H), 0.93 (t, J = 7.2 Hz, 3H). | 697. 1 |
| 11 | **WX011** | ¹H NMR (400MHz, CDCl₃) δ ppm 8.04 (br s, 1H), 7.75 (br d, J=8.3 Hz, 2H), 7.50 (br d, J=11.3 Hz, 2H), 7.45 (br d, J=7.8 Hz, 4H), 7.32 (br d, J=8.0 Hz, 2H), 7.02 - 6.92 (m, 3H), 4.16 (br d, J=4.8 Hz, 2H), 4.05 (br s, 2H), 3.93 - 3.85 (m, 2H), 3.84 - 3.73 (m, 5H), 3.63 (br d, J=8.0 Hz, 2H), 3.56 (br t, J=6.8 Hz, 2H), 3.44 - 3.33 (m, 4H), 2.93 (br s, 2H), 2.72 (br s, 1H), 1.67 (br s, 3H), 1.64 - 1.60 (m, 2H), 1.42 - 1.36 (m, 4H), 1.26 (br s, 2H), 0.94 (br t, J=7.3 Hz, 3H). | 711. 1 |
| 12 | **WX012** | ¹H NMR (400MHz, CDCl₃) δ ppm 7.74 (br d, J=9.0 Hz, 3H), 7.54 (s, 1H), 7.49 (d, J=8.8 Hz, 2H), 7.44 (s, 2H), 7.34 (d, J=8.8 Hz, 2H), 7.02 - 6.94 (m, 4H), 4.19 - 4.15 (m, 2H), 4.07 (s, 2H), 3.94 (s, 2H), 3.87 (s, 2H), 3.84 - 3.75 (m, 5H), 3.65 (s, 2H), 3.56 (t, J=6.9 Hz, 2H), 3.39 (br d, J=9.3 Hz, 4H), 2.94 (br s, 2H), 1.71 (s, 3H), 1.62 (s, 2H), 1.40 (t, J=6.9 Hz, 3H), 1.26 (s, 2H), 0.94 (t, J=7.5 Hz, 3H). | 711. 5 |
| 13 | **WX013** | ¹H NMR (400MHz, CDCl₃) δ ppm 7.96 (s, 1H), 7.75 (d, J=8.8 Hz, 2H), 7.47 - 7.43 (m, 5H), 7.33 (d, J=8.5 Hz, 2H), 7.00 - 6.94 (m, 3H), 4.19 - 4.14 (m, 2H), 4.07 (s, 2H), 3.97 (br d, J=4.8 Hz, 2H), 3.87 (br d, J=12.0 Hz, 2H), 3.84 - 3.80 (m, 3H), 3.79 - 3.75 (m, 1H), 3.68 - 3.63 (m, 2H), 3.56 (t, J=6.8 Hz, 2H), 3.49 - 3.43 (m, 2H), 3.31 - 3.24 (m, 2H), 2.71 (br s, 2H), 2.63 (br s, 2H), 2.09 (br d, J=4.5 Hz, 1H), 1.71 (s, 2H), 1.63 (br d, J=7.8 Hz, 4H), 1.41 - 1.37 (m, 3H), 1.26 (s, 2H), 0.94 (t, J=7.4 Hz, 3H). | 725. 4 |
| 14 | **WX014** | ¹H NMR (400MHz, CDCl₃) δ ppm 7.88 (s, 1H), 7.75 (d, J=8.8 Hz, 2H), 7.49 - 7.43 (m, 5H), 7.38 - 7.31 (m, 3H), 7.01 - 6.94 (m, 3H), 4.18 - 4.14 (m, 2H), 4.07 (s, 2H), 3.97 (br d, J=5.3 Hz, 2H), 3.87 (br dd, J=4.6, 7.4 Hz, 2H), 3.84 - 3.80 (m, 2H), 3.80 - 3.74 (m, 2H), 3.68 - 3.62 (m, 1H), 3.56 (t, J=6.7 Hz, 2H), 3.45 (br d, J=5.5 Hz, 2H), 3.32 - 3.25 (m, 2H), 2.70 (s, 2H), 2.63 (br s, 2H), 2.11 (br d, J=7.5 Hz, 2H), 1.71 (s, 3H), 1.67 - 1.61 (m, 4H), 1.39 (s, 3H), 0.94 (t, J=7.4 Hz, 3H). | 725. 1 |
| 15 | **WX015** | ¹H NMR (400MHz, CDCl₃) δ ppm 8.05 (s, 1H), 7.74 (d, J=8.8 Hz, 2H), 7.51 - 7.41 (m, 6H), 7.31 (d, J=8.5 Hz, 2H), 6.99 (d, J=8.8 Hz, 2H), 6.92 (d, J=8.5 Hz, 1H), 4.17 (t, J=4.9 Hz, 2H), 4.04 (s, 2H), 4.00 (br dd, J=3.4, 11.2 Hz, 2H), 3.90 - 3.79 (m, 4H), 3.56 (t, J=6.8 Hz, 2H), 3.41 - 3.33 (m, 4H), 3.30 (br d, J=7.0 Hz, 2H), 2.92 (br s, 2H), 2.00 (br s, 1H), 1.72 (br s, 2H), 1.66 - 1.57 (m, 5H), 1.46 - 1.28 (m, 7H), 0.94 (t, J=7.4 Hz, 3H). | 725. 1 |
| 16 | **WX016** | ¹H NMR (400 MHz, CDCl₃) δ ppm 8.67 (s, 1H), 7.58 (d, J = 8.4 Hz, 2H), 7.27-7.21 (m, 5H), 7.10-7.08 (m, 3H), 6.83 (d, J = 8.4 Hz, 1H), 6.77 (d, J = 8.8 Hz, 2H), 3.97 (t, J = 4.2 Hz, 2H), 3.81 (dd, J = 23.2, 14.0 Hz, 2H), 3.69-3.58 (m, 6H), 3.52-3.43 (m, 2H), 3.37 (t, J = 6.8 Hz, 2H), 3.29-3.09 (m, 3H), 2.97-2.91 (m, 1H), 2.56 (d, J = 12.0 Hz, 1H), 2.43-2.39 (m, 3H), 2.27 (s, 3H), 2.23-2.17 (m, 1H), 1.45-1.39 (m, 7H), 1.26-1.19 (m, 2H), 1.14 (t, J = 7.2 Hz, 3H), 0.75 (t, J = 7.2 Hz, 3H). | 754. 3 |
| 17 | **WX017** | ¹H NMR (400 MHz, CDCl₃) δ ppm 7.83 (s, 1H), 7.75 (d, J=8.53 Hz, 2H), 7.54 (s, 1H), 7.50 - 7.41 (m, 4H), 7.33 (d, J=8.78 Hz, 3H), 6.98 (d, J=8.78 Hz, 2H), 6.84 (d, J=8.78 Hz, 1H), 4.16 (d, J=5.52 Hz, 2H), 4.07 (br d, J=5.02 Hz, 4H), 3.89 (br d, J=4.52 Hz, 2H), 3.85 - 3.78 (m, 2H), 3.58 - 3.51 (m, 4H), 3.39 (s, 2H), 3.17 (br d, J=6.78 Hz, 2H), 2.61 (br s, 2H), 2.11 (br s, 1H), 1.69 (s, 5H), 1.64 (br s, 4H), 1.44 - 1.37 (m, 7H), 0.94 (q, J=7.28 Hz, 3H). | 739. 3 |
| 19 | **WX019** | ¹H NMR (400MHz, CDCl₃) δ ppm 7.67 (br d, J=8.5 Hz, 3H), 7.48 - 7.45 (m, 1H), 7.42 (s, 1H), 7.40 - 7.35 (m, 4H), 7.27 (d, J=8.5 Hz, 2H), 6.94 - 6.89 (m, 3H), 4.11 - 4.07 (m, 2H), 3.99 (s, 2H), 3.83 (br s, 1H), 3.78 (d, J=7.5 Hz, 1H), 3.76 - 3.72 (m, 3H), 3.48 (t, J=6.7 Hz, 2H), 3.32 (br s, 2H), 2.90 (br s, 2H), 2.71 (s, 2H), 2.42 (br s, 3H), 1.66 - 1.64 (m, 1H), 1.65 (s, 2H), 1.58 - 1.53 (m, 4H), 1.31 (t, J=7.3 Hz, 5H), 1.20 - 1.17 (m, 4H), 0.88 - 0.85 (m, 3H). | 740. 4 |
| 20 | **WX020** | ¹H NMR (400MHz, CDCl₃) δ ppm 7.76 (d, J=8.5 Hz, 1H), 7.88 - 7.69 (m, 1H), 7.83 - 7.69 (m, 1H), 7.54 (d, J=9.3 Hz, 1H), 7.51 - 7.43 (m, 5H), 7.34 (d, J=8.5 Hz, 2H), 7.02 - 6.96 (m, 3H), 4.17 (t, J=4.9 Hz, 2H), 4.07 (s, 2H), 3.88 (s, 2H), 3.87 - 3.79 (m, 5H), 3.56 (t, J=6.8 Hz, 2H), 3.40 (br s, 2H), 2.99 (br s, 2H), 2.53 (br s, 1H), 2.59 - 2.45 (m, 2H), 1.73 (s, 3H), 1.69 - 1.66 (m, 4H), 1.40 (t, J=7.4 Hz, 5H), 1.26 (s, 4H), 0.94 (s, 3H). | 740. 4 |
| 21 | **WX021** | ¹H NMR (400MHz, CDCl₃) δ ppm 7.85 (s, 1H), 7.74 (d, J=8.8 Hz, 2H), 7.51 (d, J=2.0 Hz, 1H), 7.47 (d, J=8.8 Hz, 2H), 7.45 - 7.41 (m, 3H), 7.33 (d, J=8.5 Hz, 2H), 7.04 - 6.95 (m, 3H), 4.19 - 4.13 (m, 2H), 4.05 (s, 2H), 3.97 - 3.89 (m, 2H), 3.89 - 3.84 (m, 2H), 3.84 - 3.80 (m, 2H), 3.72 - 3.65 (m, 1H), 3.56 (t, J=6.8 Hz, 2H), 3.53 - 3.45 (m, 2H), 3.44 - 3.36 (m, 2H), 2.93 (br s, 2H), 2.86 - 2.65 (m, 2H), 2.32 (s, 3H), 2.21 - 2.11 (m, 1H), 1.91 (t, J=10.8 Hz, 1H), 1.70 (s, 3H), 1.65 (br s, 2H), 1.45 - 1.36 (m, 5H), 0.94 (t, J=7.4 Hz, 3H). | 740. 5 |
| 22 | **WX022** | ¹H NMR (400MHz, CDCl₃) δ ppm 7.81 (s, 1H), 7.74 (d, J=8.5 Hz, 2H), 7.52 (d, J=2.0 Hz, 1H), 7.48 (d, J=8.8 Hz, 2H), 7.45 - 7.40 (m, 3H), 7.33 (d, J=8.5 Hz, 2H), 7.03 - 6.97 (m, 3H), 4.17 (t, J=4.9 Hz, 2H), 4.06 (s, 2H), 3.97 - 3.89 (m, 2H), 3.89 - 3.84 (m, 2H), 3.83 - 3.79 (m, 2H), 3.73 - 3.64 (m, 1H), 3.56 (t, J=6.7 Hz, 2H), 3.53 - 3.44 (m, 2H), 3.44 - 3.36 (m, 2H), 2.93 (br s, 2H), 2.82 (br d, J=11.5 Hz, 1H), 2.69 (br d, J=11.8 Hz, 1H), 2.32 (s, 3H), 2.21 - 2.12 (m, 1H), 1.91 (t, J=10.7 Hz, 1H), 1.71 (s, 3H), 1.66 - 1.62 (m, 2H), 1.44 - 1.36 (m, 5H), 0.94 (t, J=7.4 Hz, 3H). | 740. 5 |
| 23 | **WX023** | ¹H NMR (400MHz, CDCl₃) δ ppm 8.02 (s, 1H), 7.75 (d, J=8.8 Hz, 2H), 7.54 (s, 1H), 7.47 - 7.37 (m, 4H), 7.33 (d, J=8.8 Hz, 3H), 6.97 (d, J=8.8 Hz, 2H), 6.90 (d, J=8.8 Hz, 1H), 4.18 - 4.14 (m, 2H), 4.06 (s, 2H), 3.90 (br d, J=13.3 Hz, 2H), 3.85 (dd, J=5.0, 7.3 Hz, 2H), 3.83 - 3.79 (m, 2H), 3.71 - 3.61 (m, 2H), 3.56 (t, J=6.7 Hz, 2H), 3.51 (br d, J=9.3 Hz, 1H), 3.35 - 3.29 (m, 1H), 3.38 - 3.27 (m, 1H), 2.84 (br d, J=10.5 Hz, 1H), 2.67 (br d, J=11.3 Hz, 1H), 2.62 - 2.51 (m, 2H), 2.33 (s, 3H), 2.16 (dt, J=3.5, 11.4 Hz, 1H), 1.92 (t, J=10.5 Hz, 1H), 1.70 (s, 3H), 1.65 - 1.63 (m, 2H), 1.58 (br s, 2H), 1.44 - 1.36 (m, 5H), 0.94 (t, J=7.4 Hz, 3H). | 754. 5 |
| 24 | **WX024** | ¹H NMR (400MHz, CDCl₃) δ ppm 7.97 (br s, 1H), 7.76 (d, J=8.5 Hz, 2H), 7.54 (s, 1H), 7.47 - 7.38 (m, 4H), 7.33 (d, J=8.5 Hz, 3H), 6.97 (d, J=8.8 Hz, 2H), 6.90 (d, J=8.8 Hz, 1H), 4.16 (t, J=4.9 Hz, 2H), 4.06 (s, 2H), 3.91 (br d, J=13.3 Hz, 2H), 3.86 (dd, J=5.0, 7.3 Hz, 2H), 3.83 - 3.79 (m, 2H), 3.72 - 3.61 (m, 2H), 3.56 (t, J=6.7 Hz, 2H), 3.50 (br s, 1H), 3.37 - 3.27 (m, 2H), 2.85 (br d, J=9.5 Hz, 1H), 2.68 (br d, J=11.3 Hz, 1H), 2.63 - 2.52 (m, 2H), 2.33 (s, 3H), 2.20 - 2.13 (m, 1H), 1.92 (t, J=10.9 Hz, 1H), 1.70 (s, 3H), 1.64 (br s, 2H), 1.59 - 1.57 (m, 2H), 1.43 - 1.36 (m, 5H), 0.94 (t, J=7.3 Hz, 3H). | 754. 5 |
| 25 | **WX025** | ¹H NMR (400 MHz, CDCl₃) δ ppm 8.46-8.35 ppm (br s, 1H), 7.68 (d, J=8.53 Hz, 2H), 7.46 (s, 1H), 7.38-7.29 (m, 4H), 7.22 (d, J=8.53 Hz, 3H), 6.79 (d, J=8.78 Hz, 2H), 6.89 (d, J=8.78 Hz, 1H), 4.08 (t, J=4.89 Hz, 2H), 4.03 - 3.93 (m, 2H), 3.82 - 3.76 (m, 2H), 3.76 - 3.72 (m, 4H), 3.69 - 3.61 (m, 2H), 3.68 - 3.59 (m, 5H), 3.44 - 3.29 (m, 2H), 3.28 - 3.12 (m, 2H), 2.49 (br t, J=5.77 Hz, 2H), 1.60 - 1.48 (m, 7H), 1.37 - 1.31(m, 2H), 1.30-1.25 (t, J=7.40 Hz, 3H), 0.86 (t, J=7.28 Hz, 3H). | 741.4 |
| 26 | **WX026** | ¹H NMR (400 MHz, CDCl₃) δ ppm 8.70 ppm (br s, 1H), 7.77 (d, J=8.53 Hz, 2H), 7.54 (s, 1H), 7.45 - 7.35 (m, 4H), 7.29 (d, J=8.53 Hz, 2H), 7.26 (s, 1H), 6.96 (d, J=8.78 Hz, 3H), 6.86 (d, J=8.78 Hz, 1H), 4.15 (t, J=4.89 Hz, 2H), 4.10 - 3.99 (m, 2H), 3.89 - 3.83 (m, 2H), 3.83 - 3.79 (m, 4H), 3.76 - 3.68 (m, 2H), 3.68 - 3.59 (m, 5H), 3.53 - 3.37 (m, 1H), 3.34 - 3.20 (m, 2H), 2.56 (br t, J=5.77 Hz, 2H), 1.66 - 1.56 (m, 7H), 1.46 - 1.36 (m, 2H), 1.34 (t, J=7.40 Hz, 3H), 0.94 (t, J=7.28 Hz, 3H). | 741. 3 |
| 27 | **WX027** | ¹H NMR (400MHz, CDCl₃) δ ppm 8.13 (br s, 1H), 7.76 (d, J=8.53 Hz, 2H), 7.54 (s, 1H), 7.48 - 7.37 (m, 4H), 7.32 (d, J=8.78 Hz, 3H), 7.01 - 6.89 (m, 3H), 4.27-12 (br s, 3H), 4.06 (m, 2H), 3.98 - 3.89 (m, 1H), 3.89 - 3.74 (m, 5H), 3.70(m, 1H), 3.56 (t, J=6.78 Hz, 3H), 3.47 - 3.28 (m, 2H), 2.59 (br s, 2H), 2.28-2.05 (m, 1H), 2.03 - 1.81 (m, 2H), 1.69 (br s, 3H), 1.64 - 1.56 (m, 5H), 1.44 - 1.33 (m, 5H), 0.94 (t, J=7.28 Hz, 3H). | 725. 4 |
| 28 | **WX028** | ¹H NMR (400MHz, CDCl₃) δ ppm 8.29 (br s, 1H), 7.76 (d, J=8.53 Hz, 2H), 7.54 (s, 1H), 7.48 - 7.36 (m, 4H), 7.31 (br d, J=8.78 Hz, 3H), 7.01 - 6.89 (m, 3H), 4.22 (br s, 1H), 4.19 - 4.11 (m, 2H), 4.05 (s, 2H), 3.98 - 3.89 (m, 1H), 3.88 - 3.74 (m, 5H), 3.74 - 3.63 (m, 1H), 3.62 - 3.49 (m, 3H), 3.49 - 3.31 (m, 2H), 2.59 (br s, 2H), 2.11 (br d, J=6.78 Hz, 1H), 2.03 - 1.81 (m, 2H), 1.68 (br s, 3H), 1.64 - 1.56 (m, 5H), 1.44 - 1.33 (m, 5H), 0.94 (t, J=7.28 Hz, 3H). | 725. 4 |

### Experimental Example 1: CCR2/ CCR5 in vitro test

### Purpose:

The changes in intracellular calcium signal were detected by FLIPR Calcium assay, and the compound's IC50 value was used as an indicator to evaluate inhibitory effect of the compound on CCR2 and CCR5 receptors.

### Experimental Materials:

1, Cell line: Cells were seeded and incubated overnight in an incubator at 37°C and 5% CO₂.
CCR2/ CCR5 density: 1 M (20K / Well)

| **Cell line** | **Number of clones** | **Cellular generation** | **Host cell** |
|---|---|---|---|
| CCR2 | C7 | P6 | HEK293 |
| CCR5 | C13 | P4 | HEK293 |

2, Reagents: Fluo-4 Direct, (Invitrogen, Cat # f10471)
3, Device and Equipment:
384 cell plates coated with polylysine (Greiner # 781946)
384-well compound plates (Greiner # 781280)
FLIPR, a molecular device
Echo, LabCyte

4, Compound:
The compound was dissolved in DMSO to prepare a 10 mM solution, and the compound solution was placed in a nitrogen gas box.

| **Compound ID** | **purity** | **Compound weight (mg)** |
|---|---|---|
| CENICRIROC (CVC) | 97.00 | 1.15 |

### Reference antagonist compound:

| | | | |
|---|---|---|---|
| MCP-1 | Sigma | SRP3109 | 10µm stock in H₂O |
| Rantes | Sigma | SRP3269 | 10µm stock in H₂O |

### Experimental steps and methods:

Preparation of probenecid in FLIPR assay buffer: 1 mL of FLIPR assay buffer was added to 77 mg of probenecid to prepare a 250 mM solution. The 250 mM solution was freshly prepared every day.
2× (8µM) Fluo-4 Direct^{™} loading buffer (per 10 mL)
a bottle of Fluo-4 DirectTM crystal (F10471) was thawed.
10 mL of FLIPR assay buffer was added to a sample bottle.
0.2 mL of probenecid was added per 10 mL of Fluo-Direct. The final measured concentration is 2.5 mM,
Rotating and standing for> 5 minutes (protecting from light)
Freshly prepared every day
Experimental steps:
   a) Preparation of agonist compound:
      MCP-1 was serially diluted with FLIPR assay buffer starting from 0.5 µM at a dilution ratio of 1:2 and a total of 10 gradients were diluted (final 100nM). RANTES is serially diluted with FLIPR assay buffer starting from 0.5 µM at a dilution ratio of 1:3 and a total of 10 gradients were diluted (final 100nM). According to the compound plate -map, 20 µL of the serially diluted compound buffers were added to each well of the DRC plate respectively.
   b) Preparation of antagonist compound: reference antagonist compound
      The standard compound was diluted with DMSO 1:3 starting from 1 mM at a dilution ratio of 1:3 and a total of 11 gradients were diluted. The test compound was diluted with DMSO 1:3 starting from 2 mM at a dilution ratio of 1:3 and a total of 11 gradients were diluted. 250 nL of the compound solution was transferred to the cell plate (Greiner#781946) using Echo.
   c) taking the cell plate from the incubator, and gently distributing 20µL of 2× Fluo-4 Direct non-washing loading buffer to each well of the 384-well cell culture plate using a pipette, wherein each of wells in the cell plate has a final volume of 40 µL.
   d) Incubating at 37°C and 5% CO₂ for 50 minutes, and at room temperature for 10 minutes.
   e) removing the cell plate from the incubator and placing in FLIPR, and putting the composite plate and the tip box into FLIPR.
   f) For the DRC board:
      1) Running the program on FLIPRTETRA
      2) Reading the fluorescence signal
      3) Transferring 10 µL of the compound from the DRC plate to the cell plate
      4) Reading the fluorescence signal
      5) Calculating from Read 90 to the maximum allowable "maximum-minimum", and calculating the EC80 value of each of cell lines by using FLIPR
      6) Preparing the reference agonist compound at a concentration of 5× EC80
   g) For the composite plate (1-add):
      1) Running the program on FLIPRTETRA
      2) Transferring 10 µl of the reference agonist compound at a concentration of 5× EC80 from the composite plate to the cell plate.
      3) Reading the fluorescence signal.
      4)Calculating from Read 90 to the maximum allowable "maximum-minimum".
   h) Analyzing the data and calculating the IC50 value of the compound by using Prism.

The experimental results are shown in Table 1.

**Table 1. IC₅₀ (nM) test results for FLIPR detection**

| Compound numbers | CCR2 | CCR5 | Compound numbers | CCR2 | CCR5 |
|---|---|---|---|---|---|
| **WX001** | 1.33 | 4.25 | **WX015** | 7.65 | 5.62 |
| **WX002** | 3 | 8.15 | **WX016** | 3.9 | 4.12 |
| **WX003** | 37 | 8.27 | **WX017** | 2.47 | 1.7 |
| **WX004** | 3.04 | 6.3 | **WX018** | 17.9 | 3.35 |
| **WX005** | 9.2 | 4.2 | **WX019** | 4.97 | 0.65 |
| **WX006** | 7.51 | 8.44 | **WX020** | 18.1 | 6.99 |
| **WX007** | 6.76 | 6.18 | **WX021** | 48.9 | 15.7 |
| **WX008** | 3.4 | 6.04 | **WX022** | 21 | 10.3 |
| **WX009** | 4.38 | 6.78 | **WX023** | 2.62 | 2.45 |
| **WX010** | 7.48 | 7.33 | **WX024** | 1.59 | 0.76 |
| **WX011** | 6.82 | 4.33 | **WX025** | 0.69 | 1.38 |
| **WX012** | 6.19 | 3.29 | **WX026** | 0.69 | 1.37 |
| **WX013** | 3.11 | 5.45 | **WX027** | 0.41 | 1.44 |
| **WX014** | 3.78 | 6.28 | **WX028** | 1.19 | 3.15 |

**Conclusion:** the compounds of the invention have significant antagonistic effect on CCR2 and CCR5 receptors.

### Experimental Example 2. inhibition of human liver microsomal cytochrome P450 isoenzymes (CYP1A2, CYP2C9, CYP2C19, CYP2D6 and CYP3A4) activity

A total of 5 specific probe substrates of 5 CYP isozymes, which are phenacetin (PHenacetin, CYP1A2), diclofenac (Diclofenac, CYP2C9), (S)-mephenytoin ((S)-MepHenytoin, CYP2C19), dextromethorpHan (CYP2D6), and midazolam (Midazolam, CYP3A4), were incubated with human liver microsomes respectively. Then, reduced nicotinamide adenine dinucleotide phosphate (NADPH) was added to initiate reaction. After the reaction, the sample is processed and quantitatively detected for the concentrations of the five metabolites produced from the specific substrate, acetaminophen (AcetaminopHen), 4'-hydroxydiclofenac (4'-Hydroxydiclofenac), 4'-Hydroxymephenytoin (4'-HydroxymepHenytoin), Dextrorphan (DextrorpHan), and 1'-Hydroxymidazolam (1'-Hydroxymidazolam) by liquid chromatography-quadrupole mass spectrometry (LC-MS/MS) method to calculate the corresponding half inhibitory concentration (IC₅₀).

**Table 2.Half inhibitory concentrations of compounds**

| Compound numbers | IC50 (µM) | | | | |
|---|---|---|---|---|---|
| | CYP1A2 | CYP2C9 | CYP2C19 | CYP2D6 | CYP3A4-M |
| Reference compound (CVC) | >50 | >50 | >50 | >50 | 0.75 |
| **Reference A** | >50 | >50 | >50 | >50 | 0.786 |
| **WX001** | >50 | >50 | 1.42 | >50 | >50 |
| **WX002** | >50 | 10.9 | >50 | >50 | 13.3 |
| **WX011** | >50 | 22.8 | >50 | >50 | >50 |

**Conclusion:** WX002 and WX011 have no risk of inhibiting the activity of human liver microsomal cytochrome P450 isoenzymes (CYP1A2, CYP2B6, CYP2C8, CYP2C9, CYP2C19, CYP2D6 and CYP3A4). The drugs that CYP2C19 involves in their metabolism account for only 2% of marketed drugs, while the drugs that CYP3A involves in their metabolism have accounted for 50 % of marketed drugs. The reference compound and the reference A have a strong inhibitory effect on CYP3A4, which is reflected in the clinical practice of inhibiting CYP3A4 to increase exposure to other drugs metabolized by CYP3A4, thereby having a potential safety risk, that is, drug-drug interaction. WX001 has no inhibitory effect on CYP3A4, the main metabolic enzyme of drug metabolism, thereby avoiding occurrence of such risks.

### Experimental Example 3 Anti-tumor activity of compounds of the invention in animal tumor models in vivo

### Purpose:

The anti-tumor effect of the compound of the invention in combination with a anti-PD-1 antibody in mice (RMP1-14, Bioxcell, catalog number BP0146) was investigated in a colon cancer MC38 tumor model in mice *in vivo.*

### Experimental method:

Female C57BL/6 mice were subcutaneously inoculated with MC38 colon cancer cell lines in mice, and randomly grouped according to the body weight after inoculation.

The administration was carried out according to the following description.

Group 1 (A control group): the vehicle 1 (5% DMSO/95% (10% HP-β-CD: β-Cyclodextrin)) was administrated intragastrically at a dose of 0.1 mL/10g body weight twice daily, starting in the afternoon on the day of inoculation. Vehicle 2 (DPBS: Dulbecco's phosphate buffered saline) was administrated by intraperitoneal injection at a dose of 0.1 mL/10 g body weight at 11, 14 and 18 day after inoculation. Group 2: Anti-mouse PD-1 antibody (RMP1-14) was injected intraperitoneally at 11, 14 and 18 day after inoculation once. The dose at 11 day is 3 mg/kg body weight, and the dose at 14 day and the dose18 day is 6 mg/kg body weight.

Group 3: CVC (Cenicriviroc) (dissolved in 5% DMSO + 95% (10% HP-β-CD), pH 4) was administrated intragastrically at a dose of 100 mg/kg body weight twice daily, starting in the afternoon on the day of inoculation. Anti-mouse PD-1 antibody (RMP1-14) was administrated by intraperitoneal injection at 11, 14 and 18 day after inoculation. The dose at 11 day was 3 mg/kg body weight, and the doses on 14 day and 18 day were 6 mg/kg body weight.

Group 4: WX001 (dissolved in 5% DMSO+95% (10% HP-β-CD), pH 4) was administrated intragastrically at a dose of 100 mg/kg body twice daily, starting in the afternoon on the day of inoculation. Anti-mouse PD-1 antibody (RMP1-14) was administrated by intraperitoneal injection at 11, 14 and 18 day after inoculation once. The dose at day 11 was 3 mg/kg body weight, and the doses on 14 day and 18 day was 6 mg/kg body weight.

During the experiment, the mice were weighed three times a week. After tumour formation, the tumor volume was measured three times a week in synchronization with weighing the body weight. The tumor volume was calculated according to the formula of "length × width ²/2". the tumor proliferation rate and tumor inhibition rate were calculated according to the following formulas: Tumor proliferation rate = the tumor volume in the treatment group/the tumor volume in the control group × 100%; Tumor inhibition rate = (the tumor volume in the control group- the tumor volume in the treatment group)/the tumor volume in the control group.

Student's t-test was used for statistical analysis between the groups, and p<0.05 was considered as a significant difference.

### Experimental results:

**Table 3 Tumor proliferation ratio in each of groups**

| **Group** | **Tumor proliferation ratio** |
|---|---|
| Anti-PD-1 antibody group | 49.40% |
| CVC + PD-1 combination group | 52.58% |
| WX001 + PD-1 combination group | 25.42% |

The effect of WX001 on the tumor volume in the MC38 colon cancer tumor model in mice is shown in Figure 1.

**Experimental Conclusion:** In the MC38 tumor model in mice, the WX001+PD-1 combination group had a significant enhanced efficacy compared to the PD-1 single-use group. The tumor proliferation rate at 31 day was 25.42% (compared to the vehicle group, p<0.01; compared to the CVC+PD-1 combination group, p<0.01).

## Claims

1. A compound of the formula (I), or an isomer or pharmaceutically acceptable salt thereof, 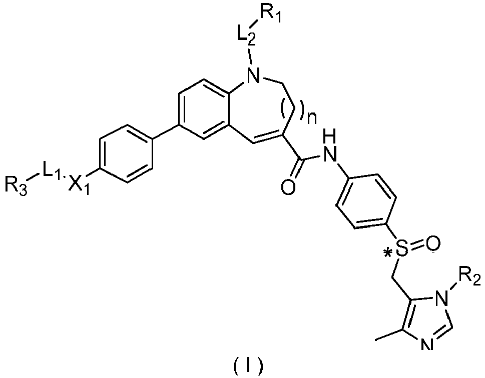 wherein,
n is selected from the group consisting of 1, 2, and 3;
R₁ is 4 to 6 membered heterocycloalkyl, which is optionally substituted by 1, 2 or 3 Rₐ;
R₂ is C₁₋₆ alkyl, which is optionally substituted by 1, 2 or 3 R_{b};
R₃ is C₁₋₆ alkoxy, which is optionally substituted by 1, 2 or 3 R_{c};
X₁ is selected from the group consisting of O, S and -NH-;
L₁ is - (CRR)ₘ-;
L₂ is -CRR-;
m is selected from the group consisting of 1, 2, 3, and 4;
Rₐ, R_{b} and R_{c} are independently selected from the group consisting of H, F, Cl, Br, I, OH, NH₂, CN, and CH₃;
each R is independently selected from the group consisting of H, F, Cl, Br, I, OH,
NH₂, CN, and CH;
the 4 to 6-membered heterocycloalkyl comprises 1, 2, 3 or 4 heteroatoms or heteroatom groups independently selected from the group consisting of -NH -, - O - S- and N; wherein the isomer thereof is a geometric or stereoisomeric isomer; and
the sulfur atom with"*" is a chiral sulfur atom, which exists as a (R) or (S) single enantiomer or is riched in one of the enantiomers.

2. The compound, or the isomer or pharmaceutically acceptable salt thereof according to claim 1, wherein R₂ is C₁₋₃ alkyl, which is optionally substituted by 1,2 or 3 R_{b}, and preferably, wherein R₂ is CH₂CH₃.

3. The compound, or the isomer or pharmaceutically acceptable salt thereof according to claim 1 or 2, wherein R₃ is C₄₋₆ alkoxy, which is optionally substituted by 1, 2 or 3 R_{c}.

4. The compound, or the isomer or pharmaceutically acceptable salt thereof according to claim 3, wherein R₃ is

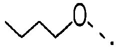

5. The compound, or the isomer or pharmaceutically acceptable salt thereof according to claim 1 or 2 or 4, wherein L₁ is -CH₂CH₂-.

6. The compound, or the isomer or pharmaceutically acceptable salt thereof according to any one of claims 1 to 5, wherein the structural unit 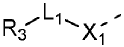 is

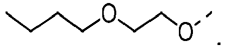

7. The compound, the isomer or pharmaceutically acceptable salt thereof according to claim 1, wherein L₂ is -CH₂-.

8. The compound, or the isomer or pharmaceutically acceptable salt thereof according to claim 7, wherein R₁ is selected from the group consisting of oxetanyl, tetrahydrofuranyl, tetrahydropyranyl, Hexapyridyl, morpholinyl and dioxanyl, which is optionally substituted by 1, 2 or 3 Rₐ.

9. The compound ,or the isomer or pharmaceutically acceptable salt thereof according to claim 8, wherein R₁ is selected from the group consisting of 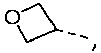 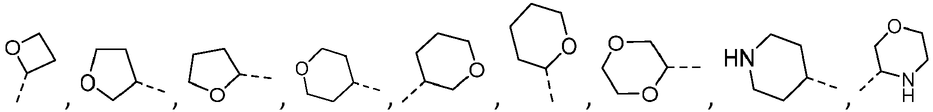 and 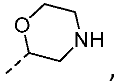 which is optionally substituted by 1, 2 or 3 Rₐ.

10. The compound, or the isomer or pharmaceutically acceptable salt thereof according to any one of claims 7 or 9, wherein the structural unit 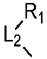 is selected from the group consisting of 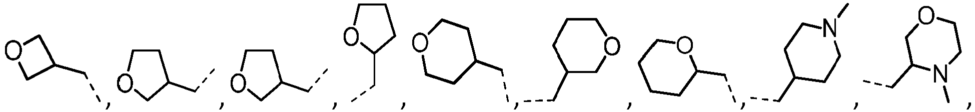 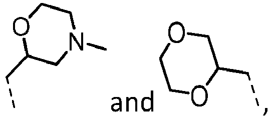 and preferably, wherein the structural unit 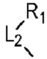 is selected from the group consisting of

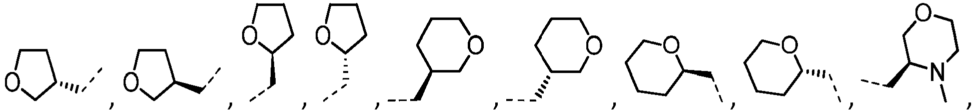

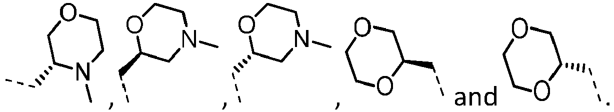

11. The compound, or the isomer or pharmaceutically acceptable salt thereof according to any one of claims 1 to 9, wherein the compound, or the isomer or pharmaceutically acceptable salt thereof is selected from the group consisting of 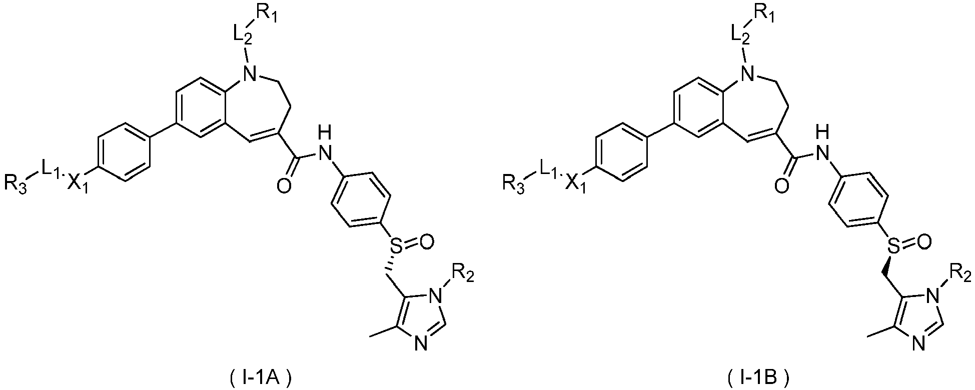 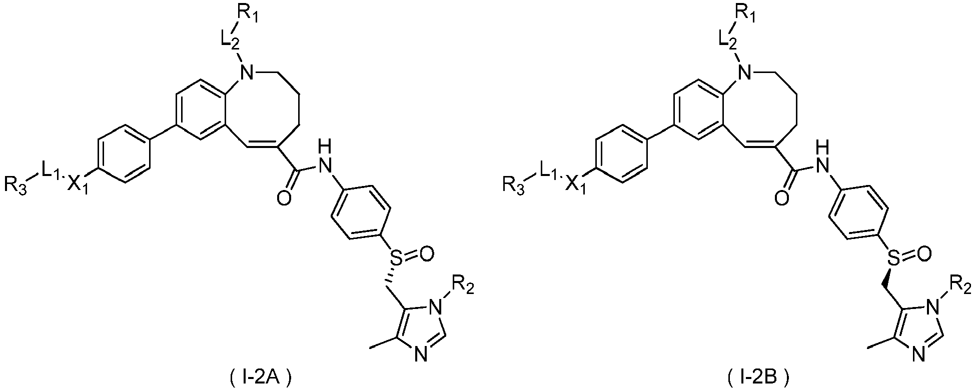 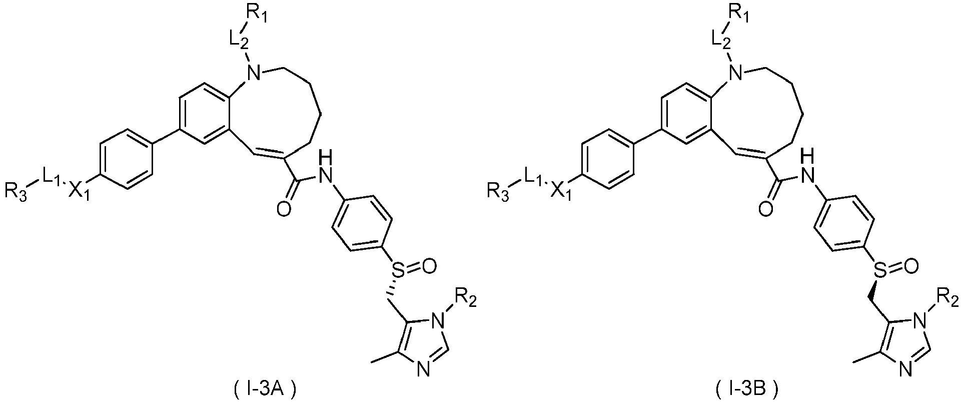 wherein, R₁, R₂, R₃, X₁, L₁, L₂ are defined in any one of claims 1 to 9.

12. A compound according to claim 1, or an isomer or pharmaceutically acceptable salt thereof of the following formula: 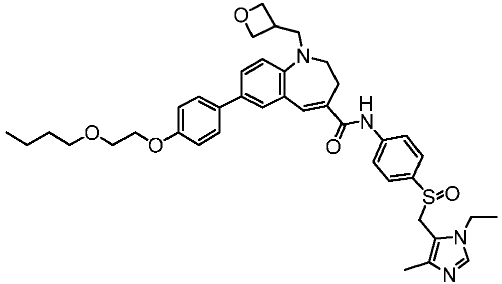 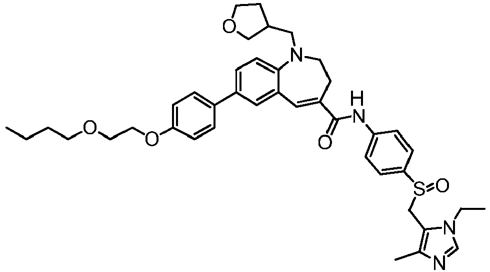 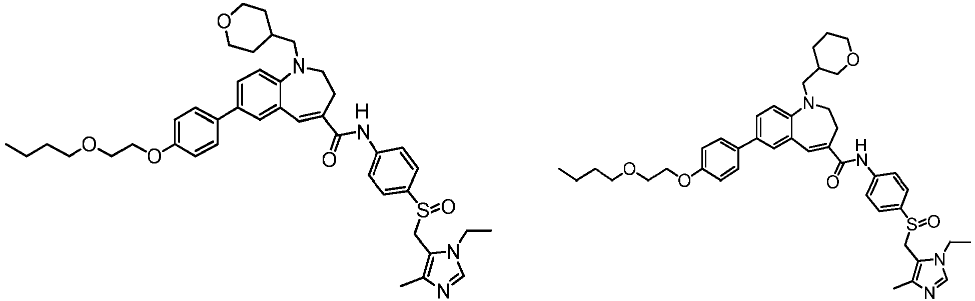 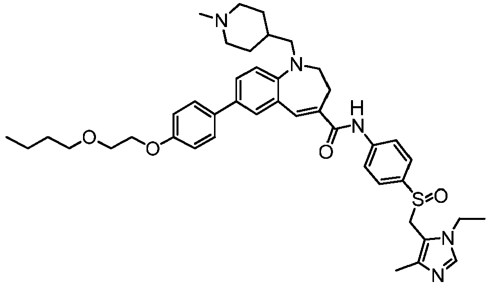 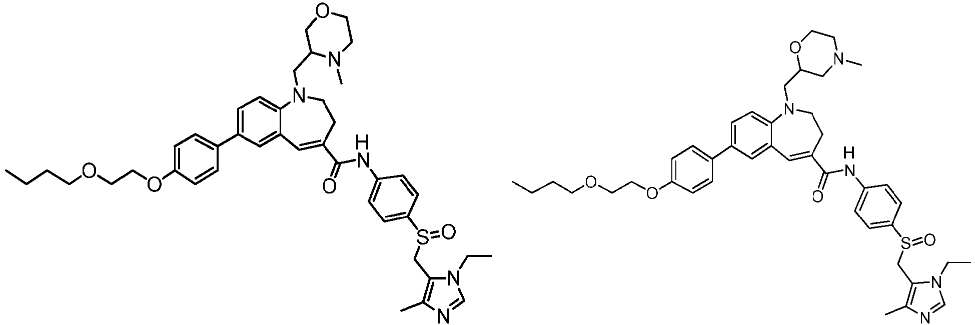 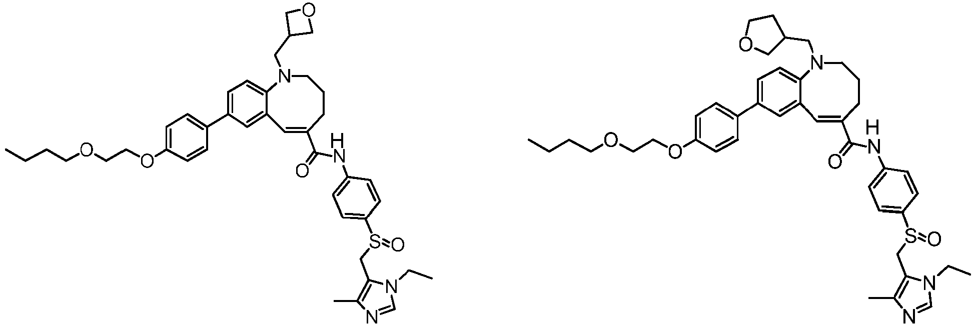 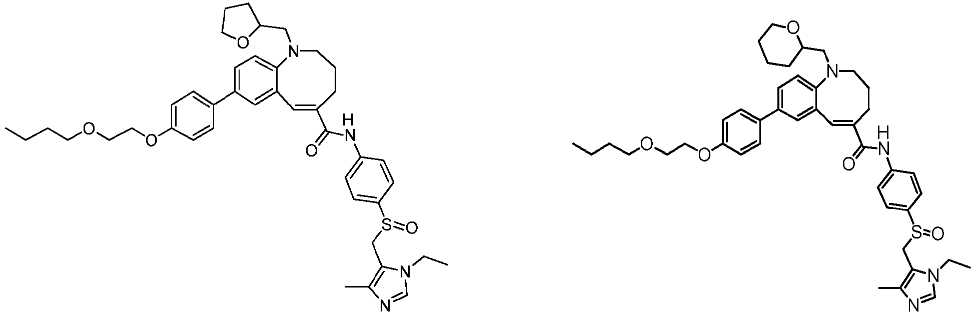 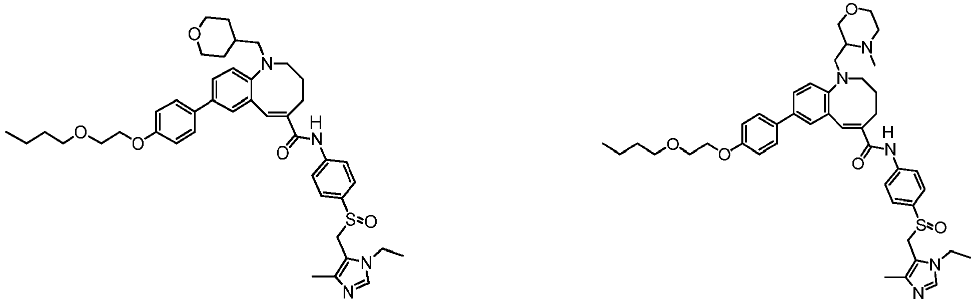 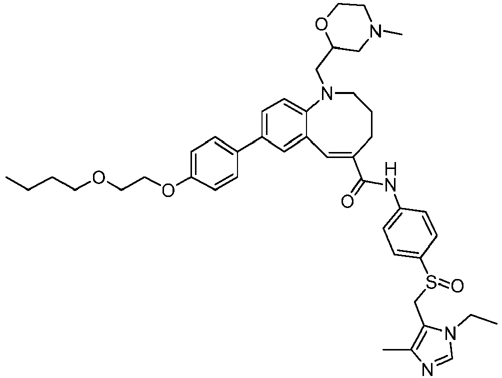 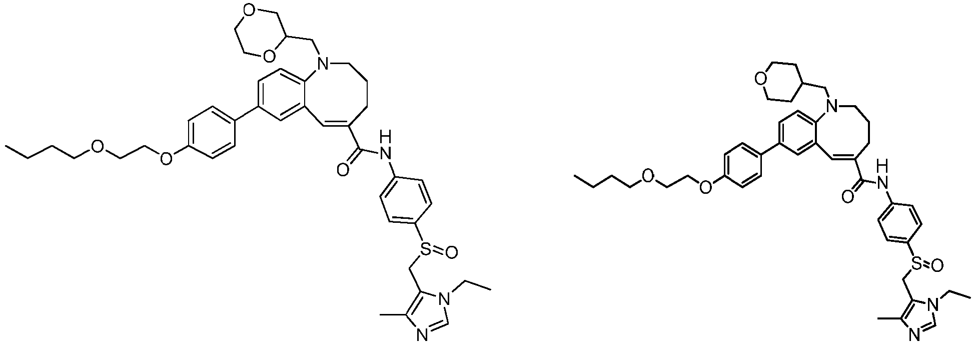 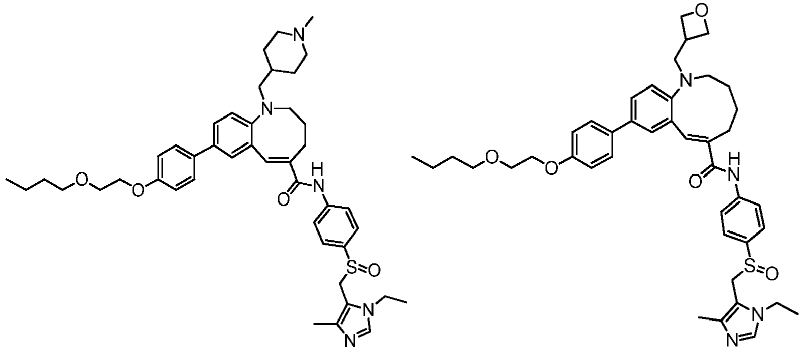 or 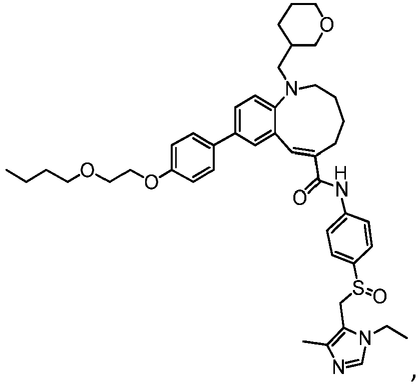 and preferably, wherein the compound, or the isomer or pharmaceutically acceptable salt thereof is selected from the group consisting of 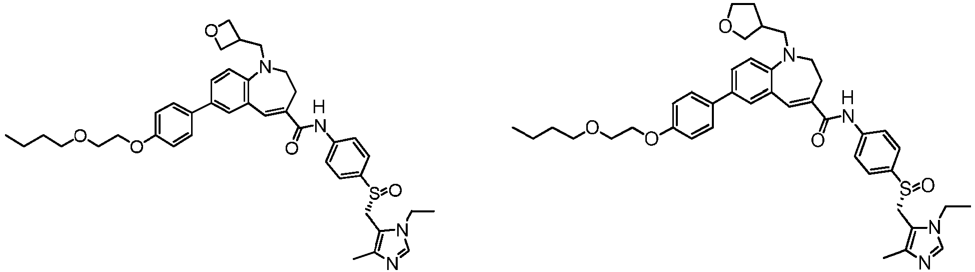 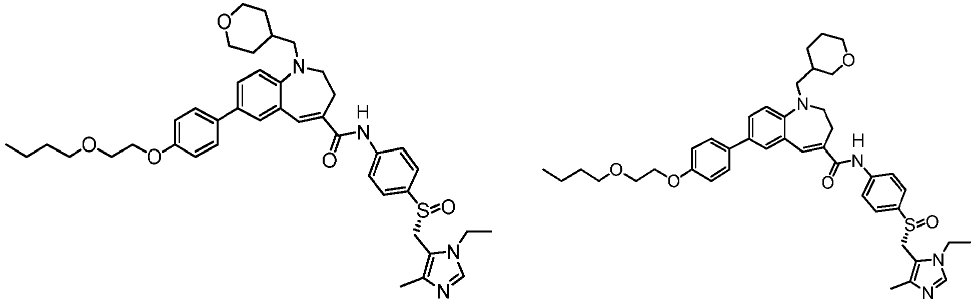 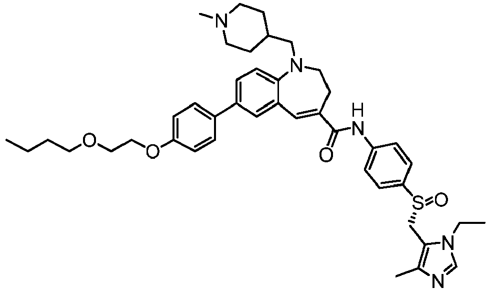 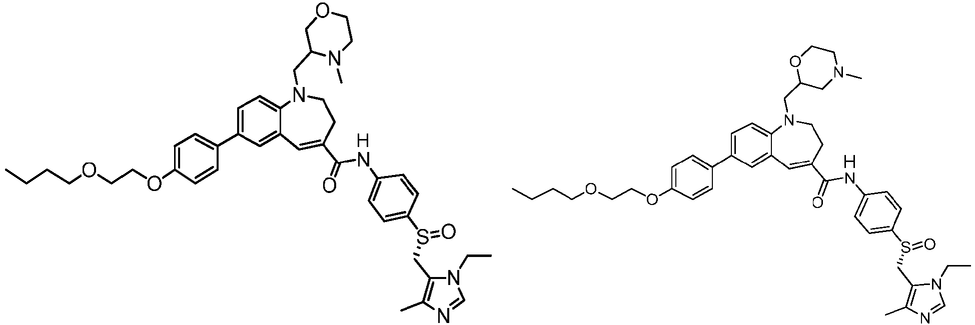 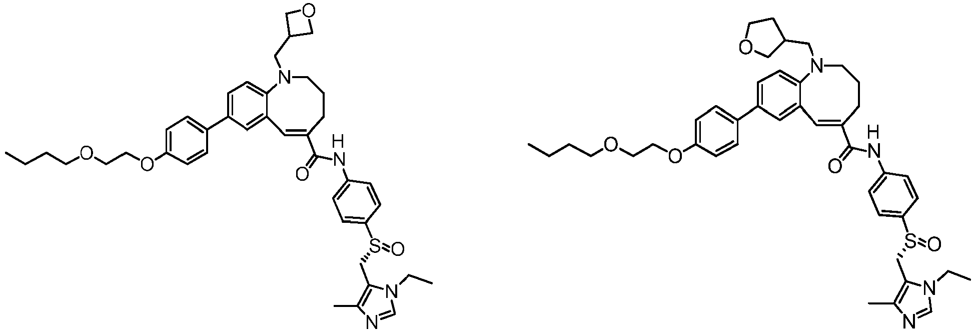 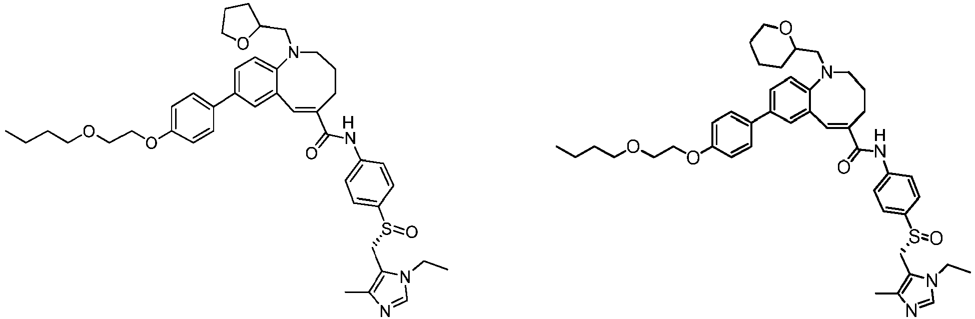 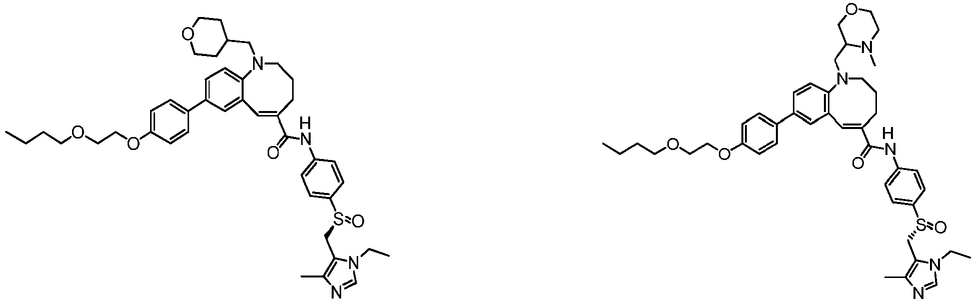 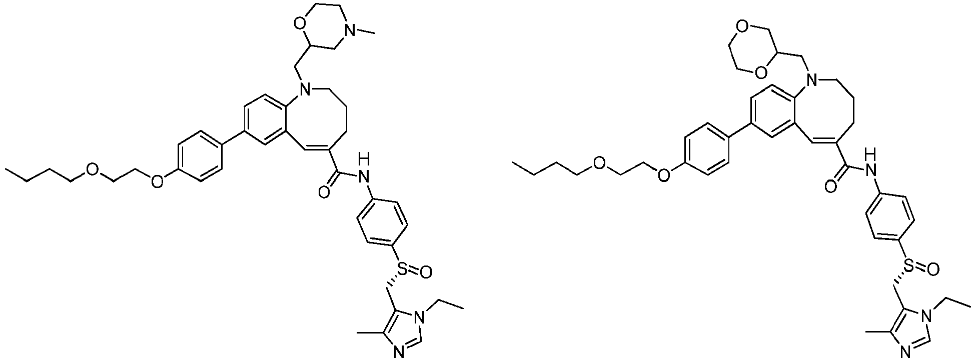 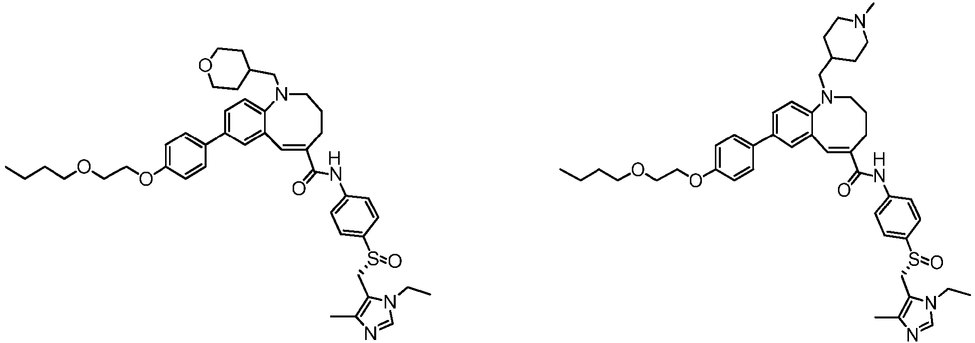 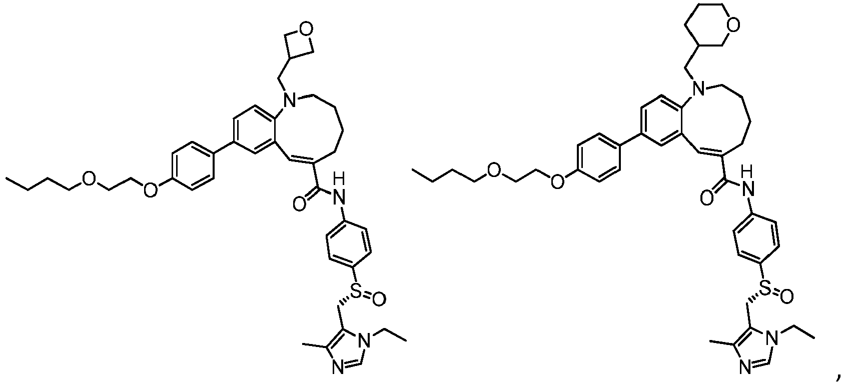 and still preferably, wherein the compound, or the isomer or pharmaceutically acceptable salt thereof is selected from the group consisting of

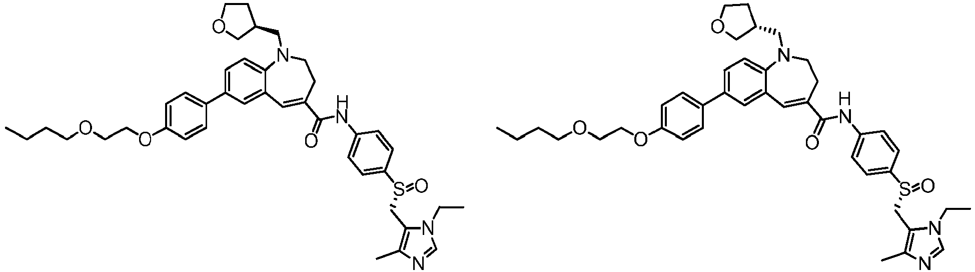

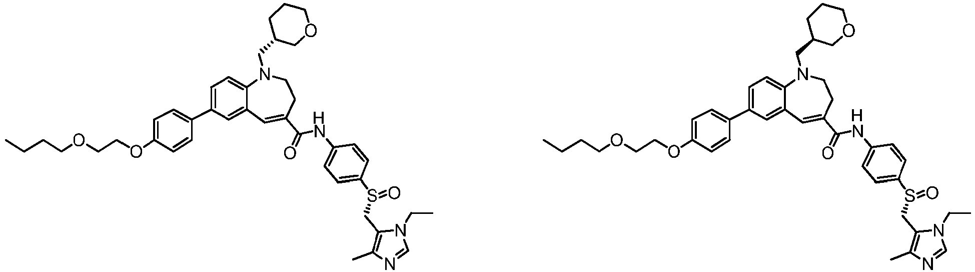

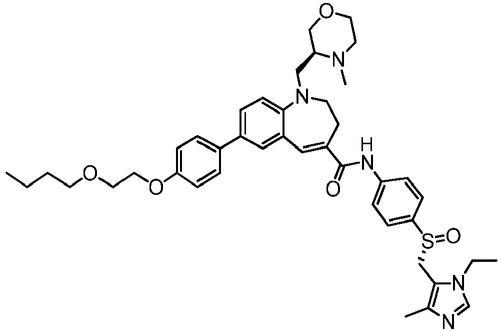

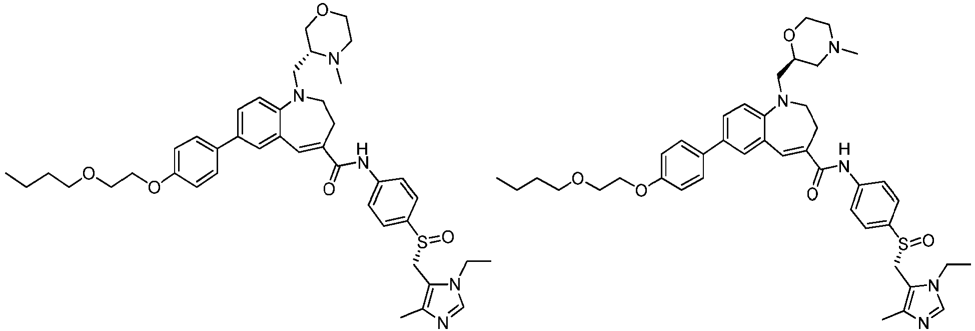

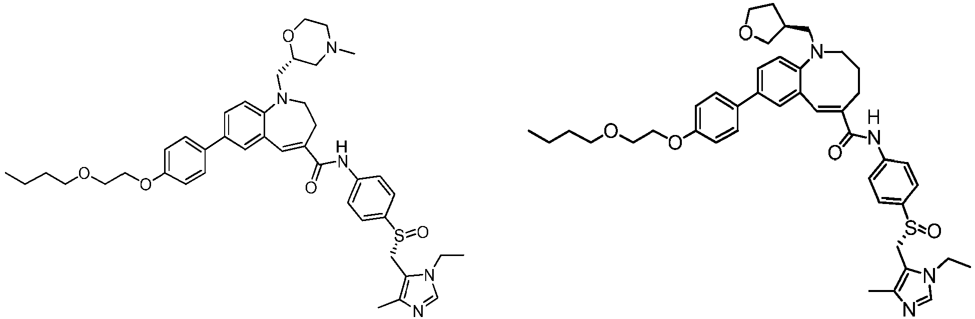

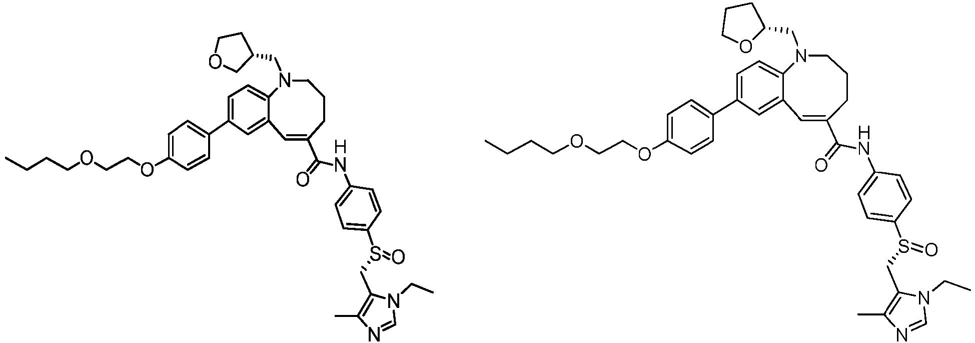

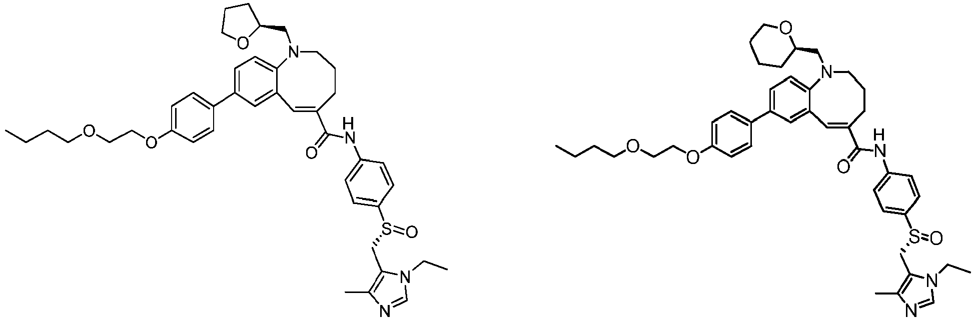

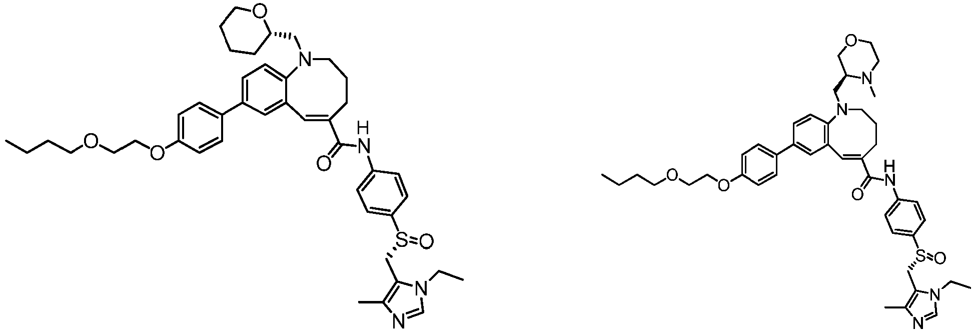

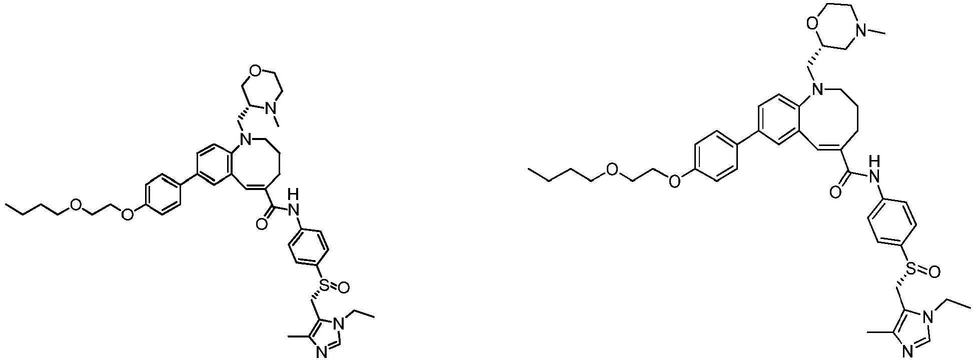

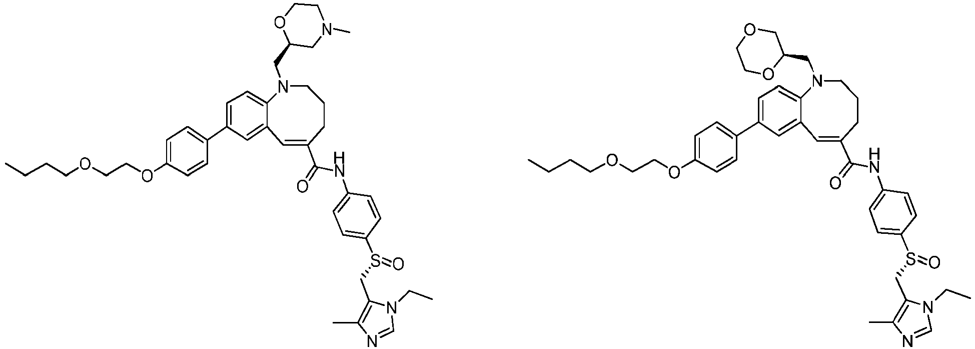

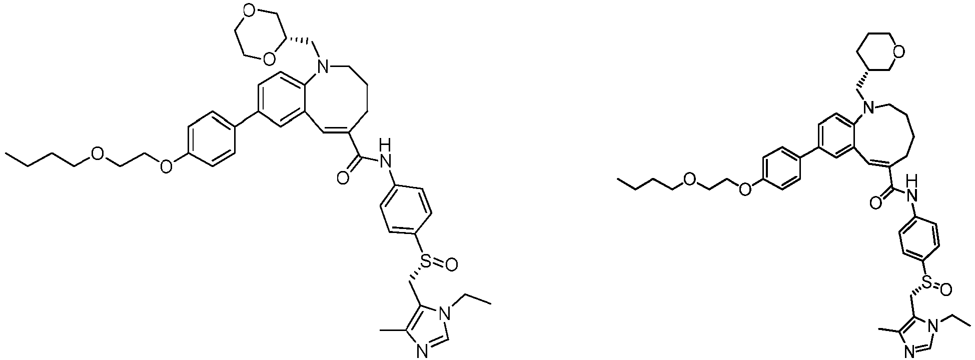

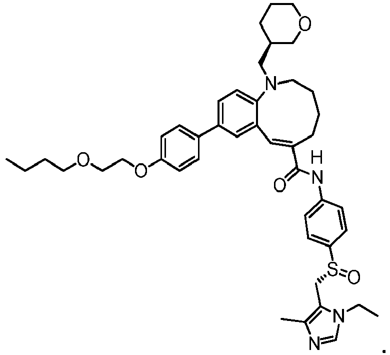

13. A pharmaceutical composition, comprising a therapeutically effective amount of the compound, or the isomer or pharmaceutically acceptable salt thereof according to any one of claims 1 to 12 as an active ingredient, and a pharmaceutically acceptable carrier.

14. The compound, or the isomer or pharmaceutically acceptable salt thereof according to any one of claims 1 to 12, or the pharmaceutical composition according to claim 13, for use in the treatment of CCR2/CCR5 antagonist-related diseases.

15. The compound, or the isomer or pharmaceutically acceptable salt thereof according to any one of claims 1 to 12, or the pharmaceutical composition according to claim 13, for use in the treatment of non-alcoholic fatty hepatitis.

## Patentansprüche

1. Verbindung oder ihr Isomer oder pharmakologisch annehmbares Salz von Gl. (I), 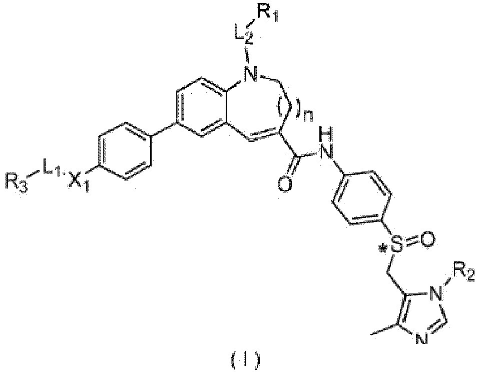 Worin
n von 1, 2 und 3;
R₁ ist eine 4 bis 6 gliedrige Hetero-Alkylgruppe, die wahlweise durch 1, 2 oder 3 Rₐ substituiert ist,
R₂ ist eine optionale C₁₋₆ Alkylgruppe, die durch 1, 2 oder 3 R_{b} substituiert ist,
R₃ ist eine C₁₋₆ Alkoxygruppe, die wahlweise durch 1, 2 oder 3 R_{c} ersetzt wird;
X₁ wird aus O, S und -NH- ausgewählt;
L₁ ist - (CRR)ₘ-;
L₂ ist -CRR-;
m von 1, 2, 3 und 4;
Rₐ, R_{b} und R_{c} wurden unabhängig voneinander aus H, F, Cl, Br, I, OH, NH₂, CN und CH₃ ausgewählt
Jedes R wurde unabhängig voneinander aus H, F, Cl, Br, I, OH, NH₂, CN und CH ausgewählt;
4 bis 6-gliedrige Heterocycloalkylgruppen enthalten 1, 2, 3 oder 4 Heteroatomgruppen, die unabhängig voneinander aus -NH-, -O-S- und N ausgewählt wurden; und wenn seine Isomere geometrische Isomere oder Stereoisomere sind, Das Schwefelatom mit "*" ist ein chirales Schwefelatom, das mit einem einzelnen Enantiomer von (R) oder (S) vorhanden oder angereichert ist.

2. Eine Verbindung oder ein Isomer davon oder ein pharmazeutisch verträgliches Salz nach Anspruch 1, wobei R₂ eine C₁₋₃-Alkylgruppe ist, die gegebenenfalls durch 1, 2 oder 3 R_{b} substituiert ist, und vorzugsweise wobei R₂ CH₂CH₃ ist.

3. Eine Verbindung oder ihr Isomer oder ein pharmazeutisch verträgliches Salz nach Anspruch 1 oder 2, wobei R₃ eine C₄₋₆-Alkoxygruppe ist, die wahlweise durch 1, 2 oder 3 R_{c} substituiert ist.

4. Eine Verbindung oder ein Isomer davon oder ein pharmazeutisch verträgliches Salz nach Anspruch 3, wobei R₃ 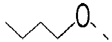 ist.

5. Eine Verbindung oder ihr Isomer oder ein pharmazeutisch verträgliches Salz nach den Ansprüchen 1 oder 2 oder 4, wobei L 1 -CH₂CH₂- ist.

6. Eine Verbindung oder ein Isomer oder ein pharmazeutisch verträgliches Salz nach einem der Ansprüche 1 bis 5, wobei die Struktureinheit 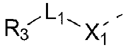 ist

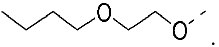

7. Verbindung nach Anspruch 1, sein Isomer oder ein pharmazeutisch akzeptables Salz, wobei L₂ -CH₂- ist.

8. Verbindung oder sein Isomer oder ein pharmazeutisch verträgliches Salz nach Anspruch 7, wobei R₁ ausgewählt ist aus einer Oxetyl-, Tetrahydrofuranyl-, Tetrahydropyranyl-, Hexapyridin-, Morpholin- und Dioxetylhexangruppe, gegebenenfalls substituiert durch 1, 2 oder 3 Rₐ.

9. Eine Verbindung oder ein Isomer davon oder ein pharmazeutisch verträgliches Salz nach Anspruch 8, wobei R₁ aus 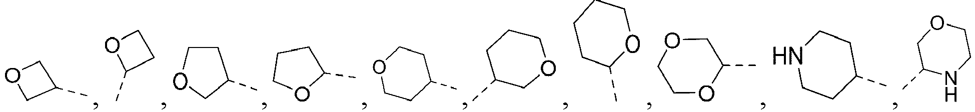 und 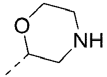 ausgewählt und wahlweise durch 1, 2 oder 3 Rₐ substituiert wird.

10. Die Verbindung oder ihr Isomer oder ein pharmazeutisch verträgliches Salz nach einem der Ansprüche 7 oder 9, wobei die Struktureinheit 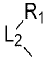 aus 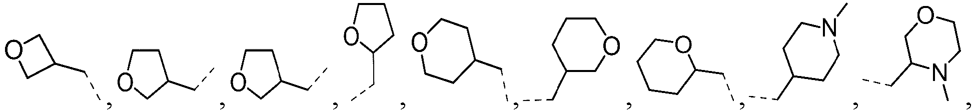 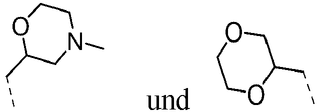 und vorzugsweise die Struktureinheit 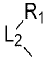 aus 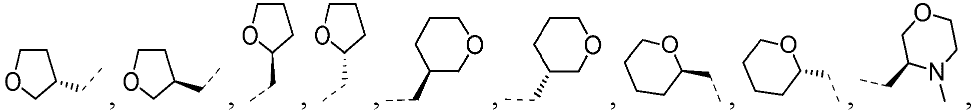 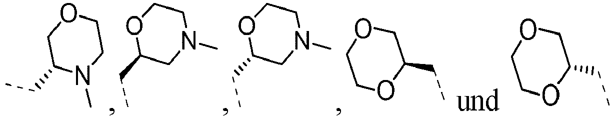 ausgewählt ist

11. Verbindung oder ihr Isomer oder ein pharmazeutisch verträgliches Salz nach einem der Ansprüche 1 bis 9, wobei die Verbindung oder ihr Isomer oder ein pharmazeutisch verträgliches Salz aus 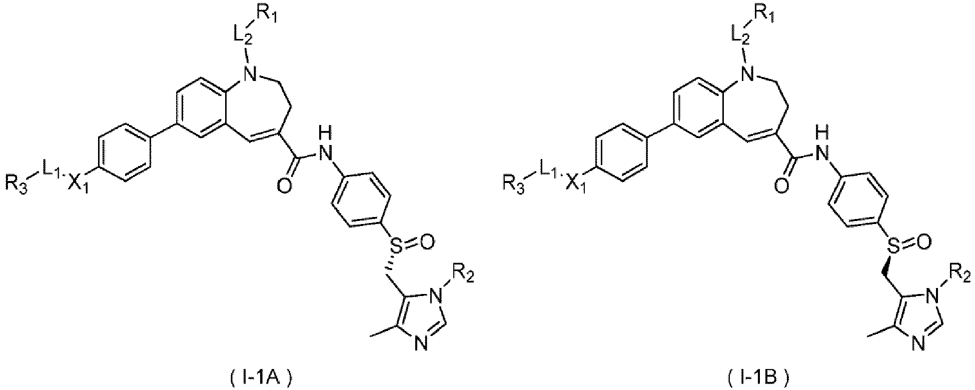 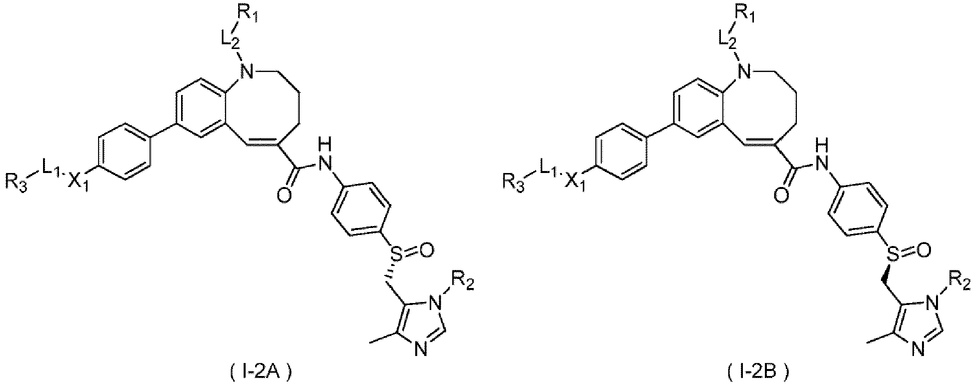 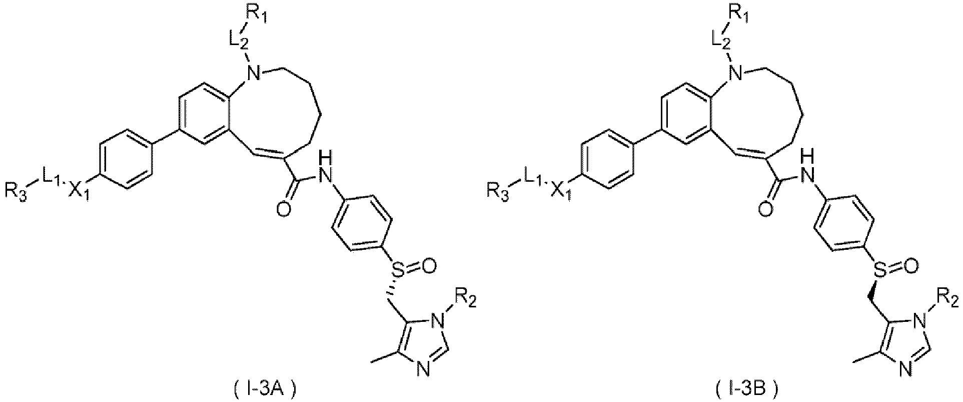 ausgewählt ist. Wobei R₁, R₂, R₃, X₁, L₁, L₂ in einem der Ansprüche 1 bis 9 definiert sind.

12. Isomere oder pharmazeutisch verträgliche Salze der Verbindung oder die folgende Formel nach Anspruch 1: 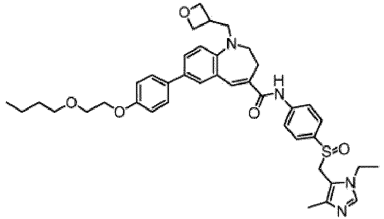 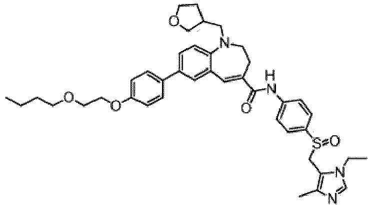 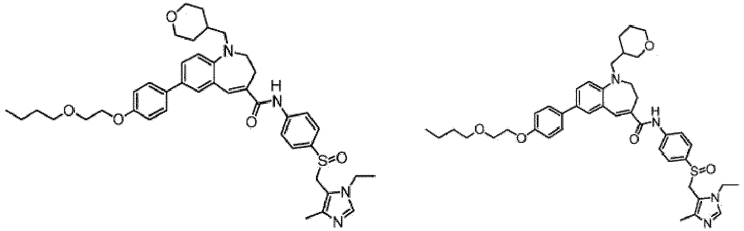 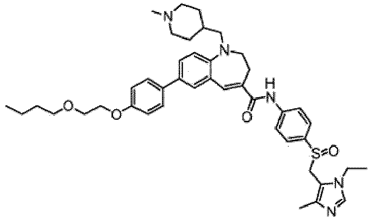 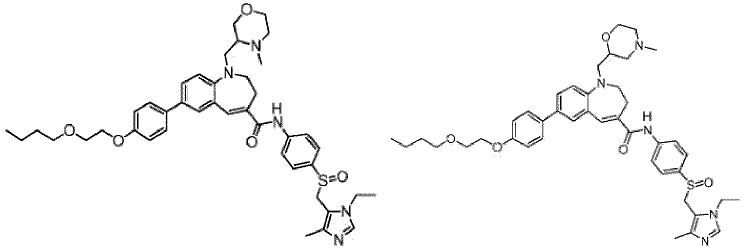 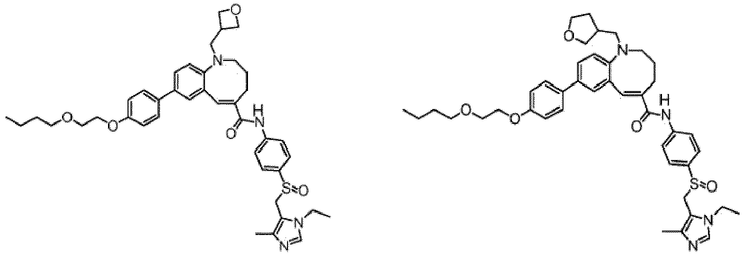 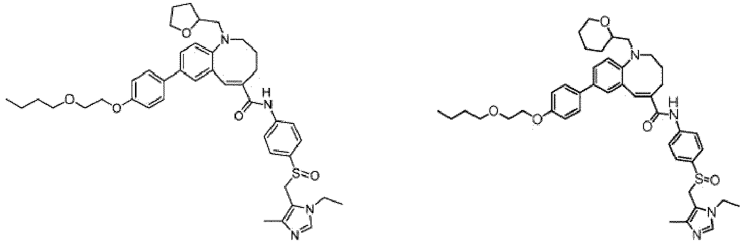 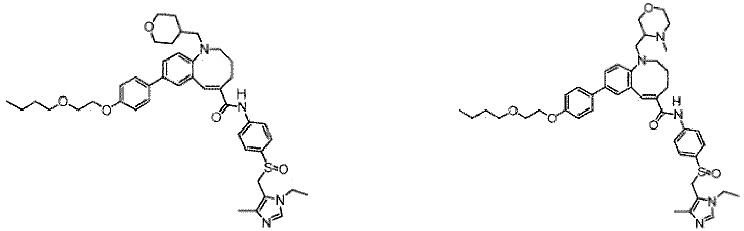 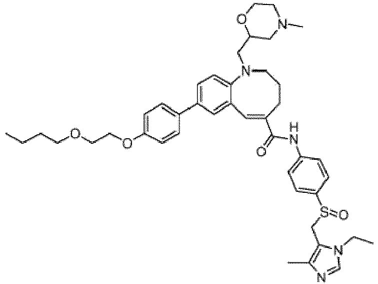 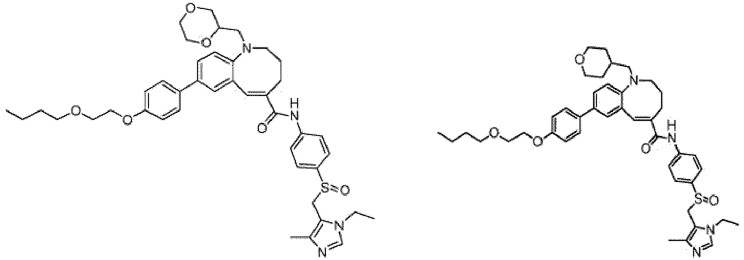 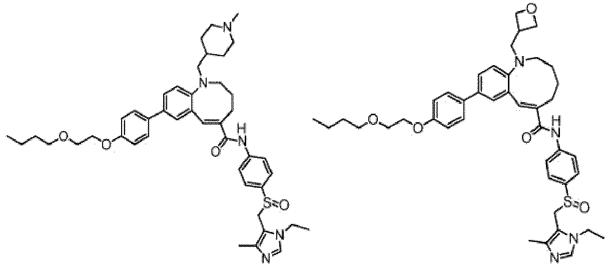 or 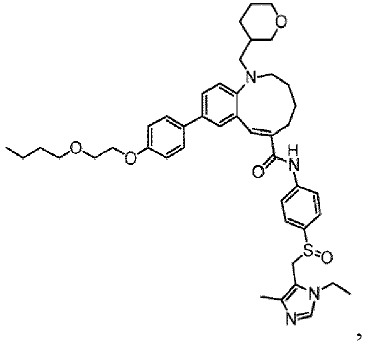 und vorzugsweise, wobei die Verbindung oder ihre Isomere oder pharmakologisch akzeptablen Salze aus 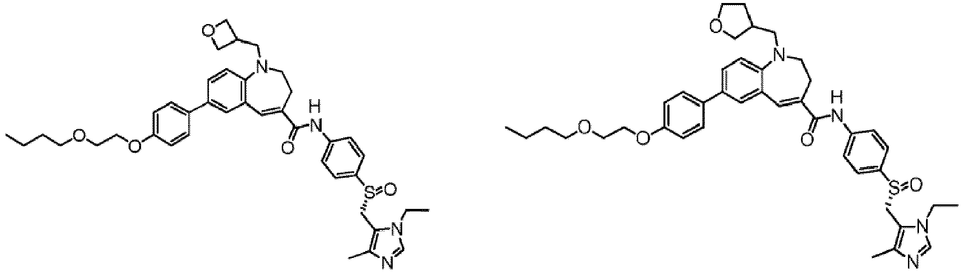 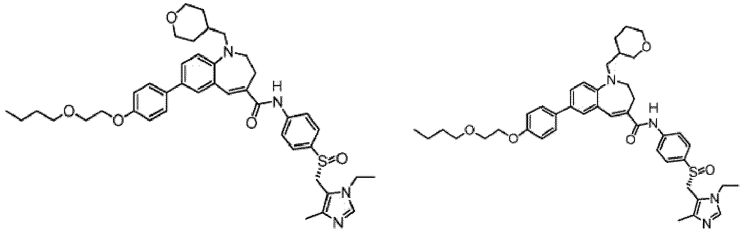 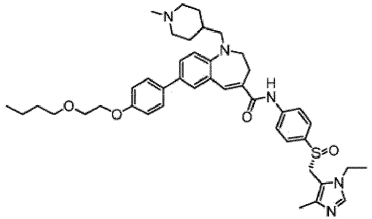 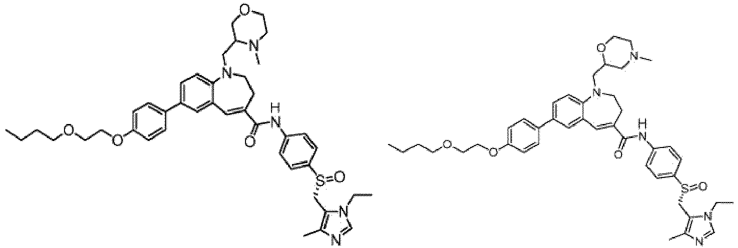 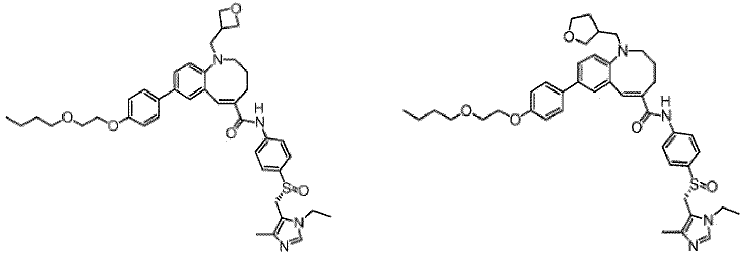 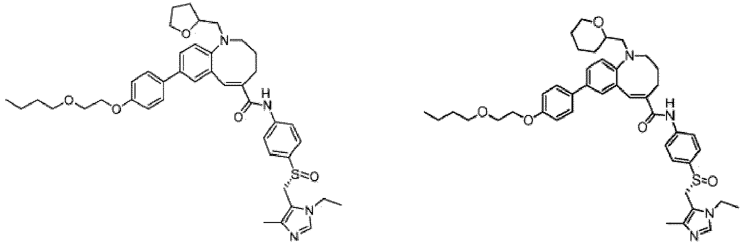 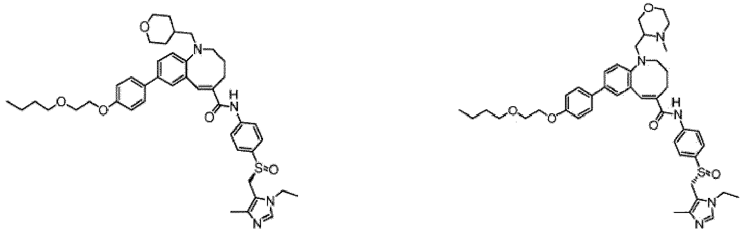 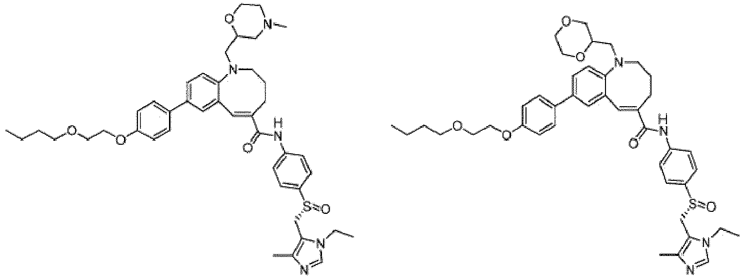 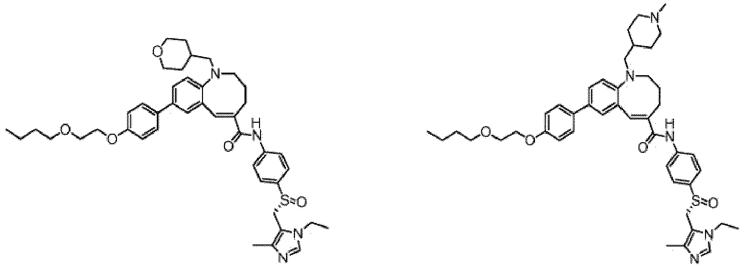 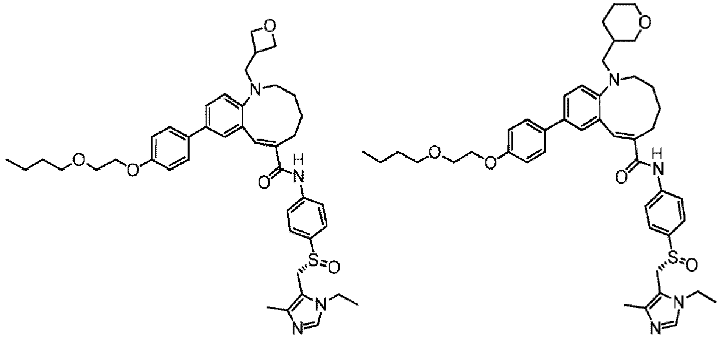 ausgewählt sind, und immer noch bevorzugt, wobei die Verbindung oder ihre Isomere oder pharmazeutisch verträgliche Salze aus 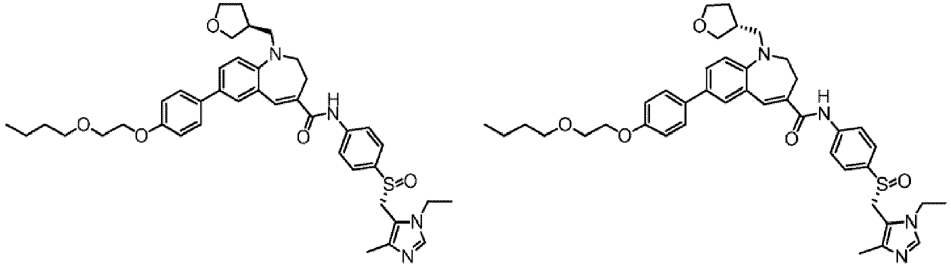 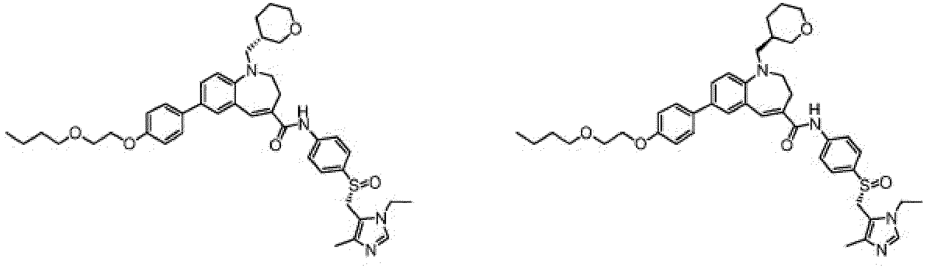 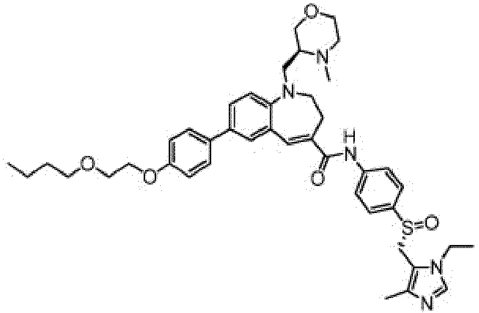 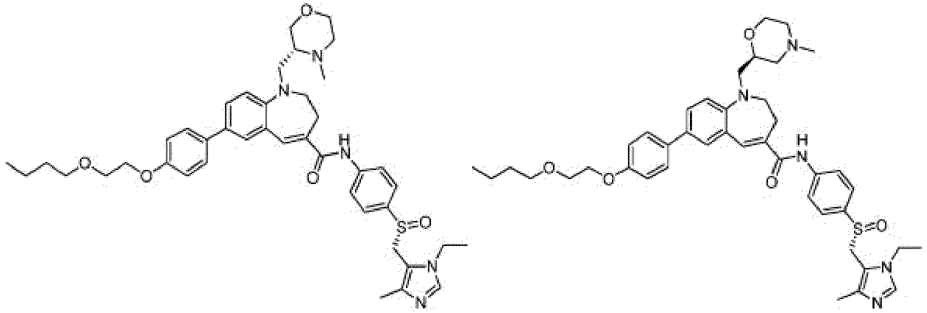 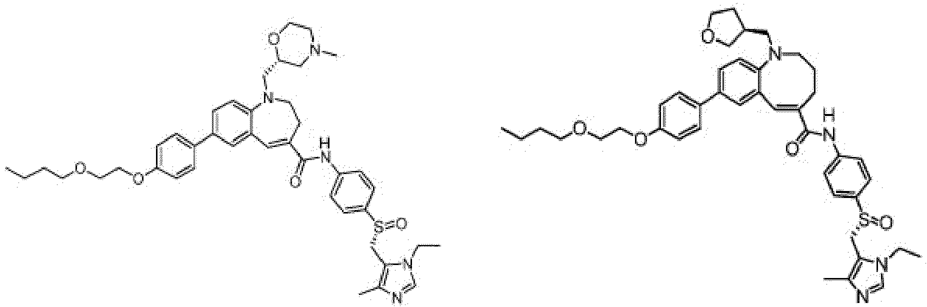 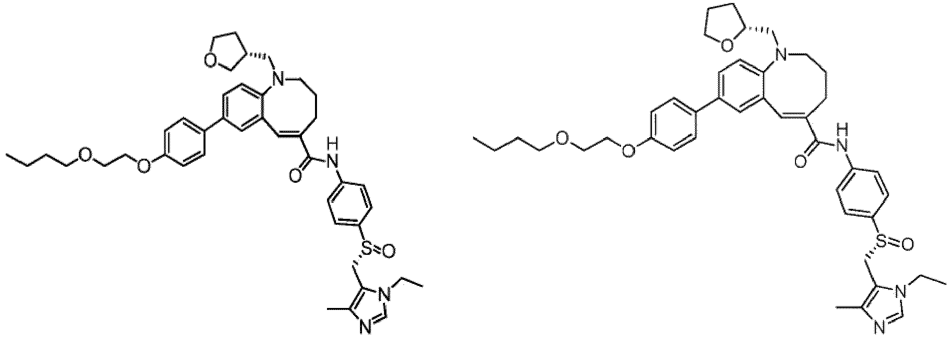 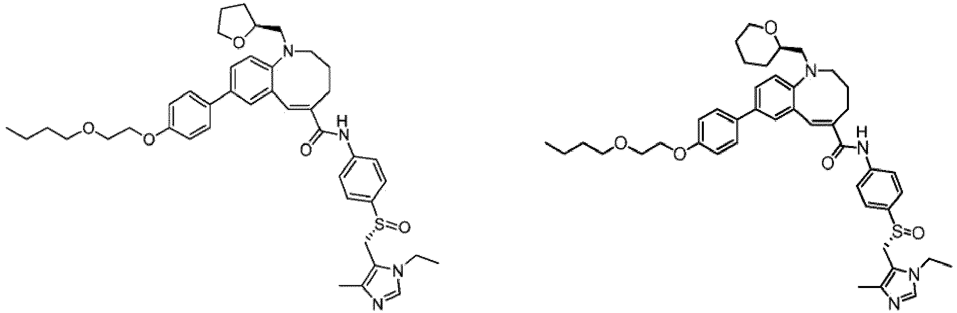 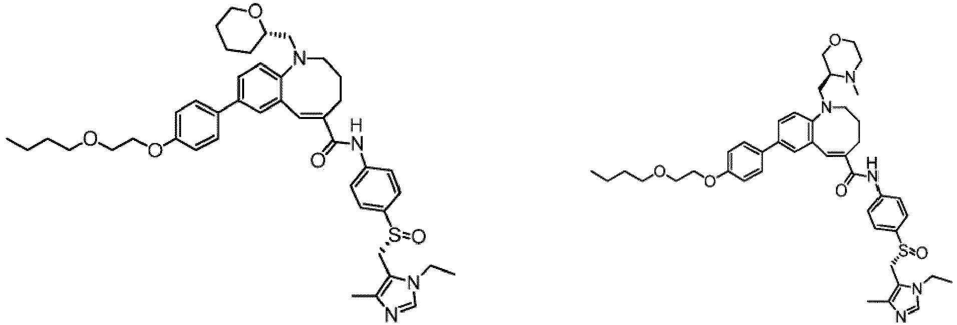 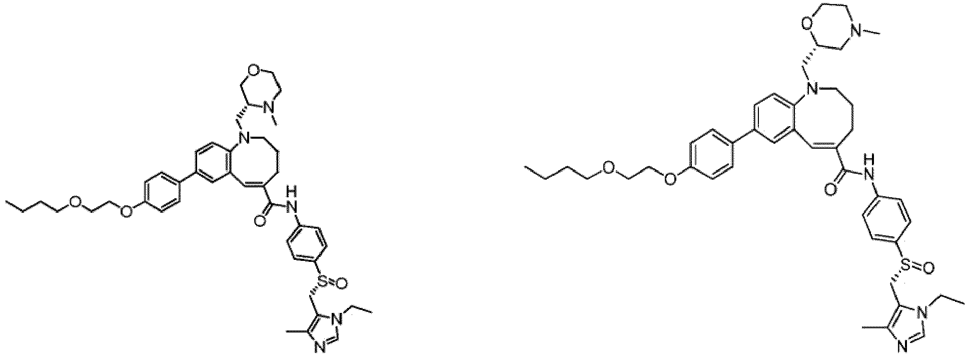 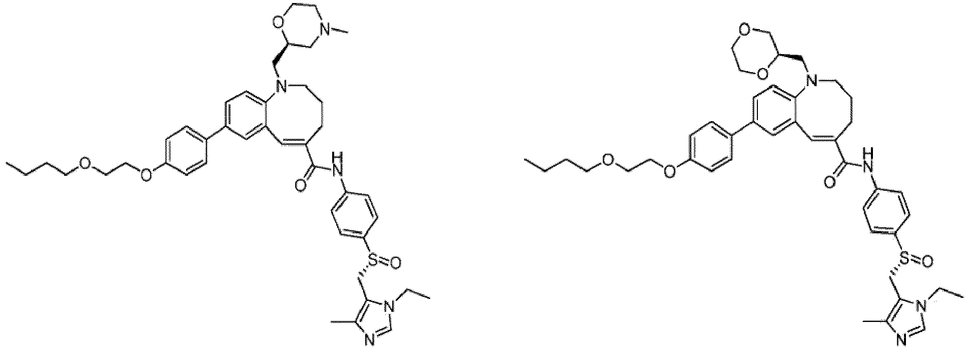 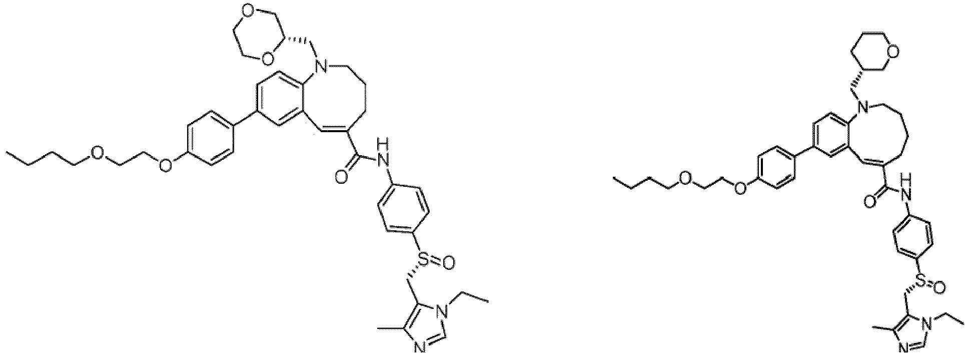 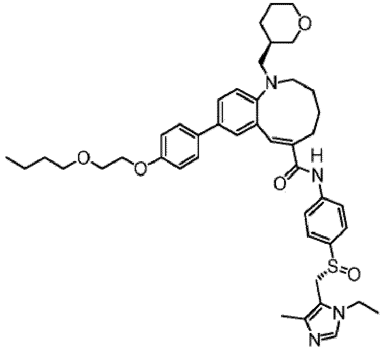 ausgewählt werden.

13. Pharmazeutische Zusammensetzung, umfassend eine therapeutisch wirksame Menge einer Verbindung oder ihres Isomers oder eines pharmazeutisch verträglichen Salzes als Wirkstoff gemäß einem der Ansprüche 1 bis 12 und einen pharmazeutisch verträglichen Träger.

14. Eine Verbindung oder ein Isomer davon oder ein pharmazeutisch verträgliches Salz nach einem der Ansprüche 1 bis 12 oder eine pharmazeutische Zusammensetzung nach Anspruch 13 zur Behandlung einer CCR2/CCR5-Antagonisten-verwandten Krankheit.

15. Verbindung oder ein Isomer davon oder ein pharmazeutisch verträgliches Salz nach einem der Ansprüche 1 bis 12 oder eine Zusammensetzung eines Arzneimittels nach Anspruch 13, das zur Behandlung der nichtalkoholischen Steatohepatitis verwendet wird.

## Revendications

1. Un composé de la formule (I) ou un isomère ou un sel pharmaceutiquement acceptable de celui-ci, 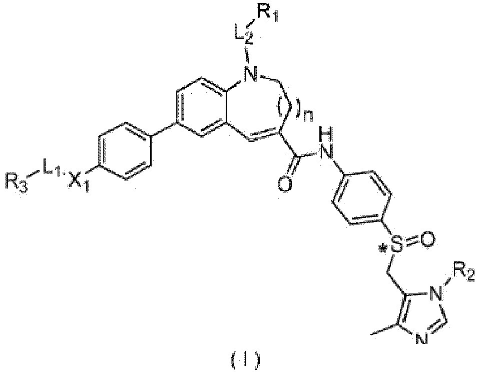 où,
n est sélectionné parmi le groupe constitué de 1, 2 et 3;
R₁ est un hétérocycloalkyle à 4 à 6 membres, qui est éventuellement substitué par 1, 2 ou 3 Rₐ;
R₂ est un alkyle en C₁₋₆, qui est éventuellement substitué par 1, 2 ou 3 R_{b};
R₃ est un alkoxy en C₁₋₆, qui est éventuellement substitué par 1, 2 ou 3 R_{c};
X₁ est sélectionné parmi le groupe constitué de O, S et -NH-;
L₁ est - (CRR)ₘ-;
L₂ est -CRR-;
m est sélectionné parmi le groupe constitué de 1, 2, 3 et 4;
Rₐ, R_{b} et R_{c} sont indépendamment sélectionnés parmi le groupe constitué de H, F, Cl, Br, I, OH, NH₂, CN et CH₃;
chaque R est indépendamment sélectionné parmi le groupe constitué de H, F, Cl, Br, I, OH, NH₂, CN et CH;
l'hétérocycloalkyle à 4 à 6 membres comprend 1, 2, 3 ou 4 hétéroatomes ou groupes hétéroatomiques sélectionnés indépendamment parmi le groupe constitué de -NH-, -O-, -S- et N; où l'isomère est un isomère géométrique ou stéréoisomère; et l'atome de soufre avec "*" est un atome de soufre chiral qui existe sous forme d'un seul énantiomère (R) ou (S) ou est enrichi en l'un des énantiomères.

2. Le composé ou l'isomère ou le sel pharmaceutiquement acceptable selon la revendication 1, où R₂ est un alkyle en C₁₋₃, qui est éventuellement substitué par 1, 2 ou 3 R_{b} et de préférence où R₂ est CH₂CH₃.

3. Le composé ou l'isomère ou le sel pharmaceutiquement acceptable selon la revendication 1 ou 2, où R₃ est un alkoxy en C₄₋₆, qui est éventuellement substitué par 1, 2 ou 3 R_{c}.

4. Le composé ou l'isomère ou le sel pharmaceutiquement acceptable selon la revendication 3, ou R₃ est

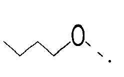

5. Le composé ou l'isomère ou le sel pharmaceutiquement acceptable selon la revendication 1, 2 ou 4, où L₁ est -CH₂CH₂-.

6. Le composé ou l'isomère ou le sel pharmaceutiquement acceptable selon l'une quelconque des revendications 1 à 5, où l'unité structurale 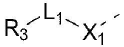 est

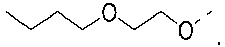

7. Le composé ou l'isomère ou le sel pharmaceutiquement acceptable selon la revendication 1, où L₂ est -CH₂-.

8. Le composé ou l'isomère ou le sel pharmaceutiquement acceptable selon la revendication 7, où R₁ est sélectionné parmi le groupe constitué d'oxétanyl, tétrahydrofuranyl, tétrahydropyranyl, hexapyridyl, morpholinyl et dioxaanyl, qui est éventuellement substitué par 1, 2 ou 3 Rₐ.

9. Le composé ou l'isomère ou le sel pharmaceutiquement acceptable selon la revendication 8, où R₁ est sélectionné parmi le groupe constitué de 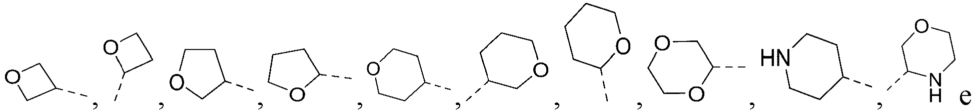 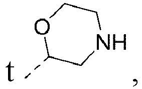 qui est éventuellement substitué par 1, 2 ou 3 Rₐ.

10. Le composé, ou l'isomère ou le sel pharmaceutiquement acceptable de celui-ci selon l'une quelconque des revendications 7 ou 9, où l'unité structurale 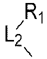 est sélectionnée parmi le groupe constitué de 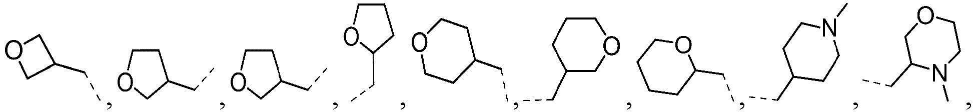 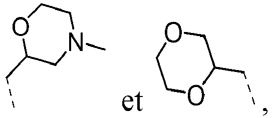 et de préférence, où l'unité structurale 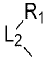 est sélectionnée parmi le groupe constitué de

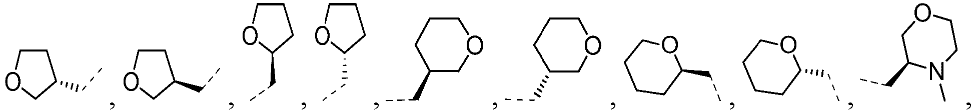

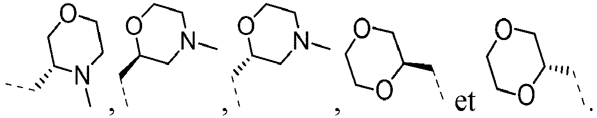

11. Le composé, ou l'isomère ou le sel pharmaceutiquement acceptable de celui-ci selon l'une quelconque des revendications 1 à 9, où le composé, ou l'isomère ou le sel pharmaceutiquement acceptable de celui-ci est sélectionné parmi le groupe constitué de 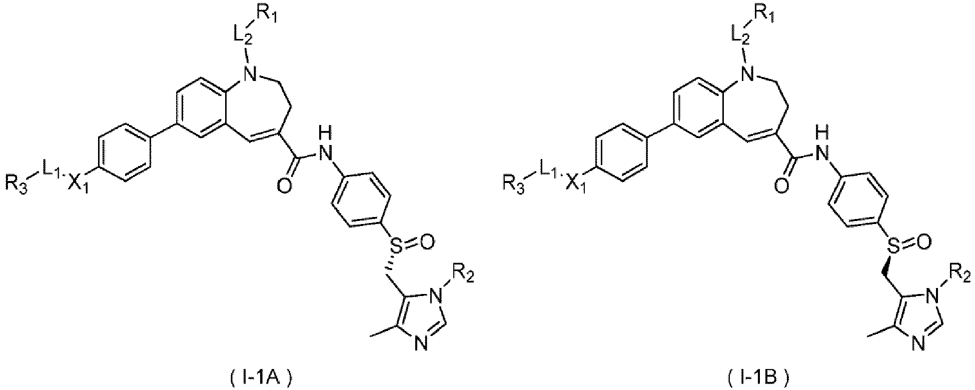 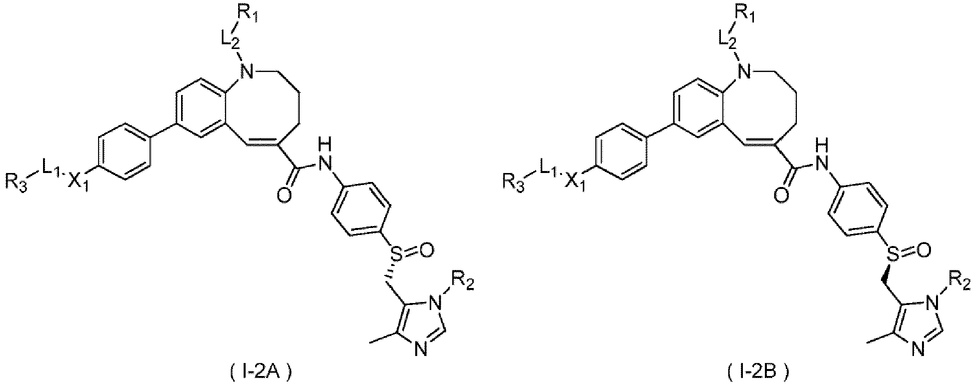 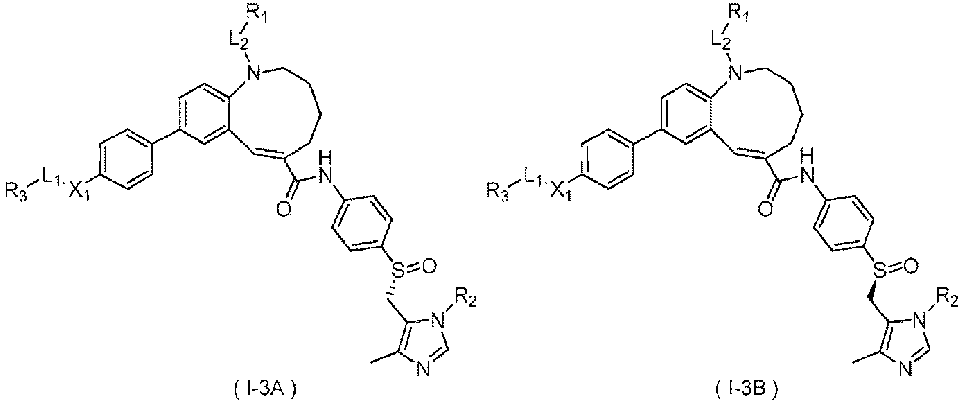 où R₁, R₂, R₃, X₁, L₁, L₂ sont définis dans l'une quelconque des revendications 1 à 9.

12. Un composé selon la revendication 1, ou un isomère ou un sel pharmaceutiquement acceptable de celui-ci de la formule suivante: 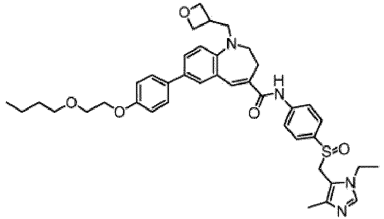 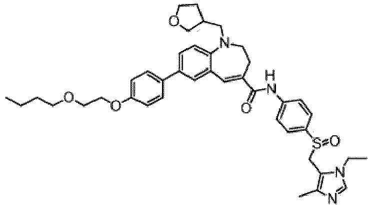 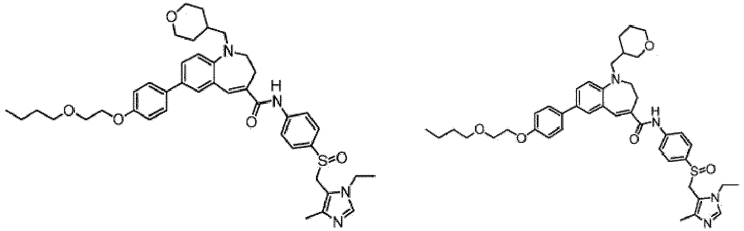 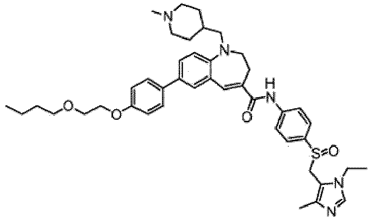 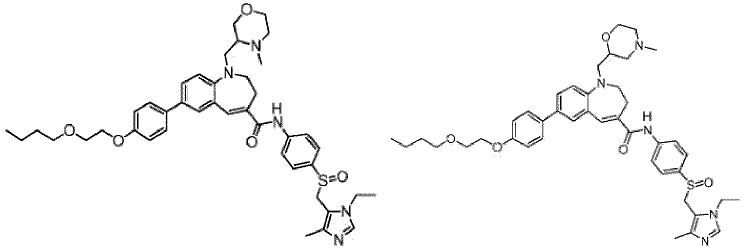 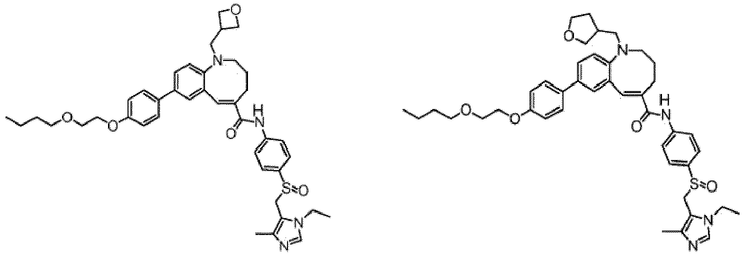 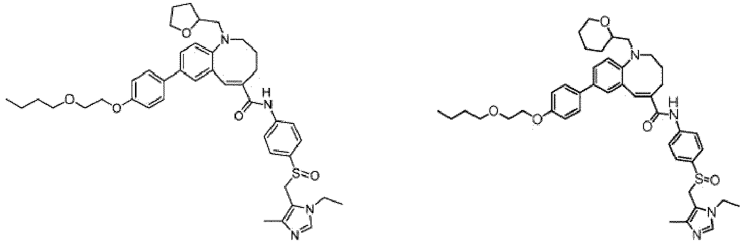 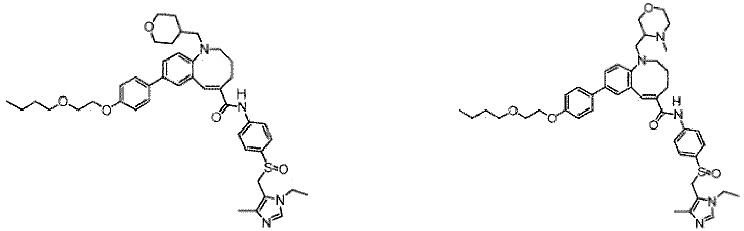 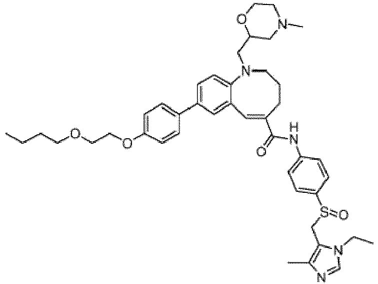 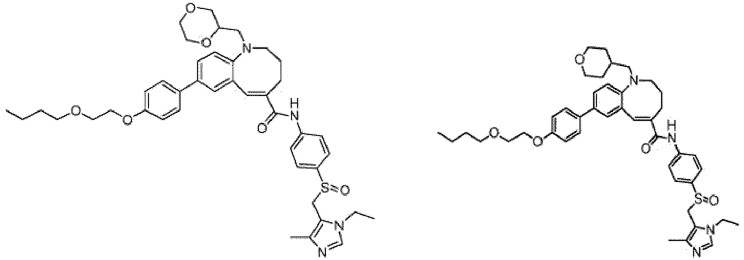 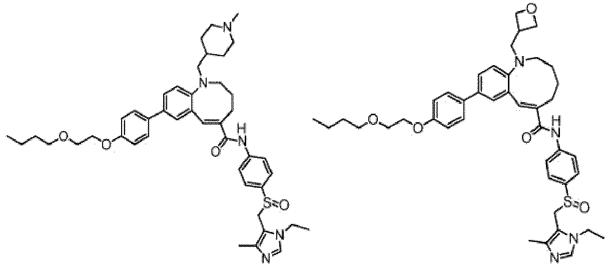 or 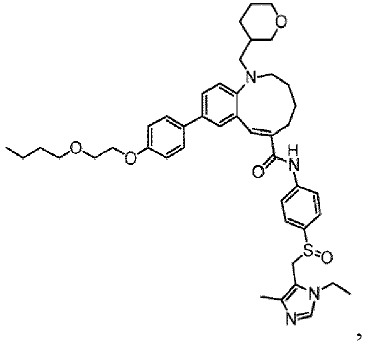 et de préférence, où le composé, ou l'isomère ou le sel pharmaceutiquement acceptable de celui-ci est sélectionné parmi le groupe constitué de 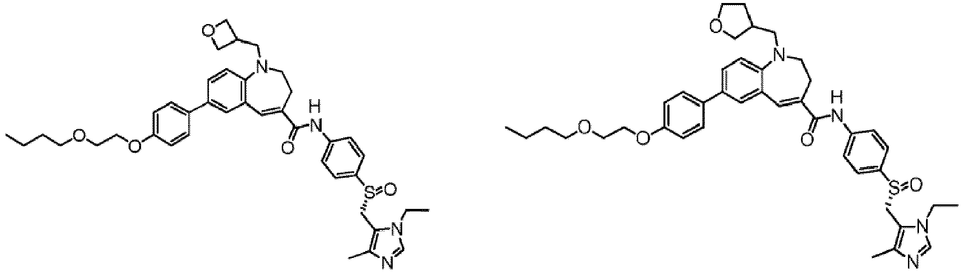 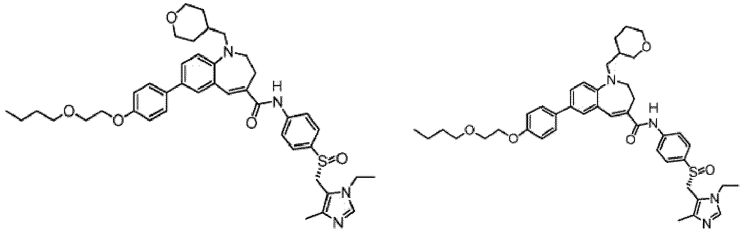 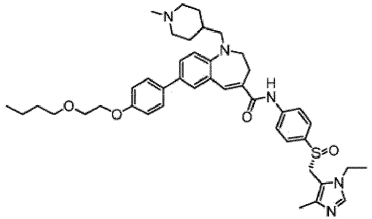 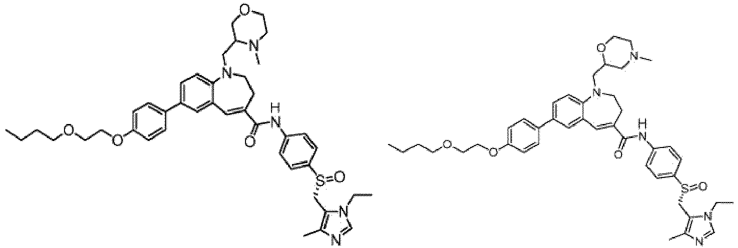 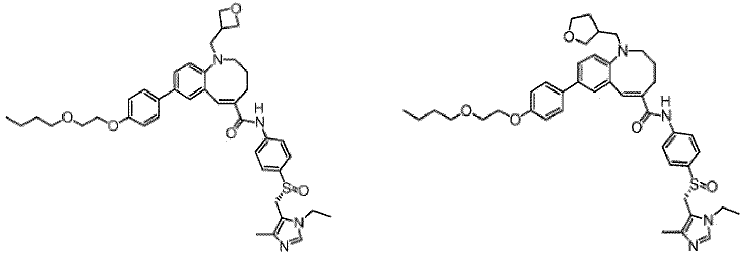 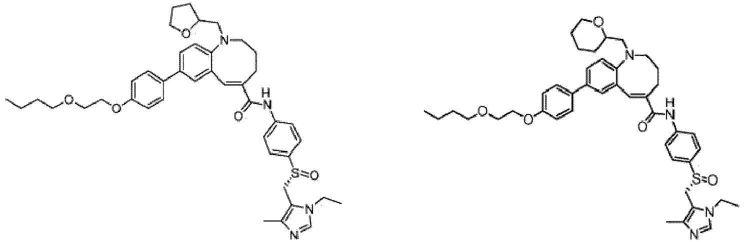 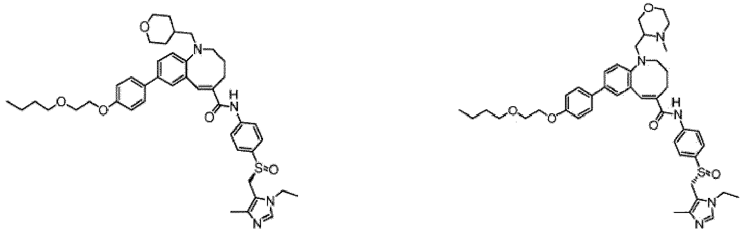 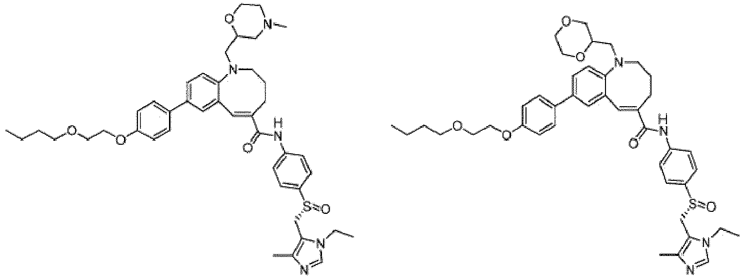 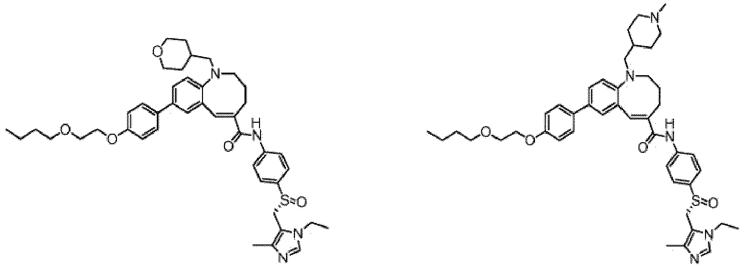 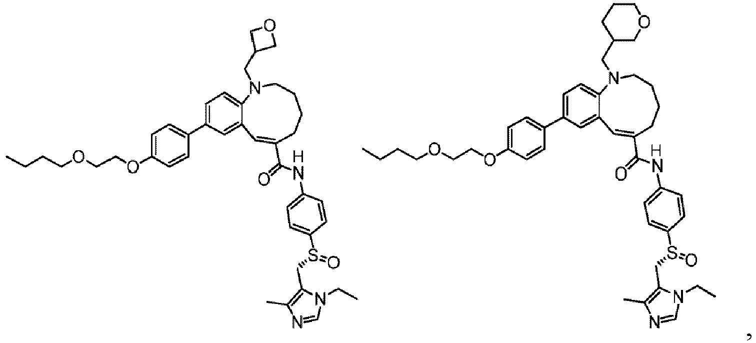 et de manière encore plus préférée, où le composé, ou l'isomère ou le sel pharmaceutiquement acceptable de celui-ci est sélectionné parmi le groupe constitué de

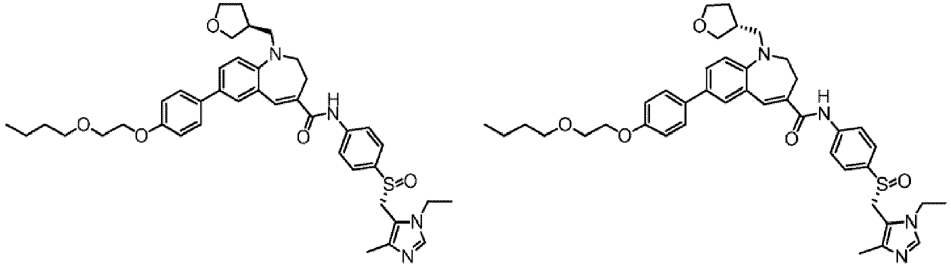

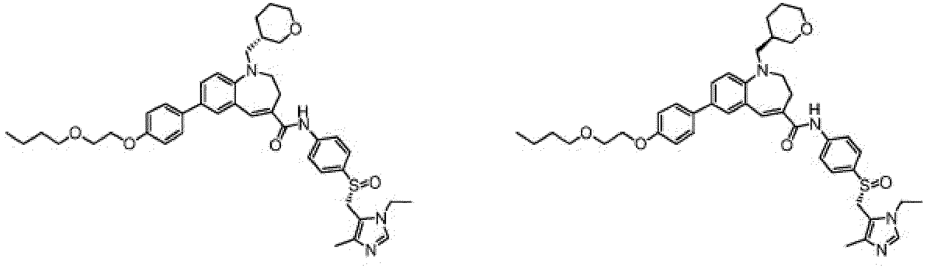

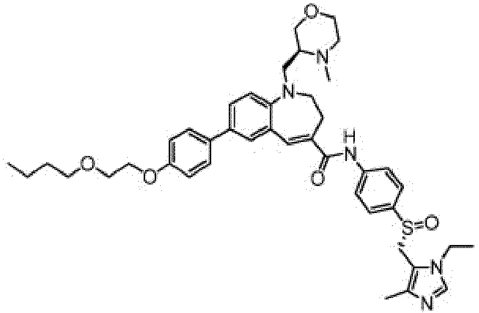

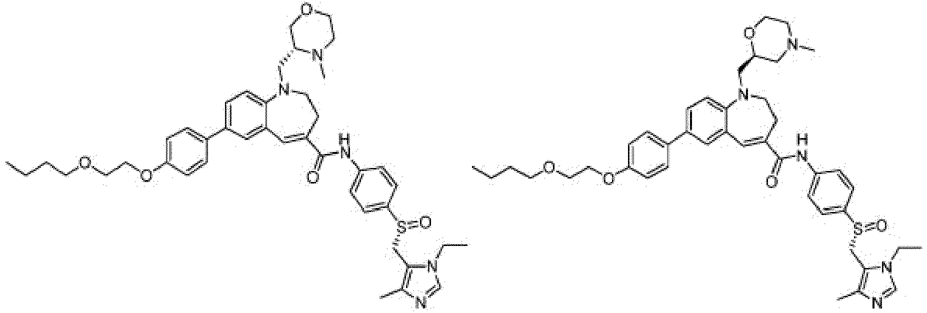

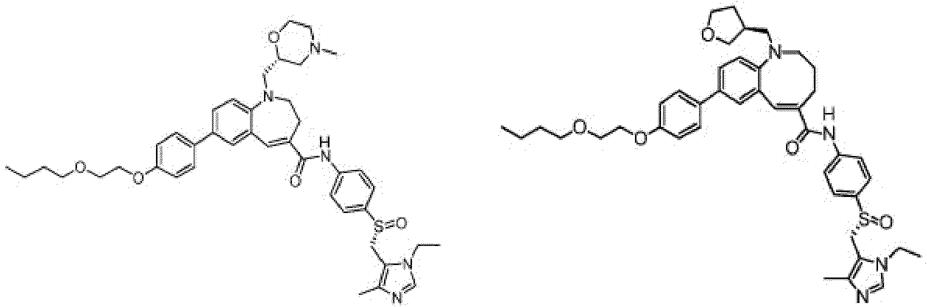

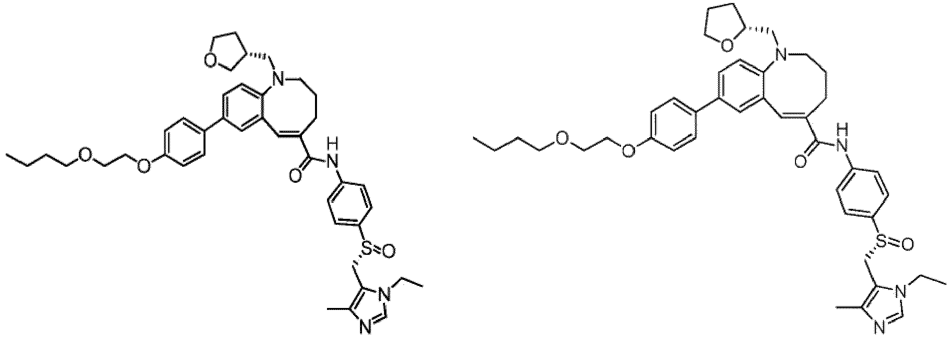

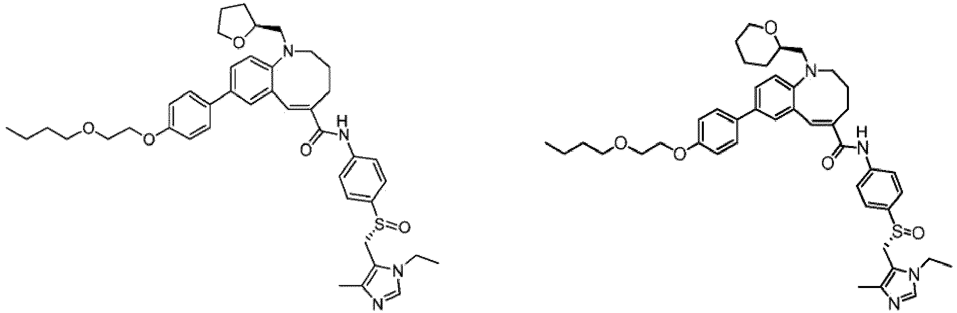

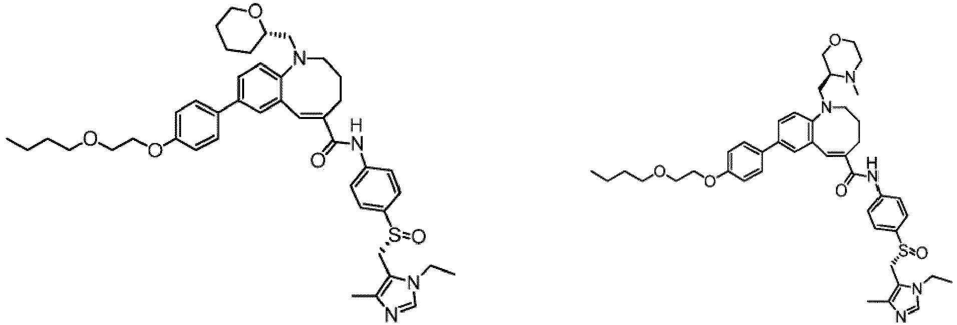

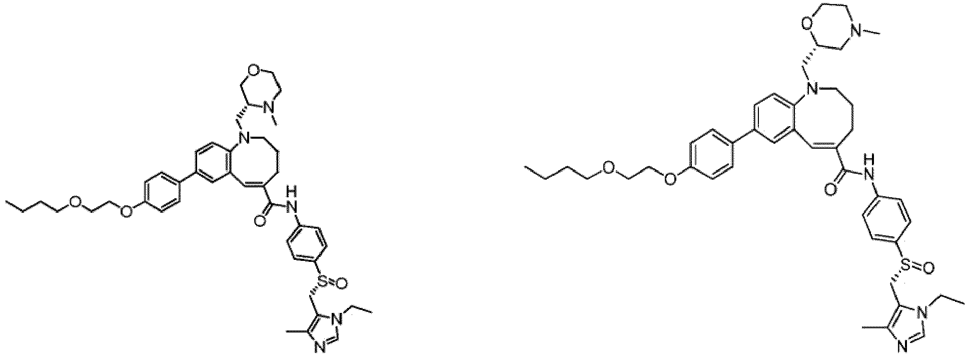

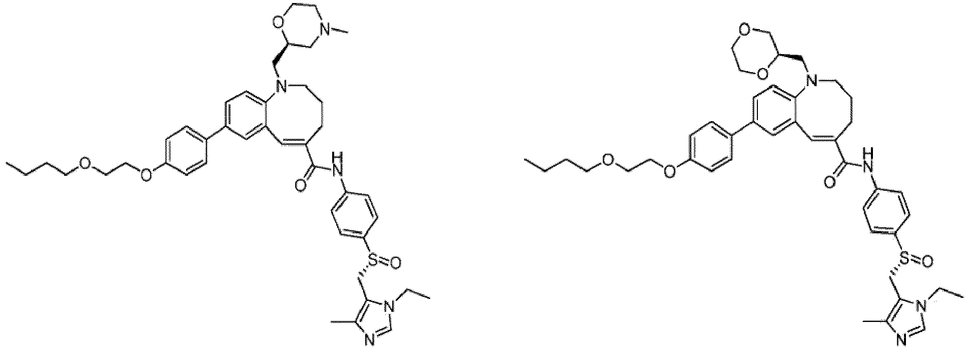

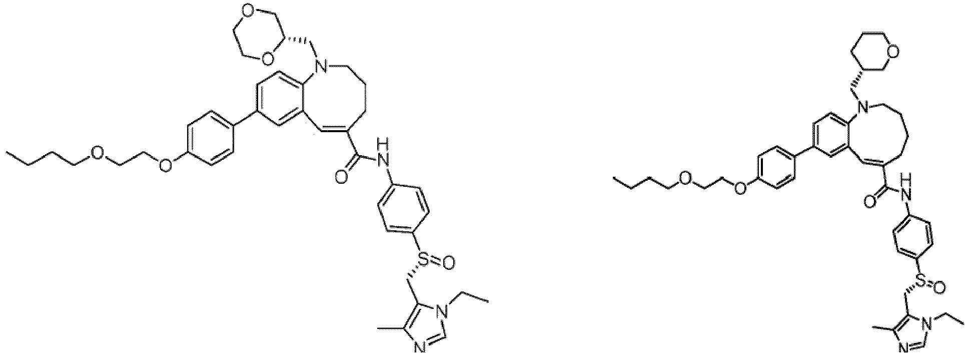

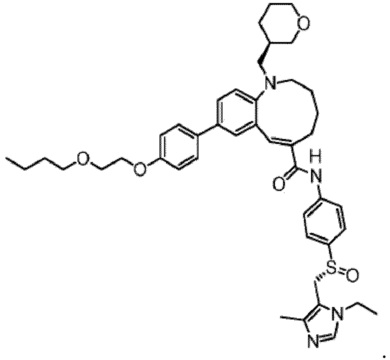

13. Une composition pharmaceutique, comprenant une quantité thérapeutiquement efficace du composé, ou de l'isomère ou du sel pharmaceutiquement acceptable de celui-ci selon l'une quelconque des revendications 1 à 12 comme un ingrédient actif, et un excipient pharmaceutiquement acceptable.

14. Le composé, ou l'isomère ou le sel pharmaceutiquement acceptable de celui-ci selon l'une quelconque des revendications 1 à 12, ou la composition pharmaceutique selon la revendication 13, pour une utilisation dans le traitement de maladies liées aux antagonistes de CCR2/CCR5.

15. Le composé, ou l'isomère ou le sel pharmaceutiquement acceptable de celui-ci selon l'une quelconque des revendications 1 à 12, ou la composition pharmaceutique selon la revendication 13, pour une utilisation dans le traitement de l'hépatite stéatosique non alcoolique.
